# EUROPEAN PATENT APPLICATION

(11) **EP 4 570 812 A1**
(43) Date of publication of application: **18.06.2025**
(21) Application number: 23857390.1
(22) Date of filing: 23.08.2023
(51) Int. Cl.: C07K 1/107, B01J 13/00, B32B 7/12, C07K 1/02, C07K 14/00, C07K 14/435, C09D 5/02, C09D 105/00, C09D 189/00, C09J 105/00, C09J 189/00, C12N 15/11, C12P 21/00

(54) **ESTERIFIED PROTEIN AND METHOD FOR PRODUCING SAME**

(30) Priority: 23.08.2022 JP 2022132682; 03.04.2023 JP 2023059940; 19.07.2023 JP 2023117429
(71) Applicant: Spiber Inc., Tsuruoka-shi, Yamagata 997-0052 (JP)
(72) Inventor: HAUSER Jacob Richard, Tsuruoka-shi, Yamagata 997-0052 (JP); SAKATA Kazuki, Tsuruoka-shi, Yamagata 997-0052 (JP); KAGATA Hideki, Tsuruoka-shi, Yamagata 997-0052 (JP); TAKAMI Taku, Tsuruoka-shi, Yamagata 997-0052 (JP)
(74) Representative: Handley, Matthew Edward
(86) International application number: PCT/JP2023/030366
(87) International publication number: WO 2024/043282

(57) **Abstract**

The present invention provides a method for producing a modified protein, the method including a step of treating a mixture containing a protein and an acylating agent by a mechanochemical method to obtain a modified protein.

## Description

### Technical Field

The present invention relates to an esterified protein and a method for producing the same.

### Background Art

In recent years, with growing interest in environmental pollution, a biodegradable protein material has attracted attention as an alternative material for a petroleum-derived material. Since the protein material exhibits physical properties depending on the type of protein, it is necessary to prepare a protein having physical properties required depending on an application.

For example, Patent Literature 1 discloses that a modified nucleic acid is prepared so as to express a desired amino acid sequence as a protein used for medicine or cosmetics to obtain a modified spider silk protein, and discloses that a substance such as a polypeptide, a polysaccharide, a marker molecule, a quantum dot, a metal, a nucleic acid, a lipid, or a low molecular weight drug is further bound to the modified spider silk protein. As described above, by another substance being bound to a protein using lysine, serine, and/or cysteine, a property different from a protein-specific property can be imparted.

### Citation List

### Patent Literature

Patent Literature 1: WO 2007/025719 A
Patent Literature 2: WO 2021/187502 A
Patent Literature 3: JP 5678283 B2
Patent Literature 4: JP 5782580 B2
Patent Literature 5: JP 5796147 B2
Patent Literature 6: JP 5823079 B2
Patent Literature 7: JP 6830604 B2

### Non Patent Literature

Non Patent Literature 1: Ochiai B. et al. ACSOmega4, 2019, 17542-17546.
Non Patent Literature 2: Weissbach U. et al. Beilstein J. Org. Chem. 2017, 13, 1788-1795.

### Summary of Invention

### Technical Problem

Usually, when a protein is modified, the protein is caused to react with a substance to be bound in a solvent capable of dissolving the protein. However, since the solvent capable of dissolving the protein is limited, there is a problem that the degree of freedom of a modification method is small, and as a result, a modified protein that can be produced is also limited. In addition, in a reaction in a solvent, the protein reacts with the solvent depending on the type of solvent used, and an unnecessary by-product may be generated. Specifically, dimethylsulfoxide (DMSO), hexafluoroisopropanol (HFIP), and the like are well known as the type of solvent capable of dissolving a protein. When an acylating agent is added to any one of these solvents in which a protein is dissolved, a Swern oxidation type oxidation reaction or acylation of an alcohol-based solvent may proceed. Therefore, an object of the present invention is to provide a new method for producing a modified protein.

### Solution to Problem

The present inventor has found that a desired modified protein can be obtained by acylating a protein under a mechanochemical condition. That is, the present invention provides the following (1) to (66).
(1) A method for producing a modified protein, the method including a step of treating a mixture containing a protein and an acylating agent by a mechanochemical method to obtain a modified protein.
(2) The method according to (1), in which the mixture further contains a base and/or a reaction accelerator.
(3) The method according to (1) or (2), in which the mixture further contains a solvent.
(4) The method according to (3), in which the amount of the solvent is 1 to 100 wt% with respect to the amount of the protein.
(5) The method according to any one of (1) to (4), further including a step of washing a product treated by the mechanochemical method.
(6) The method according to (5), further including a step of drying the washed product after the washing step.
(7) The method according to any of (1) to (6), in which the protein contains a hydrophobic protein.
(8) The method according to (7), in which the hydrophobic protein has a hydropathy index of more than 0.
(9) The method according to any of (1) to (8), in which the protein contains an artificial protein.
(10) The method according to (9), in which the artificial protein contains an artificial structural protein.
(11) The method according to any one of (1) to (10), further including a step of obtaining the protein by a microbiological method.
(12) The method for producing a modified protein according to (1), in which the acylating agent contains a hydrophilic group.
(13) The method for producing a modified protein according to (1), in which the acylating agent contains at least one selected from the group consisting of a hydroxy group, a carboxy group, an amino group, a sulfo group, and a phosphate group.
(14) The method for producing a modified protein according to (1), in which the acylating agent contains a hydrophobic group.
(15) The method for producing a modified protein according to (1), in which the acylating agent contains at least one selected from the group consisting of an alkyl group, an alkenyl group, an alkynyl group, a cycloalkyl group, and an aryl group.
(16) The method for producing a modified protein according to (1), in which the acylating agent contains a flame-retardant element (atom capable of imparting flame retardancy) .
(17) The method for producing a modified protein according to (1), in which the acylating agent contains a sulfur atom or a phosphorus atom.
(18) The method for producing a modified protein according to (1), in which the modified protein contains a flame-retardant element (atom capable of imparting flame retardancy) .
(19) The method for producing a modified protein according to (1), in which the modified protein contains a sulfur atom or a phosphorus atom.
(20) The method for producing a modified protein according to (1), in which the acylating agent is succinic anhydride or maleic anhydride.
(21) The method for producing a modified protein according to (1), in which the acylating agent contains at least one structure selected from the group consisting of a succinyl group and a maleyl group.
(22) The method for producing a modified protein according to (1), in which the acylating agent contains an alkyl group having 6 or more carbon atoms.
(23) The method for producing a modified protein according to (1), in which the acylating agent contains at least one structure selected from the group consisting of a fatty acid and an unsaturated fatty acid.
(24) The method according to (1), in which the treatment by the mechanochemical method is implemented by applying a shear force to the mixture.
(25) The method according to (24), in which the shear force is applied to the mixture using a mixer mill or an extruder.
(26) A modified protein obtained by the method according to any one of (1) to (25).
(27) A modified protein composition containing the modified protein according to (26).
(28) A modified protein molded body obtained by molding the modified protein composition according to (27).
(29) A molding material containing the modified protein according to (26).
(30) A modified protein molded body obtained by molding the molding material according to (29). (31) The modified protein molded body according to (28), which is any one of the group consisting of a fiber, a film, a gel, a porous body, a particle, and a resin.
(32) A fiber containing a modified protein obtained by the method according to any one of (1) to (25).
(33) A film containing a modified protein obtained by the method according to any one of (1) to (25).
(34) A hydrogel containing a modified protein obtained by the method according to any one of (1) to (25).
(35) A porous body containing a modified protein obtained by the method according to any one of (1) to (25).
(36) A particle containing a modified protein obtained by the method according to any one of (1) to (25).
(37) A resin containing a modified protein obtained by the method according to any one of (1) to (25).
(38) A method for producing a protein fiber, the method including: a step of treating a mixture containing a protein and an acylating agent by a mechanochemical method to obtain a modified protein; and a step of spinning the obtained modified protein.
(39) A method for producing a solution-state adhesive, the method including a step of dissolving a modified protein obtained by the method according to (1) in a solvent.
(40) A method for producing a water-dispersible adhesive, the method including a step of dispersing a modified protein obtained by the method according to (1) in an aqueous medium.
(41) A method for producing a film-shaped adhesive, the method including a step of molding a film from a solution in which a modified protein obtained by the method according to (1) is dissolved.
(41-1) A method for producing a film-shaped adhesive, the method including: a step of obtaining a modified protein by the method according to (1); and a step of dissolving the obtained modified protein in a solvent and molding a film.
(42) A method for producing a powdery adhesive, the method including a step of obtaining a powder containing a modified protein obtained by the method according to (1).
(42-1) A method for producing a powdery adhesive, the method including a step of powderizing a modified protein obtained by the method according to (1).
(42-2) A method for producing a powdery adhesive, the method including: a step of obtaining a modified protein by the method according to (1); and a step of powderizing the obtained modified protein.
(42-3) A method for producing a powdery adhesive, the method including a step of obtaining a powder composition containing a modified protein obtained by the method according to (1) and a tackiness improver or an adhesion improver.
(42-4) A method for producing a powdery adhesive, the method including: a step of adding a tackiness improver or an adhesion improver to a modified protein obtained by the method according to (1) to obtain a composition; and a step of powderizing the obtained composition.
(42-5) A method for producing a powdery adhesive, the method including: a step of obtaining a modified protein by the method according to (1); and a step of obtaining a powder composition containing the obtained modified protein and a tackiness improver or an adhesion improver.
(42-6) A method for producing a powdery adhesive, the method including: a step of obtaining a modified protein by the method according to (1); a step of adding a tackiness improver or an adhesion improver to the obtained modified protein to obtain a composition; and a step of powderizing the obtained composition.
(43) The method for producing an adhesive according to any one of (39) to (42), in which the modified protein is at least one selected from the group consisting of a maleic acid esterified protein, a succinic acid esterified protein, a sulfonic acid esterified protein, and a phosphoric acid esterified protein.
(44) The method for producing a solution-state adhesive according to (39), in which the modified protein is at least one selected from the group consisting of a maleic acid esterified protein, a succinic acid esterified protein, a sulfonic acid esterified protein, and a phosphoric acid esterified protein, and the solvent is a basic aqueous solution.
(45) The method for producing a film-shaped adhesive according to (41) or (41-1), in which the modified protein is at least one selected from the group consisting of a maleic acid esterified protein, a succinic acid esterified protein, a sulfonic acid esterified protein, and a phosphoric acid esterified protein, and the step of molding a film includes dissolving the modified protein in a basic aqueous solution.
(46) A method for producing an adhesive body, the method including: interposing a solution obtained by dissolving a modified protein obtained by the method according to (1) in a solvent between a plurality of adherends, and then removing the solvent from the solution to solidify the modified protein, thereby bonding the adherends to each other.
(46-1) A method for producing an adhesive body, the method including: a step of obtaining a modified protein by the method according to (1); a step of dissolving the obtained modified protein in a solvent to obtain a solution; and a step of interposing the obtained solution between a plurality of adherends, and then removing the solvent from the solution to solidify the modified protein, thereby bonding the adherends to each other.
(47) A method for producing an adhesive body, the method including interposing an aqueous dispersion obtained by dispersing a modified protein obtained by the method according to (1) in an aqueous medium between a plurality of adherends, and then removing the aqueous medium from the aqueous dispersion to solidify the modified protein, thereby bonding the adherends to each other.
(47-1) A method for producing an adhesive body, the method including: a step of obtaining a modified protein by the method according to (1); a step of dispersing the obtained modified protein in an aqueous medium to obtain an aqueous dispersion; and a step of interposing the obtained aqueous dispersion between a plurality of adherends, and then removing the aqueous medium from the aqueous dispersion to solidify the modified protein, thereby bonding the adherends to each other.
(48) A method for producing an adhesive body, the method including softening a film containing a modified protein obtained by the method according to (1) by swelling or heating, interposing the film between a plurality of adherends, and then curing the film in a state of being brought into pressure contact with the adherends, thereby bonding the adherends to each other.
(48-1) A method for producing an adhesive body, the method including: a step of obtaining a modified protein by the method according to (1); a step of dissolving the obtained modified protein in a solvent and molding a film; and a step of softening the obtained film by swelling or heating, interposing the film between a plurality of adherends, and then curing the film in a state of being brought into pressure contact with the adherends, thereby bonding the adherends to each other.
(49) A method for producing an adhesive body, the method including heating a powder containing a modified protein obtained by the method according to (1) in a state where the powder is interposed between a plurality of adherends, and pressurizing the powder via the adherends to solidify the powder, thereby bonding the adherends to each other.
(49-1) A method for producing an adhesive body, the method including: a step of powderizing a modified protein obtained by the method according to (1); and a step of heating the powder in a state where the obtained powder is interposed between a plurality of adherends, and pressurizing the powder via the adherends to solidify the powder, thereby bonding the adherends to each other.
(49-2) A method for producing an adhesive body, the method including: a step of obtaining a modified protein by the method according to (1); a step of powderizing the obtained modified protein; and a step of heating the powder in a state where the obtained powder is interposed between a plurality of adherends, and pressurizing the powder via the adherends to solidify the powder, thereby bonding the adherends to each other.
(49-3) A method for producing an adhesive body, the method including: a step of obtaining a powder composition containing a modified protein obtained by the method according to (1) and a tackiness improver or an adhesion improver; and a step of heating the obtained powder composition in a state where the powder composition is interposed between a plurality of adherends, and pressurizing the powder composition via the adherends to solidify the powder composition, thereby bonding the adherends to each other.
(49-4) A method for producing an adhesive body, the method including: a step of adding a tackiness improver or an adhesion improver to a modified protein obtained by the method according to (1) to obtain a composition; a step of powderizing the obtained composition; and a step of heating the obtained powder composition in a state where the powder composition is interposed between a plurality of adherends, and pressurizing the powder composition via the adherends to solidify the powder composition, thereby bonding the adherends to each other.
(49-5) A method for producing an adhesive body, the method including: a step of obtaining a modified protein by the method according to (1); a step of obtaining a powder composition containing the obtained modified protein and a tackiness improver or an adhesion improver; and a step of heating the obtained powder composition in a state where the powder composition is interposed between a plurality of adherends, and pressurizing the powder composition via the adherends to solidify the powder composition, thereby bonding the adherends to each other.
(49-6) A method for producing an adhesive body, the method including: a step of obtaining a modified protein by the method according to (1); a step of adding a tackiness improver or an adhesion improver to the obtained modified protein to obtain a composition; a step of powderizing the obtained composition; and a step of heating the obtained powder composition in a state where the powder composition is interposed between a plurality of adherends, and pressurizing the powder composition via the adherends to solidify the powder composition, thereby bonding the adherends to each other.
(50) The method for producing an adhesive body according to any one of (46) to (49), in which the modified protein is at least one selected from the group consisting of a maleic acid esterified protein, a succinic acid esterified protein, a sulfonic acid esterified protein, and a phosphoric acid esterified protein.
(51) The method for producing an adhesive body according to (46), in which the modified protein is at least one selected from the group consisting of a maleic acid esterified protein, a succinic acid esterified protein, a sulfonic acid esterified protein, and a phosphoric acid esterified protein, and the solvent is a basic aqueous solution.
(52) The method for producing an adhesive body according to (48), in which the film is obtained by cast-molding a solution in which the at least one modified protein selected from the group consisting of a maleic acid esterified protein, a succinic acid esterified protein, a sulfonic acid esterified protein, and a phosphoric acid esterified protein is dissolved in a basic aqueous solution.
(52-1) The method for producing an adhesive body according to (48-1), in which the modified protein is at least one selected from the group consisting of a maleic acid esterified protein, a succinic acid esterified protein, a sulfonic acid esterified protein, and a phosphoric acid esterified protein, and the step of molding a film is a step of dissolving the modified protein in a basic aqueous solution and then cast-molding the obtained solution.
(53) A method for producing a solution-state coating agent, the method including a step of dissolving a modified protein obtained by the method according to (1) in a solvent.
(53-1) A method for producing a solution-state coating agent, the method including: a step of obtaining a modified protein by the method according to (1); and a step of dissolving the obtained modified protein in a solvent.
(54) A method for producing a water-dispersible coating agent, the method including a step of dispersing a modified protein obtained by the method according to (1) in an aqueous medium.
(54-1) A method for producing a water-dispersible coating agent, the method including: a step of obtaining a modified protein by the method according to (1); and a step of dispersing the obtained modified protein in an aqueous medium.
(55) A method for producing a film-shaped coating agent, the method including a step of molding a film from a solution in which a modified protein obtained by the method according to (1) is dissolved.
(55-1) A method for producing a film-shaped coating agent, the method including: a step of obtaining a modified protein by the method according to (1); and a step of dissolving the obtained modified protein in a solvent and molding a film.
(56) A method for producing a powdery coating agent, the method including a step of obtaining a powder containing a modified protein obtained by the method according to (1).
(56-1) A method for producing a powdery coating agent, the method including a step of powderizing a modified protein obtained by the method according to (1).
(56-2) A method for producing a powdery coating agent, the method including: a step of obtaining a modified protein by the method according to (1); and a step of powderizing the obtained modified protein.
(56-3) A method for producing a powdery coating agent, the method including a step of obtaining a powder composition containing a modified protein obtained by the method according to (1) and a tackiness improver or an adhesion improver.
(56-4) A method for producing a powdery coating agent, the method including: a step of adding a tackiness improver or an adhesion improver to a modified protein obtained by the method according to (1) to obtain a composition; and a step of powderizing the obtained composition.
(56-5) A method for producing a powdery coating agent, the method including: a step of obtaining a modified protein by the method according to (1); and a step of obtaining a powder composition containing the obtained modified protein and a tackiness improver or an adhesion improver.
(56-6) A method for producing a powdery coating agent, the method including: a step of obtaining a modified protein by the method according to (1); a step of adding a tackiness improver or an adhesion improver to the obtained modified protein to obtain a composition; and a step of powderizing the obtained composition.
(57) The method for producing a coating agent according to any one of (41) to (44), in which the modified protein is at least one selected from the group consisting of a maleic acid esterified protein, a succinic acid esterified protein, a sulfonic acid esterified protein, and a phosphoric acid esterified protein.
(58) The method for producing a solution-state coating agent according to (51), in which the modified protein is at least one selected from the group consisting of a maleic acid esterified protein, a succinic acid esterified protein, a sulfonic acid esterified protein, and a phosphoric acid esterified protein, and the solvent is a basic aqueous solution.
(59) The method for producing a film-shaped coating agent according to (55), in which the modified protein is at least one selected from the group consisting of a maleic acid esterified protein, a succinic acid esterified protein, a sulfonic acid esterified protein, and a phosphoric acid esterified protein, and the method includes a step of dissolving the modified protein in a basic aqueous solution.
(60) A method for producing a laminate including a base material and a coating layer laminated on at least a part of a surface of the base material, the method including supplying a solution obtained by dissolving a modified protein obtained by the method according to (1) in a solvent to at least a part of the surface of the base material to coat at least a part of the surface of the base material with the solution, and then removing the solvent from the solution to solidify the modified protein, thereby laminating the coating layer on at least a part of the surface of the base material.
(60-1) A method for producing a laminate including a base material and a coating layer formed on at least a part of a surface of the base material, the method including: a step of obtaining a modified protein by the method according to (1); a step of dissolving the obtained modified protein in a solvent to obtain a solution; a step of supplying the solution to at least a part of the surface of the base material to coat at least a part of the surface of the base material with the solution; and a step of removing the solvent from the coating to form the coating layer.
(61) A method for producing a laminate including a base material and a coating layer laminated on at least a part of a surface of the base material, the method including supplying an aqueous dispersion obtained by dispersing a modified protein obtained by the method according to (1) in an aqueous medium to at least a part of the surface of the base material to coat at least a part of the surface of the base material with the aqueous dispersion, and then removing the aqueous medium from the aqueous dispersion to solidify the modified protein, thereby laminating the coating layer on at least a part of the surface of the base material.
(61-1) A method for producing a laminate including a base material and a coating layer formed on at least a part of a surface of the base material, the method including: a step of obtaining a modified protein by the method according to (1); a step of dispersing the obtained modified protein in an aqueous medium to obtain an aqueous dispersion; a step of supplying the aqueous dispersion to at least a part of the surface of the base material to coat at least a part of the surface of the base material with the aqueous dispersion; and a step of removing the aqueous medium from the coating to form the coating layer.
(62) A method for producing a laminate including a base material and a coating layer laminated on at least a part of a surface of the base material, the method including softening a film containing a modified protein obtained by the method according to (1) by swelling or heating, placing the film on at least a part of the surface of the base material, and then curing the film in a state of being brought into pressure contact with the base material, thereby laminating the coating layer on at least a part of the surface of the base material.
(62-1) A method for producing a laminate including a base material and a coating layer formed on at least a part of a surface of the base material, the method including: a step of obtaining a modified protein by the method according to (1); a step of dissolving the obtained modified protein in a solvent to mold a film; a step of softening the film by swelling or heating, and placing the film on at least a part of the surface of the base material; and a step of curing the film in a state of being brought into pressure contact with the base material to form the coating layer.
(63) A method for producing a laminate including a base material and a coating layer laminated on at least a part of a surface of the base material, the method including heating a powder containing a modified protein obtained by the method according to (1) in a state where the powder is placed on at least a part of the surface of the base material, and pressurizing the powder between a pressurizing body and the base material to solidify the powder, thereby laminating the coating layer on at least a part of the surface of the base material.
(63-1) A method for producing a laminate including a base material and a coating layer formed on at least a part of a surface of the base material, the method including: a step of powderizing a modified protein obtained by the method according to (1); and a step of heating the obtained powder in a state where the powder is placed on at least a part of the surface of the base material, and pressurizing the powder between a pressurizing body and the base material to solidify the powder, thereby laminating the coating layer on at least a part of the surface of the base material.
(63-2) A method for producing a laminate including a base material and a coating layer formed on at least a part of a surface of the base material, the method including: a step of obtaining a modified protein by the method according to (1); a step of powderizing the obtained modified protein; and a step of heating the obtained powder in a state where the powder is placed on at least a part of the surface of the base material, and pressurizing the powder between a pressurizing body and the base material to solidify the powder, thereby laminating the coating layer on at least a part of the surface of the base material.
(63-3) A method for producing a laminate including a base material and a coating layer formed on at least a part of a surface of the base material, the method including: a step of obtaining a powder composition containing a modified protein obtained by the method according to (1) and a tackiness improver or an adhesion improver; and a step of heating the obtained powder composition in a state where the powder composition is placed on at least a part of the surface of the base material, and pressurizing the powder composition between a pressurizing body and the base material to solidify the powder composition, thereby laminating the coating layer on at least a part of the surface of the base material.
(63-4) A method for producing a laminate including a base material and a coating layer formed on at least a part of a surface of the base material, the method including: a step of adding a tackiness improver or an adhesion improver to a modified protein obtained by the method according to (1) to obtain a composition; a step of powderizing the obtained composition; and a step of heating the obtained powder composition in a state where the powder composition is placed on at least a part of the surface of the base material, and pressurizing the powder composition between a pressurizing body and the base material to solidify the powder composition, thereby laminating the coating layer on at least a part of the surface of the base material.
(63-5) A method for producing a laminate including a base material and a coating layer formed on at least a part of a surface of the base material, the method including: a step of obtaining a modified protein by the method according to (1); a step of obtaining a powder composition containing the obtained modified protein and a tackiness improver or an adhesion improver; and a step of heating the obtained powder composition in a state where the powder composition is placed on at least a part of the surface of the base material, and pressurizing the powder composition between a pressurizing body and the base material to solidify the powder composition, thereby laminating the coating layer on at least a part of the surface of the base material.
(63-6) A method for producing a laminate including a base material and a coating layer formed on at least a part of a surface of the base material, the method including: a step of obtaining a modified protein by the method according to (1); a step of adding a tackiness improver or an adhesion improver to the obtained modified protein to obtain a composition; a step of powderizing the obtained composition; and a step of heating the obtained powder composition in a state where the powder composition is placed on at least a part of the surface of the base material, and pressurizing the powder composition between a pressurizing body and the base material to solidify the powder composition, thereby laminating the coating layer on at least a part of the surface of the base material.
(63-7) A method for producing a laminate including a base material and a coating layer formed on at least a part of a surface of the base material, the method including: a step of powderizing a modified protein obtained by the method according to (1); a step of adding a tackiness improver or an adhesion improver to the obtained modified protein to obtain a powder composition; a step of heating the powder composition and placing the powder composition on at least a part of the surface of the base material; and a step of pressurizing the powder composition between a pressurizing body and the base material to form the coating layer.
(64) The method for producing a laminate according to any one of (60) to (63), in which the modified protein is at least one selected from the group consisting of a maleic acid esterified protein, a succinic acid esterified protein, a sulfonic acid esterified protein, and a phosphoric acid esterified protein.
(65) The method for producing a laminate according to (60), in which the modified protein is at least one selected from the group consisting of a maleic acid esterified protein, a succinic acid esterified protein, a sulfonic acid esterified protein, and a phosphoric acid esterified protein, and the solvent is a basic aqueous solution.
(66) The method for producing a laminate according to (62), in which the film is obtained by molding a solution in which at least one modified protein selected from the group consisting of a maleic acid esterified protein, a succinic acid esterified protein, a sulfonic acid esterified protein, and a phosphoric acid esterified protein is dissolved in a basic aqueous solution.
(66-1) The method for producing a laminate according to (62), in which the modified protein is at least one selected from the group consisting of a maleic acid esterified protein, a succinic acid esterified protein, a sulfonic acid esterified protein, and a phosphoric acid esterified protein, and the step of molding a film is a step of dissolving the modified protein in a basic aqueous solution and molding the obtained solution.

### Advantageous Effects of Invention

According to the present invention, it is possible to provide a new method for producing a modified protein, and to obtain a broader modified protein having a higher degree of freedom in reaction conditions as compared with conventional modification of a protein performed in a solvent. In addition, according to the present invention, it is possible to produce a modified protein having appropriate physical properties according to a desired application.

As esterification by a mechanochemical method, acetylation of cellulose (see Non Patent Literature 1) and selective esterification of a lignin model compound using an enzyme (see Non Patent Literature 2) are known, but a case of applying the method to esterification of a protein is not known.

### Brief Description of Drawings

Fig. 1(a) is a photograph illustrating each of appearances of modified proteins of Comparative Examples 1 and 2 and Examples 1 and 2, and Fig. 1(b) is a graph comparing retention times of an unmodified protein PRT966 and the modified proteins in GPC.
Fig. 2(a) illustrates IR spectra of Comparative Example 1 and the unmodified protein PRT966, and Fig. 2(b) illustrates GPC chromatograms of Comparative Example 1 and the unmodified protein PRT966.
Fig. 3(a) illustrates IR spectra of Comparative Example 2 and the unmodified protein PRT966, and Fig. 3(b) illustrates GPC chromatograms of Comparative Example 2 and the unmodified protein PRT966.
Fig. 4(a) illustrates IR spectra of Example 1 and the unmodified protein PRT966, and Fig. 4(b) illustrates GPC chromatograms of Example 1 and the unmodified protein PRT966.
Fig. 5(a) illustrates IR spectra of Example 2 and the unmodified protein PRT966, and Fig. 5(b) illustrates GPC chromatograms of Example 2 and the unmodified protein PRT966.
Fig. 6(a) illustrates IR spectra of Example 3 and an unmodified protein PRT2882, and Fig. 6(b) illustrates GPC chromatograms of Example 3 and the unmodified protein PRT2882.
Fig. 7(a) illustrates IR spectra of Example 4 and an unmodified protein PRT2841, and Fig. 7(b) illustrates GPC chromatograms of Example 4 and the unmodified protein PRT2841.
Fig. 8(a) illustrates IR spectra of Example 5 and an unmodified protein PRT2662, and Fig. 8(b) illustrates GPC chromatograms of Example 5 and the unmodified protein PRT2662.
Fig. 9(a) illustrates IR spectra of Example 6 and an unmodified protein PRT1000, and Fig. 9(b) illustrates GPC chromatograms of Example 6 and the unmodified protein PRT1000.
Fig. 10(a) illustrates IR spectra of Example 7 and an unmodified protein PRT918, and Fig. 10(b) illustrates GPC chromatograms of Example 7 and the unmodified protein PRT918.
Fig. 11(a) illustrates IR spectra of Example 8 and the unmodified protein PRT966, and Fig. 11(b) illustrates GPC chromatograms of Example 8 and the unmodified protein PRT966.
Fig. 12(a) illustrates IR spectra of Example 9 and the unmodified protein PRT966, and Fig. 12(b) illustrates GPC chromatograms of Example 9 and the unmodified protein PRT966.
Fig. 13(a) illustrates IR spectra of Example 10 and the unmodified protein PRT966, and Fig. 13(b) illustrates GPC chromatograms of Example 10 and the unmodified protein PRT966.
Fig. 14(a) illustrates IR spectra of Example 11 and the unmodified protein PRT966, and Fig. 14(b) illustrates GPC chromatograms of Example 11 and the unmodified protein PRT966.
Fig. 15(a) illustrates IR spectra of Example 12 and the unmodified protein PRT966, and Fig. 15(b) illustrates GPC chromatograms of Example 12 and the unmodified protein PRT966.
Fig. 16(a) illustrates IR spectra of Example 13 and the unmodified protein PRT966, and Fig. 16(b) illustrates GPC chromatograms of Example 13 and the unmodified protein PRT966.
Fig. 17(a) illustrates IR spectra of Example 14 and the unmodified protein PRT966, and Fig. 17(b) illustrates GPC chromatograms of Example 14 and the unmodified protein PRT966.
Fig. 18(a) illustrates IR spectra of Example 15 and the unmodified protein PRT966, and Fig. 18(b) illustrates GPC chromatograms of Example 15 and the unmodified protein PRT966.
Fig. 19(a) illustrates IR spectra of Example 16 and the unmodified protein PRT966, and Fig. 19(b) illustrates GPC chromatograms of Example 16 and the unmodified protein PRT966.
Fig. 20(a) illustrates IR spectra of Example 17 and the unmodified protein PRT966, and Fig. 20(b) illustrates GPC chromatograms of Example 17 and the unmodified protein PRT966.
Fig. 21(a) illustrates IR spectra of Example 18 and the unmodified protein PRT966, and Fig. 21(b) illustrates GPC chromatograms of Example 18 and the unmodified protein PRT966.
Fig. 22(a) illustrates IR spectra of Example 19 and the unmodified protein PRT966, and Fig. 22(b) illustrates GPC chromatograms of Example 19 and the unmodified protein PRT966.
Fig. 23(a) illustrates IR spectra of Example 20 and the unmodified protein PRT966, and Fig. 23(b) illustrates GPC chromatograms of Example 20 and the unmodified protein PRT966.
Fig. 24(a) illustrates IR spectra of Example 21 and the unmodified protein PRT966, and Fig. 24(b) illustrates GPC chromatograms of Example 21 and the unmodified protein PRT966.
Fig. 25(a) illustrates IR spectra of Example 22 and the unmodified protein PRT966, and Fig. 25(b) illustrates GPC chromatograms of Example 22 and the unmodified protein PRT966.
Fig. 26(a) is a photograph illustrating a state where water is added to a protein or a modified protein, Fig. 26(b) is a photograph illustrating a state where acetone is added to a protein or a modified protein, and Fig. 26(c) is a photograph illustrating a state where a 0.5 w/v% sodium hydroxide aqueous solution is added to a protein or a modified protein.
Fig. 27(a) is a photograph illustrating a state of a protein immediately after DMSO is added, Fig. 27(b) is a photograph illustrating a state of being dissolved in DMSO, and Fig. 27(c) is a photograph comparing gelation states of proteins.
Fig. 28(a) illustrates IR spectra of Example 24 and the unmodified protein PRT966, and Fig. 28(b) illustrates GPC chromatograms of Example 24 and the unmodified protein PRT966.
Fig. 29(a) illustrates IR spectra of Example 25 and the unmodified protein PRT966, and Fig. 29(b) illustrates GPC chromatograms of Example 25 and the unmodified protein PRT966.
Fig. 30 is a photograph illustrating a screw segment of an extruder. In Fig. 30, FC represents an advancing conveying element, R represents a reversing element, K represents a kneading element, and C represents a compression element.
Fig. 31(a) illustrates IR spectra of Example 26 and the unmodified protein PRT966, and Fig. 31(b) illustrates GPC chromatograms of Example 26 and the unmodified protein PRT966.
Fig. 32(a) illustrates IR spectra of Example 27 and the unmodified protein PRT966, and Fig. 32(b) illustrates GPC chromatograms of Example 27 and the unmodified protein PRT966.
Fig. 33(a) illustrates IR spectra of Example 28 and the unmodified protein PRT966, and Fig. 33(b) illustrates GPC chromatograms of Example 28 and the unmodified protein PRT966.
Fig. 34(a) illustrates IR spectra of Example 29 and the unmodified protein PRT966, and Fig. 34(b) illustrates GPC chromatograms of Example 29 and the unmodified protein PRT966.
Fig. 35(a) illustrates IR spectra of Example 30 and the unmodified protein PRT966, and Fig. 35(b) illustrates GPC chromatograms of Example 30 and the unmodified protein PRT966.
Fig. 36(a) illustrates IR spectra of Example 31 and the unmodified protein PRT966, and Fig. 36(b) illustrates GPC chromatograms of Example 31 and the unmodified protein PRT966.
Fig. 37(a) illustrates IR spectra of Example 32 and the unmodified protein PRT966, and Fig. 37(b) illustrates GPC chromatograms of Example 32 and the unmodified protein PRT966.
Figs. 38(a) to 38(d) are photographs illustrating cast films of PRT966, Ac-PRT, Succ-PRT, and St-PRT, respectively.
Fig. 39(a) is a graph illustrating combustion times of samples, and Fig. 39(b) is a graph illustrating mass losses of the samples.
Fig. 40 is a graph illustrating softening points, Tg onsets, and melting temperatures Tm of the unmodified PRT966 and the modified proteins.

### Description of Embodiments

Hereinafter, modes for carrying out the present disclosure will be described in detail with reference to the drawings in some cases. Note that the following embodiments are examples for describing the present disclosure, and are not intended to limit the present disclosure to the following contents.

Materials exemplified in the present specification can be used alone or in combination of two or more thereof unless otherwise specified. The content of each component in a composition means the total amount of a plurality of substances present in the composition, unless otherwise specified, when the plurality of substances corresponding to each component in the composition is present.

A first embodiment of the present invention is a method for producing a modified protein, the method including a step of treating a mixture containing a protein and an acylating agent by a mechanochemical method to obtain a modified protein.

In the present specification, the "modified protein" is a protein in which at least a part or the whole of a lysine residue, a serine residue, a threonine residue, a tyrosine residue, or a cysteine residue in a protein as a raw material is acylated. That is, the protein as a raw material has at least one lysine residue, serine residue, threonine residue, tyrosine residue, or cysteine residue in an amino acid sequence thereof.

### <Protein>

The protein may be a natural protein or an artificial protein. For example, a protein having physical properties close to those required for a desired application can be adopted. Examples of the protein include a protein that can be used for industrial use and a protein that can be used for medical use. The phrase "can be used for industrial use" means, for example, that it can be used for various general-purpose materials used indoors or outdoors. Specific examples of the protein that can be used for industrial use include a structural protein. Specific examples of the structural protein include spider silk, silkworm silk, keratin, collagen, elastin, resilin, and proteins derived therefrom. A protein to be used may be an artificial fibroin or an artificial spider silk fibroin (artificial modified spider silk fibroin). Specific examples of the protein that can be used for medical use include an enzyme, a regulatory protein, a receptor, a peptide hormone, a cytokine, a membrane or a transport protein, an antigen used for vaccination, a vaccine, an antigen-binding protein, an immunostimulatory protein, an allergen, and a full length antibody or an antibody fragment or a derivative thereof.

The artificial protein includes a recombinant protein and a synthetic protein. That is, in the present specification, the "artificial protein" means a protein artificially produced. The artificial protein may be a protein having a domain sequence different from an amino acid sequence of a naturally derived protein, or may be a protein having a domain sequence identical to an amino acid sequence of a naturally derived protein. The "artificial protein" may be a protein obtained by using an amino acid sequence of a naturally derived protein as it is, a protein in which an amino acid sequence is modified based on an amino acid sequence of a naturally derived protein (for example, a protein in which an amino acid sequence is modified by modifying a cloned gene sequence of a naturally derived protein), or a protein artificially designed and synthesized independently of a naturally derived protein (for example, a protein having a desired amino acid sequence by chemically synthesizing a nucleic acid encoding a designed amino acid sequence). Note that, in the artificial protein, an amino acid sequence can be freely designed unlike a natural protein. Therefore, when the artificial protein is contained in a molding material or a molded body of the present embodiment described later, a function, a property, a physical property, and the like of the molding material or the molded body can be arbitrarily controlled by appropriately designing the amino acid sequence of the artificial protein. In addition, since a uniform molecular design is always possible, a protein that has high homology with a target protein and is suitable for a purpose can be stably obtained. As a result, the quality a molding material or a molded body obtained using the artificial protein is advantageously stabilized. From this viewpoint, an artificial structural protein is advantageously used as the artificial protein.

The number of amino acid residues of each of the protein and the modified protein according to the present embodiment is not particularly limited, and may be, for example, 50 or more. In addition, the number of amino acid residues is, for example, 100 or more, 150 or more, 200 or more, 250 or more, 300 or more, 350 or more, 400 or more, 450 or more, or 500 or more. The number of amino acid residues may be, for example, 5,000 or less, 4,500 or less, 4,000 or less, 3,500 or less, 3,000 or less, 2,500 or less, 2,000 or less, 1,500 or less, or 1,000 or less. As the number of amino acid residues is smaller, solubility in a solvent tends to increase.

A molecular weight of the protein according to the present embodiment is not particularly limited, and may be, for example, 2 kDa or more and 500 kDa or less. In addition, the molecular weight of the protein according to the present embodiment may be, for example, 2 kDa or more, 3 kDa or more, 4 kDa or more, 5 kDa or more, 6 kDa or more, 7 kDa or more, 8 kDa or more, 9 kDa or more, 10 kDa or more, 20 kDa or more, 30 kDa or more, 40 kDa or more, 50 kDa or more, 60 kDa or more, 70 kDa or more, 80 kDa or more, 90 kDa or more, or 100 kDa or more, and may be 500 kDa or less, 400 kDa or less, less than 360 kDa, 300 kDa or less, or 200 kDa or less.

The protein according to the present embodiment contains at least one nucleophilic functional group such as a serine residue, a threonine residue, a tyrosine residue, a lysine residue, or a cysteine residue. In the modified protein, a hydroxy group of a serine residue, a threonine residue, or a tyrosine residue, an amino group of a lysine residue or the like, or a sulfhydryl group of a cysteine residue in the protein is acylated. When the protein has a serine residue, a threonine residue, or a tyrosine residue, the protein can be more efficiently acylated by an acylating agent. In consideration of improvement in reactivity between the protein and the acylating agent, improvement in productivity of the modified protein, and the like, the total content of a serine residue, a threonine residue, and a tyrosine residue in the protein (a ratio of the total number of serine residues, threonine residues, and tyrosine residues to the total number of amino acid residues) may be, for example, 4% or more, 4.5% or more, 5% or more, 5.5% or more, 6% or more, 6.5% or more, or 7% or more. Note that an upper limit value of the total content of a serine residue, a threonine residue, and a tyrosine residue is not particularly limited, but may be, for example, 35% or less, 33% or less, 30% or less, 25% or less, or 20% or less in consideration of amino acid compositions of various proteins that can be suitably used as the protein and the modified protein according to the present embodiment.

### (Hydrophobic protein)

The protein may be, for example, a hydrophobic protein. In a case where the protein is a hydrophobic protein, when a molded body is produced using a modified protein thereof as at least a part of a raw material, water resistance of the molded body is improved, and for example, when the molded body is used as a general-purpose material for industrial use, a life of the molded body can be advantageously extended. In addition, in the modified protein, for example, when a functional substance is bound thereto, by controlling hydrophobicity or hydrophilicity of the functional substance, hydrophobicity or hydrophilicity of the entire conjugate of the functional substance and the modified protein can be arbitrarily adjusted. When the protein has hydrophobicity (that is, when the protein is a hydrophobic protein), the entire conjugate can be shifted to a hydrophobic side as compared with a case where the protein has hydrophilicity, and thus hydrophobicity or hydrophilicity of the entire conjugate can be controlled over a wider range.

Hydrophobicity in a protein can be estimated using a value of an average hydropathy index (hereinafter, also referred to as "HI") as an index. In the present specification, the value of the average hydropathy index of a hydrophobic protein only needs to be more than 0, and may be, for example, 0.00 or more, 0.10 or more, 0.20 or more, 0.22 or more, 0.25 or more, 0.30 or more, 0.35 or more, 0.40 or more, 0.45 or more, 0.50 or more, 0.55 or more, 0.60 or more, 0.65 or more, or 0.70 or more. An upper limit value thereof is not particularly limited, and may be, for example, 1.00 or less or 0.7 or less.

Note that the value of the average hydropathy index (HI) of the hydrophobic protein and a hydrophobic degree of a repetitive sequence unit described later are determined according to a known method () using a known hydrophobicity index of an amino acid residue. A known hydrophobicity index of an amino acid residue is presented in Table 1. For example, the hydrophobic degree may be calculated according to a method described in Kyte J & Doolittle R (1982) "A simple method for displaying the hydropathic character of a protein", J. Mol. Biol., 157, pp. 105 to 132.

**[Table 1]**

| Amino acid | HI | Amino acid | HI |
|---|---|---|---|
| Isoleucine (Ile) | 4.5 | Tryptophan (Trp) | -0.9 |
| Valine (Val) | 4.2 | Tyrosine (Tyr) | -1.3 |
| Leucine (Leu) | 3.8 | Proline (Pro) | -1.6 |
| Phenylalanine (Phe) | 2.8 | Histidine (His) | -3.2 |
| Cysteine (Cys) | 2.5 | Asparagine (Asn) | -3.5 |
| Methionine (Met) | 1.9 | Aspartic acid (Asp) | -3.5 |
| Alanine (Ala) | 1.8 | Glutamine (Gln) | -3.5 |
| Glycine (Gly) | -0.4 | Glutamic acid (Glu) | -3.5 |
| Threonine (Thr) | -0.7 | Lysine (Lys) | -3.9 |
| Serine (Ser) | -0.8 | Arginine (Arg) | -4.5 |

The hydrophobic protein preferably has low solubility in a lithium bromide aqueous solution (concentration: 9 M) at 60°C. That is, in a case where the hydrophobic protein is dissolved in a lithium bromide aqueous solution (concentration: 9 M) at 60°C, a maximum concentration may be, for example, less than 30 mass%, less than 25 mass%, less than 20 mass%, less than 15 mass%, less than 10 mass%, less than 5 mass%, or less than 1 mass%. Note that the hydrophobic protein may be completely insoluble in a lithium bromide aqueous solution (concentration: 9 M) at 60°C. When the hydrophobic protein has low solubility in a lithium bromide aqueous solution at 60°C, an obtained modified protein easily acquires favorable water resistance (water resistance particularly suitable for industrial applications).

The hydrophobic protein preferably has a contact angle with water of 55° or more. The contact angle of the hydrophobic protein with water is more preferably 60° or more, 65° or more, or 70° or more. The contact angle with water can be evaluated by forming a membrane made of a hydrophobic protein on a base material, dropping water on the membrane, and measuring the contact angle after five seconds. When the contact angle of the hydrophobic protein with water is 55° or more, an obtained modified protein easily acquires favorable water resistance (water resistance particularly suitable for industrial applications).

The hydrophobic protein preferably has excellent hot water resistance. Regarding the hot water resistance, for example, even when an aqueous dispersion of 5 w/v% of a hydrophobic protein is prepared and the dispersion is heated to 100°C, preferably, the protein is not decomposed for at least five hours. When the hydrophobic protein is excellent in hot water resistance, an obtained modified protein easily acquires favorable water resistance (water resistance particularly suitable for industrial applications) and hot water resistance.

### <Structural protein>

The protein according to the present embodiment may be, for example, a structural protein. The structural protein is a type of protein that can be used for industrial use, and means a protein related to a structure of a living body, a protein constituting a structure created by a living body, or a protein derived therefrom. For example, the structural protein is a protein not directly involved in a biochemical reaction but constituting a nail, hair, skin, tendon, cartilage, and intracellular/extracellular matrix, and may be a protein in which a molecule itself is fibrous and which has no hydrophobic core, like collagen or keratin, or a protein polymer in which a large number of monomers are polymerized in a fibrous form although the protein is a spherical protein when the protein is a monomer, like actin. The structural protein also refers to a protein that self-aggregates under predetermined conditions to form a structure such as a fiber, a film, a resin, a gel, a micelle, or a nanoparticle. Furthermore, the structural protein can also be said to be a protein in which a motif formed of a characteristic amino acid sequence or an amino acid residue is repeatedly present to form a skeleton of an organism or a material. Examples of the structural protein in nature include fibroin, keratin, collagen, elastin, and resilin. A fibroin of silk forms a fiber with a structure in which a polypeptide chain of a β sheet extends, and keratin has a plurality of polypeptide chains of an α helix bundled in a rope shape. In elastin, relatively nearly spherical polypeptide chains are crosslinked by covalent bonding to form a rubber-like elastic network. In collagen, elongated fibrous polypeptide chains form a triple helical structure (also called "collagen fibril"), and in each polypeptide chain, a glycine residue is present every third residue, proline residues are present in an amount of 20% or more of all amino acid residues, and about a half of the proline residues are hydroxyprolines, which contribute to stabilization of the triple helical structure.

The structural protein may be an artificial structural protein. In the present specification, the "artificial structural protein" means a structural protein artificially produced. The artificial structural protein may be a structural protein produced from a microorganism by a genetic recombination technique, may have the same amino acid sequence as that of a natural structural protein, or may have an amino acid sequence improved from a viewpoint of productivity, moldability, or the like.

When the structural protein is molded, an amino acid having a relatively smaller side chain is more likely to form a hydrogen bond, and a molded body having higher strength is more likely to be obtained. In addition, an alanine residue and a glycine residue are amino acids having nonpolar side chains, and therefore are arranged so as to face inward in a folding process during polypeptide production and easily have an α-helix structure or a β-sheet structure. Therefore, a ratio of an amino acid such as a glycine residue or an alanine residue is desirably high. An alanine residue content only needs to be, for example, 10 to 40%, and may be 12 to 40%, 15 to 40%, 18 to 40%, 20 to 40%, or 22 to 40% from a viewpoint of obtaining a molded body having better strength. A glycine residue content only needs to be, for example, 10 to 55%, and may be 11 to 55%, 13 to 55%, 15 to 55%, 18 to 55%, 20 to 55%, 22 to 55%, or 25 to 55% from a viewpoint of obtaining a molded body having better strength.

In the present specification, the "alanine residue content" means the number of alanine residues to the total number of amino acid residues constituting a protein, and is a value represented by the following formula.

Alanine residue content = (number of alanine residues contained in protein/total number of amino acid residues of protein) × 100 (%)

In addition, a glycine residue content, a serine residue content, a threonine residue content, a proline residue content, and a tyrosine residue content have the same meanings as those obtained by replacing the alanine residue with the glycine residue, the serine residue, the threonine residue, the proline residue, and the tyrosine residue, respectively, in the above formula.

In the structural protein, an amino acid having a relatively large side chain or an amino acid having flexibility is preferably uniformly contained in the entire sequence to a certain extent. Specifically, the structural protein may include a motif containing a tyrosine residue, a threonine residue, or a proline residue repeatedly in a cycle. When such a structural protein is used, formation of strong intermolecular hydrogen bonding is easily inhibited during processing of a molded body obtained by molding, and processability is easily improved. For example, the total content of a proline residue, a threonine residue, and a tyrosine residue in any consecutive 20 amino acid residues may be 5% or more, more than 5.5%, 6.0% or more, more than 6.5%, 7.0% or more, more than 7.5%, 8.0% or more, more than 8.5%, 9.0% or more, 10.0% or more, or 15.0% or more. In addition, for example, the total content of a proline residue, a threonine residue, and a tyrosine residue in any consecutive 20 amino acid residues may be 50% or less, 40% or less, 30% or less, or 20% or less.

In an artificial structural protein according to an embodiment, similarly to the protein described above, the total content of a serine residue, a threonine residue, and a tyrosine residue may be, for example, 4% or more, 4.5% or more, 5% or more, 5.5% or more, 6% or more, 6.5% or more, or 7% or more from a viewpoint of improving reactivity with an acylating agent, improving productivity of a reaction-modified protein obtained by reaction with the acylating agent, or the like. The total content of a serine residue, a threonine residue, and a tyrosine residue may be, for example, 35% or less, 33% or less, 30% or less, 25% or less, or 20% or less.

An artificial structural protein according to an embodiment may have a repetitive sequence. That is, the artificial structural protein according to the present embodiment may have a plurality of amino acid sequences (repetitive sequence units) having high sequence identity in the artificial structural protein. The number of amino acid residues of the repetitive sequence unit is preferably 6 to 200. The sequence identity between the repetitive sequence units may be, for example, 85% or higher, 90% or higher, 95% or higher, 96% or higher, 97% or higher, 98% or higher, or 99% or higher. A hydrophobic degree (hydropathy index) of the repetitive sequence unit may be, for example, -0.80 or more, -0.70 or more, -0.60 or more, -0.50 or more, -0.40 or more, -0.30 or more, -0.20 or more, -0.10 or more, 0.00 or more, 0.22 or more, 0.25 or more, 0.30 or more, 0.35 or more, 0.40 or more, 0.45 or more, 0.50 or more, 0.55 or more, 0.60 or more, 0.65 or more, or 0.70 or more. Note that an upper limit value of the hydrophobic degree of the repetitive sequence unit is not particularly limited, and may be, for example, 1.0 or less or 0.7 or less.

An artificial structural protein according to an embodiment may include an (A)ₙ motif. In the present specification, the (A)ₙ motif means an amino acid sequence mainly containing an alanine residue. The number of amino acid residues in the (A)ₙ motif may be 2 to 27 and may be an integer of 2 to 20, 2 to 16, or 2 to 12. A ratio of the number of alanine residues to the total number of amino acid residues in the (A)ₙ motif may be 40% or more, and may also be 50% or more, 600 or more, 70% or more, 80% or more, 83% or more, 85% or more, 86% or more, 90% or more, 95% or more, or 100% (which means that the (A)ₙ motif consists only of alanine residues).

The artificial structural protein may be an artificial fibroin. Examples of the fibroin include a naturally derived fibroin. Examples of the naturally derived fibroin include a fibroin produced by an insect or a spider. A natural fibroin is a fibrous protein, has a molecular weight of about 370,000, is constituted by two subunits, and has a high content of a glycine residue, an alanine residue, a serine residue, and a tyrosine residue, and these amino acid residues account for nearly 90% of the total number of amino acid residues. The natural fibroin has a crystalline region rich in amino acid residues having relatively small side chains, such as glycine, alanine, and serine, and an amorphous region having amino acid residues having relatively large side chains, such as tyrosine.

More specific examples of the naturally derived fibroin include a fibroin whose sequence information is registered in NCBI GenBank. For example, it can be confirmed by extracting a sequence in which spidroin, ampullate, fibroin, "silk and polypeptide", or "silk and protein" is described as a keyword in DEFINITION from sequences including INV as DIVISION among pieces of sequence information registered in NCBI GenBank, a sequence in which a specific character string of product is described from CDS, or a sequence in which a character string specific to TISSUE TYPE is described from SOURCE.

In the present specification, the "artificial fibroin" means an artificially produced fibroin (artificial fibroin). The artificial fibroin may be a fibroin having an amino acid sequence different from or identical to an amino acid sequence of a naturally derived fibroin. The artificial fibroin can be produced by a known method, for example, a method described in WO 2021/187502 A.

The artificial fibroin may be a fibrous protein having a structure similar to that of a naturally derived fibroin, or may be fibroin having a sequence similar to a repetitive sequence of a naturally derived fibroin. The "sequence similar to a repetitive sequence of a fibroin" may be an actual sequence of a naturally derived fibroin, or may be a sequence similar thereto.

The "artificial fibroin" may be a fibroin whose amino acid sequence is modified based on a naturally derived fibroin (for example, a fibroin whose amino acid sequence is modified by modifying a cloned gene sequence of a naturally derived fibroin) or a fibroin obtained by artificially designing an amino acid sequence independently of a naturally derived fibroin (for example, a fibroin having a desired amino acid sequence by chemically synthesizing a nucleic acid encoding the designed amino acid sequence) as long as the artificial fibroin has an amino acid sequence specified in the present disclosure. Note that a fibroin obtained by modifying an amino acid sequence of the artificial fibroin is also included in the artificial fibroin as long as an amino acid sequence thereof is different from an amino acid sequence of a naturally derived fibroin. Examples of the artificial fibroin include an artificial silk fibroin (a fibroin obtained by modifying an amino acid sequence of a silk protein produced by silkworms) and an artificial spider silk fibroin (a fibroin obtained by modifying an amino acid sequence of a spider silk protein produced by a spider). The artificial fibroin preferably contains an artificial spider silk fibroin as a molding material, and more preferably consists of the artificial spider silk fibroin because the artificial spider silk fibroin is relatively easily fibrillated and has a high fiber forming ability.

The artificial fibroin according to the present embodiment may be a protein including a domain sequence represented by Formula 1: [(A)ₙ motif-REP]ₘ or Formula 2: [(A)ₙ motif-REP]ₘ-(A)ₙ motif. An amino acid sequence (N-terminal sequence or C-terminal sequence) may be further added to any one or both of an N-terminal side and a C-terminal side of the domain sequence of the artificial fibroin. The N-terminal sequence and the C-terminal sequence are typically regions not containing repeats of amino acid motifs that are characteristic of fibroin and each consist of about 100 residues of amino acids, but are not limited thereto.

In the present specification, the "domain sequence" is an amino acid sequence that produces a crystal region (typically, corresponding to the (A)ₙ motif of the amino acid sequence) and an amorphous region (typically, corresponding to REP of the amino acid sequence) specific to fibroin, and means an amino acid sequence represented by Formula 1: [(A)ₙ motif-REP]ₘ or Formula 2: [(A)ₙ motif-REP]ₘ(A)ₙ motif. Here, the (A)ₙ motif represents an amino acid sequence mainly containing alanine residues, and the number of amino acid residues is 2 to 27. The number of amino acid residues in the (A)ₙ motif may be an integer of 2 to 20, 4 to 27, 4 to 20, 8 to 20, 10 to 20, 4 to 16, 8 to 16, or 10 to 16. In addition, a ratio of the number of alanine residues to the total number of amino acid residues in the (A)ₙ motif only needs to be 40% or more, and may also be 50% or more, 60% or more, 70% or more, 80% or more, 83% or more, 85% or more, 86% or more, 90% or more, 95% or more, or 100% (which means that the (A)ₙ motif consists only of alanine residues). At least seven of the plurality of (A)ₙ motifs in the domain sequence may consist only of alanine residues. REP represents an amino acid sequence consisting of 2 to 200 amino acid residues. REP may also be an amino acid sequence consisting of 10 to 200 amino acid residues. m represents an integer of 2 to 300, and may be an integer of 3 to 300, 4 to 300, 5 to 300, 6 to 300, or 10 to 300. The plurality of (A)ₙ motifs may be the same amino acid sequence or different amino acid sequences. The plurality of REPs may be the same amino acid sequence or different amino acid sequences.

Specific examples of the artificial fibroin include an artificial fibroin derived from a major dragline silk protein produced in a major ampullate gland of a spider as described in WO 2021/187502 A (first artificial fibroin), an artificial fibroin containing a domain sequence in which the content of glycine residues is reduced (second artificial fibroin), an artificial fibroin containing a domain sequence in which the content of an (A)ₙ motif is reduced (third artificial fibroin), an artificial fibroin in which the content of glycine residues and the content of an (A)ₙ motif are reduced (fourth artificial fibroin), an artificial fibroin containing a domain sequence including a region locally having a high hydropathy index (fifth artificial fibroin), and an artificial fibroin containing a domain sequence in which the content of glutamine residues is reduced (sixth artificial fibroin). The definition of each of the first to sixth artificial fibroins is incorporated in the present specification by reference with the content described in WO 2021/187502 A.

The artificial fibroin may include a tag sequence at one or both of an N-terminal and a C-terminal thereof. This enables isolation, immobilization, detection, visualization, and the like of the artificial fibroin. PRT966 is the sixth artificial fibroin including a tag sequence.

Examples of the tag sequence include an affinity tag using specific affinity (binding property or affinity) for another molecule. Specific examples of the affinity tag include a histidine tag (His tag). The His-tag is a short peptide in which about 4 to 10 histidine residues are arranged and can be used for isolating an artificial fibroin by chelating metal chromatography because the His-tag has a property of specifically binding to a metal ion such as nickel. Specific examples of the tag sequence include an amino acid sequence set forth in SEQ ID NO: 12 (an amino acid sequence including a His-tag sequence and a hinge sequence).

In addition, a tag sequence such as glutathione-S-transferase (GST) that specifically binds to glutathione or a maltose binding protein (MBP) that specifically binds to maltose can also be used.

Furthermore, an "epitope tag" using an antigen-antibody reaction can also be used. By adding a peptide (epitope) exhibiting antigenicity as a tag sequence, an antibody to the epitope can be bound. Examples of the epitope tag include an HA (a peptide sequence of influenza virus hemagglutinin) tag, a myc tag, and a FLAG tag. By using the epitope tag, an artificial fibroin can be easily purified with high specificity.

Furthermore, a tag sequence that can be cleaved by a specific protease can also be used. By treating a protein adsorbed via the tag sequence with a protease, an artificial fibroin from which the tag sequence is cleaved can be collected.

Specific examples of the artificial fibroin include artificial fibroins presented in Table 2 below.

**[Table 2]**

| | Alanine residue content (%) | Glycine residue content (%) | Serine residue content (%) | Threonine residue content (%) | Tyrosine residue content (%) | Glutamine residue content (%) | Lysine residue content (%) |
|---|---|---|---|---|---|---|---|
| PRT799 (SEQ ID NO: 1) | 19.8 | 31.0 | 9.3 | 0 | 7.0 | 17.3 | 0.1 |
| PRT966 (SEQ ID NO: 2) | 19.7 | 31.2 | 9.4 | 0 | 7.1 | 0 | 0 |
| PRT918 (SEQ ID NO: 3) | 19.5 | 30.9 | 9.7 | 0 | 7.0 | 0 | 0 |
| PRT313 (SEQ ID NO: 4) | 25.7 | 36.0 | 8.9 | 0 | 6.4 | 8.2 | 0 |
| PRT1000 (SEQ ID NO: 5) | 19.4 | 30.8 | 9.6 | 0 | 7.0 | 0 | 0 |
| PRT1001 (SEQ ID NO: 6) | 19.3 | 30.7 | 9.6 | 0 | 6.9 | 0 | 0 |
| PRT587 (SEQ ID NO: 7) | 19.7 | 31.1 | 9.4 | 0 | 7.1 | 17.3 | 0 |

The artificial fibroin may be an artificial fibroin having at least two or more of the characteristics of the first artificial fibroin, the second artificial fibroin, the third artificial fibroin, the fourth artificial fibroin, the fifth artificial fibroin, and the sixth artificial fibroin.

A molecular weight of the artificial fibroin according to the present embodiment is not particularly limited, and may be, for example, 2 kDa or more and 700 kDa or less The molecular weight of the artificial fibroin according to the present embodiment may be, for example, 2 kDa or more, 3 kDa or more, 4 kDa or more, 5 kDa or more, 6 kDa or more, 7 kDa or more, 8 kDa or more, 9 kDa or more, 10 kDa or more, 20 kDa or more, 30 kDa or more, 40 kDa or more, 50 kDa or more, 60 kDa or more, 70 kDa or more, 80 kDa or more, 90 kDa or more, or 100 kDa or more, and may be 700 kDa or less, 600 kDa or less, 500 kDa or less, 400 kDa or less, less than 360 kDa, 300 kDa or less, or 200 kDa or less.

In the present embodiment, the above-described protein is mixed with an acylating agent, and the obtained mixture is treated by a mechanochemical method to chemically modify the protein.

The acylating agent only needs to be a reagent capable of acylating a hydroxy group, an amino group, or a sulfhydryl group, and examples thereof include: an activated carboxylic acid derivative such as a carboxylic acid halide (for example, a carboxylic acid chloride or a carboxylic acid bromide) or a carboxylic anhydride (for example, an acid anhydride or a cyclic acid anhydride); an activated sulfonic acid derivative such as a sulfonic acid halide (for example, a sulfonic acid chloride or a sulfonic acid bromide) or a sulfonic anhydride; and a phosphoric acid derivative such as a phosphorus halide (for example, phosphorus trichloride or phosphorus oxychloride) or a phosphoric acid triester (for example, tris(bistrifluoroethyl) phosphate). These acylating agents introduce an acyl group corresponding to a carboxylic acid, a sulfonic acid, or a phosphoric acid. Examples of the carboxylic acid include: a saturated monocarboxylic acid such as acetic acid, propionic acid, butyric acid, valeric acid, caproic acid, enanthic acid (heptanoic acid), caprylic acid (octanoic acid), pelargonic acid (nonanoic acid), capric acid, lauric acid, myristic acid, palmitic acid, margaric acid, stearic acid, or isostearic acid; an unsaturated monocarboxylic acid such as oleic acid, linoleic acid, linolenic acid, arachidonic acid, eicosapentaenoic acid, or docosahexaenoic acid; and a dicarboxylic acid such as maleic acid. Examples of acid halides corresponding to these carboxylic acids include acetyl chloride, acetyl bromide, propionyl chloride, propionyl bromide, lauroyl chloride, myristoyl chloride, palmitoyl chloride, and stearoyl chloride. Examples of acid anhydrides corresponding to these carboxylic acids include acetic anhydride, propionic anhydride, and maleic anhydride. Examples of the sulfonic acid include methanesulfonic acid, ethanesulfonic acid, propanesulfonic acid, trifluoromethanesulfonic acid, nonafluorobutanesulfonic acid, benzenesulfonic acid, p-toluenesulfonic acid, m-toluenesulfonic acid, and o-toluenesulfonic acid. Examples of acid halides corresponding to these sulfonic acids include methanesulfonyl chloride, methanesulfonyl bromide, ethanesulfonyl chloride, ethanesulfonyl bromide, propanesulfonyl chloride, propanesulfonyl bromide, benzenesulfonyl chloride, p-toluenesulfonyl chloride, m-toluenesulfonyl chloride, and o-toluenesulfonyl chloride. Examples of acid anhydrides corresponding to these carboxylic acids include methanesulfonic anhydride, ethanesulfonic anhydride, trifluoromethanesulfonic anhydride, and nonafluorobutanesulfonic anhydride.

When the acylating agent is maleic anhydride, an obtained modified protein (maleic acid esterified protein) has a terminal carboxy group. The maleic acid esterified protein can be converted into a sulfosuccinylated protein by being caused to further react with sodium bisulfite by a mechanochemical method. The maleic acid esterified protein can be further converted into a (dialkoxyphosphoryl) succinylated protein by being caused to further react with a dialkyl phosphite by a mechanochemical method.

The amount of the acylating agent used can be 1.5 to 7 equivalents per nucleophilic functional group contained in the protein. The amount of the acylating agent used may preferably be 1.5 to 6.5 equivalents, 1.5 to 6 equivalents, 1.5 to 5.5 equivalents, 1.5 to 5 equivalents, 1.5 to 4 equivalents, 2 to 7 equivalents, 2 to 6.5 equivalents, 2 to 6 equivalents, 2 to 5.5 equivalents, 2 to 5 equivalents, 2 to 4.5 equivalents, 2.5 to 7 equivalents, 2.5 to 6.5 equivalents, 2.5 to 6 equivalents, 2.5 to 5.5 equivalents, 2.5 to 5 equivalents, or 2.5 to 4.5 equivalents per nucleophilic functional group. The "equivalent per nucleophilic functional group" means a molar equivalent of the acylating agent to one nucleophilic functional group contained in the protein. By setting the amount of the acylating agent used to a value within the above range, reactivity or reaction efficiency between the protein and the acylating agent in the mechanochemical treatment is enhanced, and a target modified protein is more efficiently obtained.

The treatment by the mechanochemical method is a treatment of performing a chemical reaction caused by directly absorbing mechanical energy. The mechanical energy may be exerted by, for example, an impact force or a shear force. Specifically, such a mechanochemical treatment is performed using a rolling ball mill, a medium stirring mill, a planetary mill, a jet mill, a mixer mill, or an extruder (twin-screw extruder). Specifically, the protein and the acylating agent are put in a grinding jar, and a medium ball or the like is put therein depending on the type of mill to be used. Thereafter, the grinding jar is set in a mixer mill apparatus and vibrated at a predetermined frequency and for a predetermined reaction time. At this time, a solvent, a base, a reaction accelerator, and the like may be further added to the grinding jar. Alternatively, the protein and the acylating agent are put in a mixer mill and a milling procedure is initiated to knead the reactants continuously. At this time, a solvent, a base, a reaction accelerator, and the like may be further added to the mixer mill. Alternatively, the protein and the acylating agent are put in an extruder and kneaded. At this time, a solvent, a base, a reaction accelerator, and the like may be further added to the extruder. Note that, when the mechanochemical treatment is performed using the extruder, a target esterified protein (modified protein) can be continuously produced.

The frequency can be appropriately adjusted by a person skilled in the art depending on reaction, and may be, for example, 10 to 50 Hz, 10 to 45 Hz, 10 to 40 Hz, 10 to 35 Hz, 15 to 50 Hz, 15 to 45 Hz, 15 to 40 Hz, 15 to 35 Hz, 20 to 50 Hz, 20 to 45 Hz, 20 to 40 Hz, or 20 to 35 Hz.

The reaction time can be up to a time point at which the protein serving as a raw material disappears or detection of an acylated protein (modified protein) is observed at a certain level or more in an infrared (IR) absorption spectrum, gel filtration chromatography (GPC), or the like. The reaction time may be any time, and may be, for example, 30 to 150 minutes, 30 to 120 minutes, 30 to 110 minutes, 30 to 100 minutes, 30 to 95 minutes, 60 to 150 minutes, 60 to 120 minutes, 60 to 110 minutes, 60 to 100 minutes, 60 to 95 minutes, 70 to 150 minutes, 70 to 120 minutes, 70 to 110 minutes, 70 to 100 minutes, 70 to 95 minutes, 80 to 150 minutes, 80 to 120 minutes, 80 to 110 minutes, 80 to 100 minutes, or 80 to 95 minutes.

The solvent only needs to be a solvent capable of swelling the protein or a solvent capable of dissolving at least one of the protein and the acylating agent, but the solvent should be a compound that is liquid at normal temperature and normal pressure and does not chemically react with the protein. A mechanochemical treatment to which such a solvent is added is known as liquid assisted grinding (LAG), and is a method for more efficiently advancing a desired reaction by adding a small amount of solvent into a grinding jar. Examples of the solvent include dimethyl sulfoxide (DMSO), N,N-dimethylformamide (DMF), N,N-dimethylacetamide (DMAc), N-methylpyrrolidone (NMP), dihydrolevoglucosenone, and an ionic liquid. The amount of the solvent used only needs to be, for example, 0.01 g to 1 g, and is preferably 0.01 g to 0.8 g, more preferably 0.01 g to 0.6 g, still more preferably 0.01 g to 0.4 g, and most preferably 0.01 g to 0.2 g with respect to 1 g of the mass of the protein. In other words, the amount of the solvent used only needs to be, for example, 1 to 100 wt%, and is preferably 1 to 80 wt%, more preferably 1 to 60 wt%, still more preferably 1 to 40 wt%, and most preferably 1 to 20 wt% with respect to the protein. It is considered that an extremely small amount of the solvent forms a microscopic reaction field by swelling the protein, locally bringing the protein into a dissolved state, or the like. Note that addition of the solvent is not necessarily required, and for example, when the acylating agent is a liquid at normal temperature and normal pressure and has the above function as the solvent, the addition of the solvent may be unnecessary.

The base may be any base as long as it can assist acylation of a hydroxy group of a serine residue, a threonine residue, or a tyrosine residue, an amino group of a lysine residue, or a sulfhydryl group of a cysteine residue in the protein. Examples of the base include a tertiary amine, a nitrogen-containing aromatic compound, and an inorganic base, and more specific examples thereof include triethylamine, DIPEA (N,N-diisopropylethylamine), DABCO (1,4-diazabicyclo [2.2.2] octane), quinuclidine, DBU (1,8-diazabicyclo [5.4.0] undec-7-ene), DBN (1,5-diazabicyclo [4.3.0] non-5-ene), pyridine, imidazole, sodium hydroxide, potassium hydroxide, sodium bicarbonate, potassium bicarbonate, sodium carbonate, potassium carbonate, cesium carbonate, tripotassium phosphate, sodium acetate, and potassium acetate. The amount of substance of the base used may be, for example, 1 to 10, 1 to 8, 1 to 7, 1 to 6, or 1 to 5 when the amount of substance of a nucleophilic functional group contained in the protein is 1. Note that the amount of substance of the base has a preferable range depending on the type of base used and the like, and for example, when an inorganic base such as sodium hydroxide is used as the base, the above amount of substance is preferably 1 to 5 and more preferably 1 to 2, and when a tertiary amine such as DIPEA is used as the base, the above amount of substance is preferably 1 to 5 and more preferably 3.5 to 5.

The reaction accelerator may be any one that is known to accelerate an acylation reaction in the field of organic chemistry, and examples thereof include 4-dimethylaminopyridine (DMAP). When the acylation is acetylation, no reaction accelerator needs to be used.

When the acylation is succinylation, addition of the reaction accelerator can increase a speed of the acylation reaction. In this case, the amount of the reaction accelerator used only needs to be more than 0 equivalents and 5 equivalents or less, and is preferably 3.5 equivalents to 5 equivalents with respect to the amount of substance 1 of a nucleophilic functional group contained in the protein (one amino acid residue having a nucleophilic functional group). The amount of the reaction accelerator used only needs to be more than 0 mol and 1 mol or less, and may be 0.3 mol to 1 mol with respect to 1 mol of the amount of the base used.

When the acylation is stearylation, addition of the reaction accelerator can increase a speed of the acylation reaction. In this case, the amount of the reaction accelerator used only needs to be 1 equivalent to 5 equivalents, and is preferably 3.5 equivalents to 5 equivalents with respect to the amount of substance 1 of a nucleophilic functional group contained in the protein (one amino acid residue having a nucleophilic functional group). The amount of the reaction accelerator used only needs to be 0.2 to 1 mol, and may be 0.7 to 1 mol with respect to 1 mol of the amount of the base used.

After completion of the reaction, the mixture is removed from the grinding jar, and may be washed with a solvent. By washing, an unreacted acylating agent and an acid generated by the reaction can be removed. Examples of the solvent used for washing include water, acetonitrile, acetone, tetrahydrofuran, ethyl acetate, and hexane. After washing, the solvent used for washing may be distilled off by drying a product. Drying may be performed under reduced pressure.

When the modified protein has a hydrophilic group (for example, a hydroxy group, a carboxy group, an amino group, a sulfo group, or a phosphate group) at a terminal thereof, a contact angle decreases. Use of a coating containing such a modified protein further increases wettability and hydrophilicity. When the modified protein has a hydrophobic group (for example, an alkyl group, an alkenyl group, an alkynyl group, a cycloalkyl group, or an aryl group) at a terminal thereof, a contact angle increases. Use of a coating containing such a modified protein further increases water repellency and hydrophobicity. When the contact angle is 90° to 150°, it is suitable for use for the purpose of waterproofing and water repellency, and when the contact angle is 110° to 150°, a self-washing effect of removing water droplets including surface dirt can be expected because of excellent water repellency of the material. When the contact angle is 70 to 90°, it is suitable for use for the purpose of moisturization, and when the contact angle is 40 to 90°, an effect of facilitating dyeing of the material can be expected by improving a liquid permeation effect and a liquid dispersion effect.

When the modified protein contains a flame-retardant element (for example, when the modified protein is a sulfonated or phosphorylated succinylated protein, a sulfonic acid esterified protein, or a phosphoric acid esterified protein), use of a coating containing such a modified protein further enhances flame retardancy. The coating containing such a modified protein is suitable for use for the purpose of improving safety and durability of a relevant product. Conventionally, it is known that flame retardancy can be imparted by blending a polyphosphoric acid salt or a polynucleotide salt with a protein. As the flame retardant, for example, those described in the Japan Rubber Association, "Review Special issue, Organic compounding agent, Flame retardant" (Hitoshi Nishizawa, 2006, p. 316 to 322) are known. However, only by blending the polyphosphoric acid salt or the polynucleotide salt, when the protein containing the polyphosphoric acid salt or the polynucleotide salt is immersed in water, the polyphosphoric acid salt or the polynucleotide salt may flow out, and the effect may be reduced. The modified protein according to the present embodiment has an effect that imparted flame retardancy is hardly reduced even when the modified protein is immersed in water. The flame-retardant element is an atom (an atom capable of imparting flame retardancy) often found in a structure of a flame retardant, and examples of the flame-retardant element include a halogen atom, a phosphorus atom, a sulfur atom, a boron atom, and a silicon atom.

A limiting oxygen index (LOI) value of the modified protein may be 18 or more, 20 or more, 22 or more, 24 or more, 26 or more, 27 or more, 28 or more, 29 or more, or 30 or more. In the present specification, the LOI value is a value measured in accordance with the test method for a particulate or low-melting point synthetic resin, defined in Fire Defense Hazardous Material No. 50 designated by Chief of Hazardous Materials Regulation Division, Fire Defense Agency, dated May 31, 1995.

In a case where the modified protein has a hydrophilic group at a terminal thereof (for example, in a case where the modified protein is a succinylated protein or a maleic acid esterified protein), when the modified protein has a long-chain alkyl group (an alkyl group having 4 or more carbon atoms is preferable, an alkyl group having 6 or more carbon atoms is more preferable, and an alkyl group having 8 or more carbon atoms is still more preferable) by incorporating a water molecule, an effect of promoting movement between polymers can be expected by reducing interaction between chains, and therefore a softening point is lower and a glass transition temperature (Tg) is lower than those of an unmodified protein. When the unmodified protein is heated to around 150°C, decomposition behavior is significant, and therefore it is difficult to soften the unmodified protein by heating the unmodified protein to 150°C or higher. However, in the case of the modified protein according to the present embodiment, since the softening point and the glass transition temperature are lowered, the modified protein can be softened at a temperature lower than 150°C, and is easily processed or molded. At a lower temperature, an influence of decomposition can be further suppressed, and deterioration of a material, a significant change in color, and a decrease in strength can be prevented. From this viewpoint, the softening point is desirably 130°C or lower, and more desirably 100°C or lower. The above modified protein is suitable for use for the purpose of molding using an extruder or the like and improving material flexibility at a temperature equal to or lower than the softening point.

In addition, when a molded body containing the modified protein further contains a plasticizer, the molded body has a softening point and a glass transition temperature that are further lowered. As the plasticizer, a component known in the present industry field can be used, and examples thereof include polyhydric alcohols such as ethylene glycol, propylene glycol, butylene glycol, and glycerol. However, only by blending a plasticizer, the plasticizer may flow out during processing or due to a change with time, and the effect may be reduced. The modified protein according to the present embodiment has an effect that an effect of lowering the softening point and the glass transition temperature of a molded body is hardly reduced even when heating during processing or long-term storage is assumed.

The modified protein according to the present embodiment can be used, for example, alone or as a composition formed by blending, adding, or mixing another solid or liquid component. That is, the modified protein can be formed as a modified protein composition containing the modified protein. Such a modified protein composition has a solid or liquid form depending on a component other than the modified protein. In addition, the modified protein composition can be used as, for example, a molding material for molding a fiber, a film, a gel, a porous body, a particle, a resin, or the like. In other words, a modified protein composition molded body is obtained by molding the modified protein composition by a predetermined method. As the other component contained in the solid or liquid modified protein composition, water, an organic solvent, an ionic liquid, a supercritical liquid, or the like can be adopted. Note that the modified protein composition may contain a tackiness improver or an adhesion improver particularly when used as an adhesive, a coating agent, or the like. Examples of the tackiness improver include a rosin derivative, and examples of the adhesion improver include a saccharide such as starch or sucrose.

Note that a molded body obtained by molding the modified protein composition can be produced by a known method according to the type of the molded body and the like. For example, the molded body is obtained by a known spinning method such as a wet spinning method, a dry-wet spinning method, or a dry spinning method using a dope solution containing a fiber, the modified protein, and a solvent. In addition, a film, a gel, a porous body, a particle, a resin, or the like can be produced, for example, in accordance with the methods described in JP 5678283 B2, JP 5782580 B2, JP 5796147 B2, JP 5823079 B2, JP 6830604 B2, and the like. Furthermore, the resin can also be obtained, for example, by removing, from the modified protein composition (for example, gel-like) containing the modified protein and a predetermined solvent or dispersion medium, the solvent or the dispersion medium to solidify the modified protein composition.

The molded body described above can also be molded using only the modified protein as a material. In this case, the modified protein can also be said to be a molding material of the molded body.

The modified protein according to the present embodiment can be used as an adhesive for bonding an adherend or a coating agent for laminating a coating layer on a base material in a solution state, an aqueous dispersion state, a film state, or a powder state containing the modified protein as a main component. The solution-state adhesive or the solution-state coating agent, the water-dispersible adhesive or the water-dispersible coating agent, the film-shaped adhesive or the film-shaped coating agent, the powdery adhesive or the powdery coating agent can be obtained by, for example, the following production method. In the following description of an embodiment of an adhesive, "laminate" refers to a state where one or more films are placed on a base material and subjected to pressure bonding, and "adhesive body" refers to a film itself having a property that can be bonded to a base material by pressure bonding. In addition, in the description of an embodiment of a coating agent, a member that applies pressure to a coating agent layer such that the coating agent layer is in close contact with a surface of a base material is referred to as "pressing body" or "pressurizing body".

The solution-state adhesive or the solution-state coating agent containing the modified protein can be produced by a method including a step of dissolving a modified protein obtained by the present embodiment method in a solvent. According to such a method, unlike a conventional solution-state adhesive or solution-state coating agent containing a petroleum-derived component, a biodegradable solution-state adhesive or solution-state coating agent can be easily obtained.

The solvent used in the production of the solution-state adhesive or the solution-state coating agent only needs to be a liquid capable of dissolving the modified protein, and examples thereof include water, a basic aqueous solution, an acidic aqueous solution, an aqueous medium such as an aqueous solution containing an inorganic salt, an organic solvent, and a mixed solvent thereof. Examples of the organic solvent include formic acid, dimethyl sulfoxide (DMSO), hexafluoroisopropanol (HFIP), N,N-dimethylformamide (DMF), N,N-dimethylacetamide (DMAc), N-methylpiperidone (NMP), and dihydrolevoglucosenone.

The modified protein contained in the solution-state adhesive or the solution-state coating agent is obtained by the production method according to the present embodiment, and is not particularly limited as long as the modified protein can be dissolved in the solvent described above. An adhesive or a coating agent in which the modified protein is dissolved in an aqueous medium has an advantage that handling properties and the like are superior to those of an adhesive or a coating agent in which the modified protein is dissolved in an organic solvent. As such an aqueous solution-state adhesive or an aqueous solution-state coating agent, for example, one obtained by dissolving a modified protein such as a maleic acid esterified protein, a succinic acid esterified protein, a sulfonic acid esterified protein, or a phosphoric acid esterified protein in a basic aqueous solution is suitably used. These modified proteins have high solubility in a basic aqueous solution. Therefore, when an aqueous solution-state adhesive or an aqueous solution-state coating agent in which any of the above-described modified proteins is dissolved in a basic aqueous solution is used, it is possible to apply the adhesive or the coating agent having a high concentration to a bonding surface of an adherend described later or a laminate surface, and improvement of adhesive strength thereof can be expected. In particular, in the case of the aqueous solution-state adhesive or coating agent, molecules of the modified protein are easily loosened, easily enter fine recesses present on a surface of the adherend, and an adhesive force can be improved.

A concentration of the modified protein in the solution-state adhesive or the solution-state coating agent is appropriately determined based on a solubility of the modified protein in a solvent or the like. The concentration may be, for example, 5 to 40 mass%, 10 to 35 mass%, 15 to 20 mass%, or 20 to 25 mass%. In addition, the solution-state adhesive or the solution-state coating agent may contain, as necessary, components other than the modified protein, such as various additives contained in a known solution-state adhesive or solution-state coating agent.

The solution-state adhesive containing the modified protein is used for producing an adhesive body in which a plurality of adherends is bonded to each other. For example, the solution-state adhesive is interposed between a plurality of adherends to be bonded, then a solvent in the solution-state adhesive is removed to solidify the modified protein, whereby the adherends can be bonded to each other to obtain an adhesive body. According to such a method, since an adhesive body is obtained using a biodegradable adhesive, there is an advantage that an adhesive body capable of reducing an environmental load at the time of disposal can be easily produced. Note that a material of the adherend is not particularly limited as long as the adherends can be bonded to each other with a solution-state adhesive containing a modified protein, and the adherend may be an organic substance (for example, a cellulose product such as paper or wood, a synthetic resin, or a protein product) or an inorganic substance (a metal or a non-metal such as glass).

A specific method for producing the adhesive body using the solution-state adhesive is not limited at all. For example, the solution-state adhesive is put on a bonding surface by applying or dropping the solution-state adhesive to a bonding surface of at least one of adherends to be bonded to each other, or by bringing the bonding surfaces of the adherends into contact with a liquid surface of the solution-state adhesive or immersing the bonding surfaces in the liquid surface, then the adherends are caused to overlap with each other or to butt against each other at the bonding surfaces, whereby the solution-state adhesive may be interposed between the plurality of adherends. In addition, when the solvent in the solution-state adhesive interposed between the plurality of adherends is removed to solidify the modified protein, for example, heating, air drying, or natural drying may be performed in a state where pressure is applied to the plurality of adherends from at least one of an overlapping direction and a butting direction of the bonding surfaces such that the overlapped or butted bonding surfaces are brought into close contact with each other. Of course, when bonding is possible without applying such pressure, it is not necessary to apply pressure.

The solution-state coating agent containing the modified protein is used for producing a laminate in which a coating layer is laminated on the whole or a part of a surface of a base material. For example, the solution-state coating agent is supplied to at least a part of a surface of a base material on which a coating layer is to be laminated, the surface part of the base material is coated with the solution-state coating agent, and then a solvent in the solution-state adhesive is removed to solidify the modified protein. This makes it possible to obtain a laminate by laminating the coating layer on at least a part of the surface of the base material. Also, by such a method, a laminate can be obtained using a biodegradable coating agent, and therefore there is an advantage that a laminate capable of reducing an environmental load at the time of disposal can be easily produced. Note that a material of the base material is not particularly limited as long as a solution-state coating agent containing the modified protein can be bonded to the base material, and the base material may be similar to an adherend to be bonded with the solution-state adhesive described above.

A specific method for producing a laminate using the solution-state coating agent is also not limited at all. For example, by applying or dropping the solution-state coating agent to at least a part of a surface of the base material, bringing at least a part of the surface of the base material into contact with a liquid surface of the solution-state coating agent, or immersing at least a part of the surface of the base material in the liquid surface, the solution-state coating agent may be supplied to at least a part of the base material so as to form a layer having a predetermined thickness to perform coating. When the modified protein in the coating agent layer (solution-state coating agent) laminated on the surface of the base material is solidified, for example, the coating agent layer on the surface of the base material may be heated, air-dried, or naturally dried to remove the solvent in the coating agent layer. Note that, at this time, a predetermined pressing body (pressurizing body) may be placed on the coating agent layer so as to cover the entire surface of the coating agent layer, as necessary, and the coating agent layer may be pressed (pressurized) against the surface of the base material such that the coating agent layer is in close contact with the surface of the base material. At this time, it is preferable to prevent the pressing body from being bonded to the solution-state coating agent. For example, by applying a release agent to a contact surface of the pressing body with the coating agent layer, attaching release paper to the contact surface, performing a surface treatment such that the coating agent is not bonded to the contact surface, or using a material that is not bonded to the solution-state coating agent as the pressing body, the adhesion of the solution-state coating agent to the pressing body can be prevented. Note that examples of the pressing body include materials exhibiting releasability with respect to the solution-state coating agent, such as polytetrafluoroethylene (PTFE), polypropylene (PP), polyethylene (PE), and silicone rubber. Examples of the surface treatment of the pressing body include a coating treatment with silicone oil.

The water-dispersible adhesive or the water-dispersible coating agent containing the modified protein can be produced by a method including a step of dispersing a modified protein obtained by the present embodiment method in an aqueous medium (aqueous liquid). According to such a method, unlike a conventional water-dispersible adhesive or water-dispersible coating agent containing a petroleum-derived component, a biodegradable water-dispersible adhesive or water-dispersible coating agent can be easily obtained.

Examples of the aqueous medium used for producing the water-dispersible adhesive or the water-dispersible coating agent include water in which the modified protein can be dispersed, a basic aqueous solution, an acidic aqueous solution, and an aqueous solution containing an inorganic salt. When the solvent used dissolves the modified protein, the solvent is suitable for preparing the solution-state adhesive or coating agent, and when the solvent used does not completely dissolve the modified protein, the solvent is suitable for preparing the water-dispersible adhesive or coating agent.

The modified protein contained in the water-dispersible adhesive or the water-dispersible coating agent is obtained by the production method according to the present embodiment, and is not particularly limited as long as the modified protein can be dispersed in the aqueous medium described above. Examples of the modified protein include a maleic acid esterified protein, a succinic acid esterified protein, a sulfonic acid esterified protein, and a phosphoric acid esterified protein. These modified proteins have relatively high water absorbency, and therefore a larger amount of the modified proteins can be dispersed in an aqueous medium. Therefore, in the water-dispersible coating agent containing any of these modified proteins, the content ratio of the modified protein can be advantageously increased, and the content ratio of the aqueous medium can be effectively reduced. As a result, an improvement in a solidification speed of the modified protein by removal of the aqueous medium can be expected, and thus an advantage that an adhesion speed to an adherend can be increased can be obtained. Note that the water-dispersible adhesive and the water-dispersible coating agent may contain components other than the modified protein, such as various additives contained in a known water-dispersible adhesive or water-dispersible coating agent, as necessary.

The water-dispersible adhesive containing the modified protein is used for producing an adhesive body in which a plurality of adherends is bonded to each other. For example, the water-dispersible adhesive is interposed between a plurality of adherends to be bonded, then an aqueous medium in the water-dispersible adhesive is removed to solidify the modified protein, whereby adherends can be bonded to each other to obtain an adhesive body. According to such a method, since an adhesive body is obtained using a biodegradable adhesive, there is an advantage that an adhesive body capable of reducing an environmental load at the time of disposal can be easily produced. Note that a material of the adherend is not particularly limited as long as the adherend can be bonded with the water-dispersible adhesive containing the modified protein, and the adherend may be similar to an adherend to be bonded with the solution-state adhesive containing the modified protein.

A specific method for producing the adhesive body using the water-dispersible adhesive is not also limited at all. For example, when the water-dispersible adhesive is interposed between a plurality of adherends to be bonded, a similar method to the method used when the solution-state adhesive is interposed between a plurality of adherends can be adopted. In addition, also when the aqueous medium in the water-dispersible adhesive interposed between the adherends is removed to solidify the modified protein, a similar method to the method used when the solvent in the solution-state adhesive interposed between the adherends is removed to solidify the modified protein can be adopted.

The water-dispersible coating agent containing the modified protein is used for producing a laminate in which a coating layer is laminated on the whole or a part of a surface of a base material. For example, the water-dispersible coating agent is supplied to at least a part of a surface of a base material on which a coating layer is to be laminated, the surface part of the base material is coated with the solution-state coating agent, and then the aqueous medium in the water-dispersible adhesive is removed to solidify the modified protein. This makes it possible to obtain a laminate by laminating the coating layer on at least a part of the surface of the base material. Also, by such a method, a laminate can be obtained using a biodegradable coating agent, and therefore there is an advantage that a laminate capable of reducing an environmental load at the time of disposal can be easily produced. Note that a material of the base material is not particularly limited as long as the water-dispersible coating agent containing the modified protein can be bonded to the base material, and the base material may be similar to a base material on which a coating layer is formed with the solution-state coating agent described above.

A specific method for producing a laminate using the water-dispersible coating agent is not also limited at all. For example, when the water-dispersible coating agent is supplied to at least a part of the surface of the base material, a similar method to the method used when the above-described solution-state coating agent is supplied to the surface of the base material can be adopted. In addition, when the aqueous medium in the water-dispersible coating agent supplied to the surface of the base material is removed to solidify the modified protein, a method similar to the method used when the solvent in the solution-state coating agent supplied to the surface of the base material is removed to solidify the modified protein can be adopted.

The film-shaped adhesive or the film-shaped coating agent containing the modified protein can be produced by a method including a step of molding a film from a modified protein solution in which a modified protein obtained by the present embodiment method is dissolved. According to such a method, unlike a conventional film-shaped adhesive or film-shaped coating agent containing a petroleum-derived component, a biodegradable film-shaped adhesive or film-shaped coating agent having can be easily obtained.

As the solution used for producing the film-shaped adhesive or the film-shaped coating agent, for example, a modified protein solution used as the solution-state adhesive or the solution-state coating agent is suitably used. In particular, one obtained by dissolving a modified protein such as a maleic acid esterified protein, a succinic acid esterified protein, a sulfonic acid esterified protein, or a phosphoric acid esterified protein in a basic aqueous solution is suitably used. A film-shaped adhesive or a film-shaped coating agent obtained by cast-molding such a modified protein solution has high affinity for water. Therefore, as described later, in obtaining an adhesive body or a laminate, when a film-shaped adhesive or a film-shaped coating agent is put between adherends or on a surface of the base material and swollen, the film-shaped adhesive or the film-shaped coating agent absorbs more moisture to be sufficiently softened. As a result, it may be desired to improve adhesive strength of the film-shaped adhesive or the film coating agent to the adherend or the base material. Note that, as a method for molding a film from a modified protein solution, for example, a method for cast-molding a protein solution is adopted. Generally known procedures and known conditions can be adopted to perform the cast molding.

The film-shaped adhesive containing the modified protein is used for producing an adhesive body in which a plurality of adherends is bonded to each other. For example, in a state where a film-shaped adhesive is interposed between a plurality of adherends to be bonded, the film-shaped adhesive is swollen or heated to be softened, and then the film-shaped adhesive is cured in a state of being brought into pressure contact with the adherends, whereby the adherends can be bonded to each other to obtain an adhesive body. In addition, the film-shaped adhesive may be swollen or heated to be softened in advance, interposed between a plurality of adherends, and then cured in a state of being brought into pressure contact with the adherends. According to these methods, since an adhesive body is obtained using a biodegradable adhesive, there is an advantage that an adhesive body capable of reducing an environmental load at the time of disposal can be easily produced. Note that the adherend may be any adherend as long as the adherend can be bonded with the film-shaped adhesive containing the modified protein, and the adherend may be similar to an adherend to be bonded with the solution-state adhesive containing the modified protein.

The film-shaped adhesive is softened when it absorbs moisture or is heated, and is cured by subsequent drying or cooling. In addition, when the modified protein (which may be a protein before acylation) contained in the film-shaped adhesive has a property of exhibiting a shrinkage behavior by contact with water or heating when a film is molded, the film-shaped adhesive also shrinks by contact with water or heating. Therefore, when being swollen between adherends or heated, the film-shaped adhesive containing the modified protein is softened and partially bites into a gap present on a bonding surface of each of the adherends, and in particular, when the modified protein has the above-described shrinkage property, the film-shaped adhesive shrinks in a state of biting into the gap present on the bonding surface. Then, when the film-shaped adhesive is cured in this state, it is considered that the film-shaped adhesive and the adherends are bonded to each other by an anchor effect, and the adherends are bonded to each other. It is also conceivable that a hydrogen bond is generated between the modified protein contained in the film-shaped adhesive and the adherends, whereby the film-shaped adhesive and the adherends are bonded to each other.

Therefore, when the film-shaped adhesive is swollen to be softened, an aqueous liquid such as water that is absorbed by the film-shaped adhesive and can shrink the film-shaped adhesive, or a solution or a dispersion containing water is suitably used. Then, for example, by causing the film-shaped adhesive to absorb water by dropping the above-described aqueous liquid or immersing the film-shaped adhesive in the aqueous liquid, the film-shaped adhesive may be swollen to be softened. In addition, when the film-shaped adhesive softened by swelling is cured, for example, a method for heating and drying, air-drying, or naturally drying the film-shaped adhesive in a state where the film-shaped adhesive is interposed between adherends can be adopted.

When the film-shaped adhesive is heated to be softened, the film-shaped adhesive may be heated before the film-shaped adhesive is interposed between adherends, or the whole of the adherends and the film-shaped adhesive may be heated after the film-shaped adhesive is interposed between the adherends. A heating temperature is not particularly limited as long as the heating temperature is a temperature at which the film-shaped adhesive can be softened and the modified protein contained in the film-shaped adhesive is not adversely affected (for example, not decomposed). When the film-shaped adhesive softened by heating is cured, a method for cooling the film-shaped adhesive with a cooling device or allowing the film-shaped adhesive to cool can be adopted.

In order to cause the film-shaped adhesive softened by swelling or heating to sufficiently bite into a gap of a bonding surface of the adherend, it is desirable to bring an interface between the softened film-shaped adhesive and the adherend into close contact with each other. Therefore, for example, it is preferable to apply pressure to a plurality of adherends from at least one of an overlapping direction and a butting direction of the bonding surfaces to cure the softened film-shaped adhesive while pressing the softened film-shaped adhesive against the adherends. Any general method can be adopted as the method for pressurizing the adherends here.

The film-shaped coating agent containing the modified protein is used for producing a laminate in which a coating layer is laminated on the whole or a part of a surface of a base material. For example, in a state where the film-shaped coating agent is placed so as to cover at least a part of a surface of the base material, the film-shaped coating agent is swollen or heated to be softened, and then cured in a state of being brought into pressure contact with the surface of the base material, whereby a coating layer can be laminated on at least a part of the surface of the base material to obtain a laminate. In addition, the film-shaped coating agent may be swollen or heated to be softened in advance, placed so as to cover at least a part of the surface of the base material, and then cured in a state of being brought into pressure contact with adherends. According to these methods, since a laminate is obtained using a biodegradable coating agent, there is an advantage that a laminate capable of reducing an environmental load at the time of disposal can be easily produced. Note that the base material may be any material as long as the film-shaped coating agent containing the modified protein can be bonded to the base material, and the base material may be similar to an adherend to be bonded with the film-shaped adhesive containing the modified protein.

A mechanism by which the film-shaped coating agent is bonded to the surface of the base material is considered to be similar to a mechanism by which the film-shaped adhesive is bonded to a bonding surface of the adherend. Therefore, as a specific method for producing a laminate using the film-shaped coating agent, a similar method to the method used when an adhesive body is obtained using the film-shaped adhesive can be adopted. Note that, when a softened film-shaped coating agent is cured while being brought into pressure contact with the surface of the base material, for example, the film-shaped coating agent may be pressed against the surface of the base material using a pressing body similar to a pressing body used when the solution-state coating agent with which a top surface of the base material is coated is pressed against the surface of the base material.

As the powdery adhesive or the powdery coating agent containing a modified protein, a powder composition containing a powder of a modified protein obtained by the present embodiment method is used. This powder composition may contain auxiliary components such as various addition residues as long as the powder composition contains the modified protein as a main component.

The modified protein contained in the powdery adhesive or the powdery coating agent is not particularly limited as long as the modified protein is obtained by the production method according to the present embodiment. Examples of the modified protein include a maleic acid esterified protein, a succinic acid esterified protein, a sulfonic acid esterified protein, and a phosphoric acid esterified protein. As described above, these modified proteins have higher water absorbency than other modified proteins. Therefore, a powdery adhesive or a powdery coating agent containing such a modified protein has a relatively high water content. Therefore, it is possible to reduce a heating temperature and a pressurization amount necessary for converting the powdery adhesive or the powdery coating agent into a resin (solidifying the powdery adhesive or the powdery coating agent) in a state where the powdery adhesive or the powdery coating agent is placed on a bonding surface of the adherend or a surface of the base material as much as possible. As a result, when a powdery adhesive or a powdery coating agent containing the above-described modified protein is used, an advantage that a bonding operation to an adherend or a base material can be advantageously simplified is expected. Note that the powdery adhesive or the powdery coating agent may contain components other than the modified protein, such as various additives contained in a known powdery adhesive or powdery coating agent, as necessary.

The powdery adhesive containing the modified protein is also used for producing an adhesive body in which a plurality of adherends is bonded to each other. For example, in a state where the powdery adhesive is interposed between a plurality of adherends to be bonded, the powdery adhesive is heated and pressurized via the adherends to solidify the powdery adhesive, whereby the adherends can be bonded to each other to obtain an adhesive body. According to such a method, since an adhesive body is obtained using a biodegradable adhesive, there is an advantage that an adhesive body capable of reducing an environmental load at the time of disposal can be easily produced. Note that the adherend may be any adherend as long as the adherend can be bonded with the powdery adhesive containing the modified protein, and for example, the adherend may be similar to an adherend to be bonded with the solution-state adhesive containing the modified protein.

A specific method for producing the adhesive body using the powdery adhesive is not also limited at all. For example, when the powdery adhesive interposed between a plurality of adherends to be bonded is heated and pressurized, the adherends are sandwiched by metal plates and the like from both sides opposite to the bonding surface side, and the metal plates are heated to heat the powdery adhesive interposed between the adherends together with the adherends. At the same time, the metal plates are pressurized with a hand press machine or the like, and the powdery adhesive is pressurized for a predetermined time via the adherends. As a result, the powdery adhesive is converted into a resin and solidified to bond the adherends. Note that a heating temperature, a pressurization amount, and a heating and pressurization time when the powdery adhesive is solidified are appropriately selected from a range in which the modified protein can be converted into a resin according to the type of modified protein contained in the powdery adhesive.

The powdery coating agent containing the modified protein is also used for producing a laminate in which a coating layer is laminated on the whole or a part of a surface of a base material. For example, in a state where the powdery coating agent is placed on at least a part of a surface of a base material, the powdery coating agent is heated and pressurized between a pressurizing body and the base material to be solidified, whereby a coating layer is laminated on at least a part of the surface of the base material.

A specific method for producing a laminate using the powdery coating agent is not also limited at all. For example, a metal plate or the like is disposed so as to cover the entire powdery adhesive placed on the surface of the base material, and the metal plate is heated to heat the powdery adhesive. At the same time, the powdery adhesive is pressurized between the metal plate and the base material for a predetermined time with a hand press machine or the like, whereby the powdery coating agent is converted into a resin and solidified. Note that a heating temperature, a pressurization amount, and a heating and pressurization time of the powdery coating agent are similar to those when an adhesive body is obtained using the powdery adhesive.

### Examples

The present invention will be described in more detail below.

Abbreviations used in Examples are used in a sense commonly used in the field of organic chemistry or genetic engineering unless otherwise stated. Some examples are described below.
Ac₂O: acetic anhydride
DBU: 1,8-diazabicyclo [5.4.0] undec-7-ene
DIPEA: N,N-diisopropylethylamine
DMAP: 4-dimethylaminopyridine
DMSO: dimethyl sulfoxide
FT-IR spectrum: Fourier transform infrared spectroscopic spectrum
GPC: gel filtration chromatography
HFIP: hexafluoroisopropanol
RO water: water treated using reverse osmosis membrane (RO membrane)
TCEP: tris(2-carboxyethyl) phosphine

In order to evaluate a ball-mill potential in chemical modification of a recombinant protein, acylation of the recombinant protein was performed both by a mechanochemical method and a conventional solution method, and products were evaluated by various analytical methods.

### 1. Acetylation by solution method

### (Comparative Example 1) Acetylation of 100 kDa recombinant protein (in absence of base)

A recombinant protein PRT966 (500 mg, 4.80 µmol) was suspended in DMSO (10 mL) while being stirred using a magnetic stirrer hot plate and then dissolved by continuous heating and stirring (80°C, 1 hour) of the mixture to obtain a clear brown solution. Subsequently, Ac₂O (311 µL, 3.30 mmol, 3.5 equivalents per hydroxy group) was added to the reaction solution, and then stirred (80°C, 6 hours). Thereafter, the reaction mixture was cooled to room temperature, and was gradually separated into ice-cooled (0°C) RO water (500 mL) while being gently stirred to precipitate a product (1 hour). Thereafter, filtration was performed under reduced pressure, and the product was sufficiently washed with RO water and acetone. Thereafter, the product was completely dried under reduced pressure in a vacuum oven (80°C, 1 hour) to obtain a sticky brown solid, which was directly subjected to analysis.

### (Comparative Example 2) Acetylation of 100 kDa recombinant protein (in presence of base)

A recombinant protein PRT966 (500 mg, 4.80 µmol) was suspended in DMSO (10 mL) while being stirred using a magnetic stirrer hot plate and then dissolved by continuous heating and stirring (80°C, 1 hour) of the mixture to obtain a clear brown solution. Next, Ac₂O (311 µL, 3.30 mmol, 3.5 equivalents per hydroxy group) and DIPEA (574 µL, 3.30 mmol, 3.5 equivalents per hydroxy group) were added to the reaction solution and stirred (80°C, 6 hours). Thereafter, the reaction mixture was cooled to room temperature, and was gradually added into ice-cooled (0°C) RO water (500 mL) while being gently stirred to precipitate a product (1 hour). Thereafter, the product was filtered under reduced pressure, and sufficiently washed with RO water and acetone. Thereafter, the product was completely dried under reduced pressure in a vacuum oven (80°C, 1 hour) to obtain a light brown solid, which was directly subjected to analysis.

### 2. Acetylation by mechanochemical method

### (Example 1) Acetylation of 100 kDa recombinant protein (in absence of base)

A recombinant protein PRT966 (500 mg, 4.80 µmol) and Ac₂O (311 µL, 3.30 mmol, 3.5 equivalents per hydroxy group) were put in a 10 mL stainless steel cup (manufactured by Retsch GmbH), 20 stainless steel balls (diameter: 5 mm, manufactured by Retsch GmbH) were added thereto, and the mixture was milled (frequency: 30 Hz, 90 minutes) with a mixer mill type ball mill (mixer mill MM400, manufactured by Retsch GmbH) to obtain a weak yellow white amorphous solid. The reaction mixture was removed from the grinding jar and suspended in RO water (25 mL) while being stirred (10 minutes). Thereafter, the product was filtered under reduced pressure, and sufficiently washed with RO water and acetone. Thereafter, the product was completely dried under reduced pressure in a vacuum oven (80°C, 1 hour) to obtain a white amorphous powder, which was directly subjected to analysis.

### (Example 2) Acetylation of 100 kDa recombinant protein (in presence of base)

A 100 kDa recombinant protein PRT966 (500 mg, 4.80 µmol), Ac₂O (311 µL, 3.30 mmol, 3.5 equivalents per hydroxy group), and DIPEA (574 µL, 3.30 mmol, 3.5 equivalents per hydroxy group) were put in a 10 mL stainless steel cup (manufactured by Retsch GmbH), 20 stainless steel balls (diameter: 5 mm, manufactured by Retsch GmbH) were added thereto, and the mixture was milled (frequency: 30 Hz, 90 minutes) with a mixer mill type ball mill (trade name: mixer mill MM400, manufactured by Retsch GmbH) to obtain a waxy brown solid. The reaction mixture was removed from the grinding jar and suspended in RO water (25 mL) while being stirred (10 minutes). Thereafter, the product was filtered under reduced pressure, and sufficiently washed with RO water and acetone. Thereafter, the product was completely dried under reduced pressure in a vacuum oven (80°C, 1 hour) to obtain a weak yellow white amorphous powder, which was directly subjected to analysis.

### 3. Comparison between acetylated product obtained by liquid phase method and acetylated product obtained by mechanochemical method

### (1) IR absorption spectrum analysis

A Fourier transform infrared absorption (FT-IR) spectrum was performed using a Nicolet iS50 FT-IR spectrometer (trade name, manufactured by Thermo Fisher Scientific). A neat state sample was analyzed using attenuated total reflection (ATR) of diamond crystals, and an absorption frequency was recorded in wavenumbers (cm⁻¹) to 0.1 cm⁻¹ units. The spectra were collected at a resolution of 4 cm⁻¹ in a range of 760 to 3800 cm⁻¹, and an average value of 64 scans was calculated.

### (2) GPC analysis

In GPC, an analyte was separated using a liquid chromatography system (trade name: Agilent 1260 Infinity II, manufactured by Agilent Technologies Inc.) equipped with a refractive index (RI) detector, a styrenedivinylbenzene copolymer column (inner diameter: 4.6 mm × 150 mm) stationary phase equipped with a 0.5 µm guard column filter (trade name: Shodex GPC HK-G, manufactured by Showa Denko K.K.), and HFIP (manufactured by Central Glass Co., Ltd.). The mobile phase was monitored using an Agilent 1260 Infinity II refractive index detector (RID) (manufactured by Agilent Technologies Inc.) at 40°C with a flow rate of 0.15 mL/min. A protein was analyzed as a 0.1 wt% solution obtained by adding sodium trifluoroacetate to HFIP (final concentration: 1 mM). To a protein having a cysteine residue, TCEP (final concentration: 10 mM) was added in order to prevent formation of a disulfide bond. A sample was dissolved (45°C, 1500 rpm, 1 hour) using a heating and vibrating device (trade name: Front Lab MyBL-100S, manufactured by AS ONE Corporation). The resulting solution was caused to pass through a 0.45 µm hydrophilic PTFE membrane filter (trade name: Dismic 25HP, manufactured by Advan Tech), and remaining insoluble matters were removed.

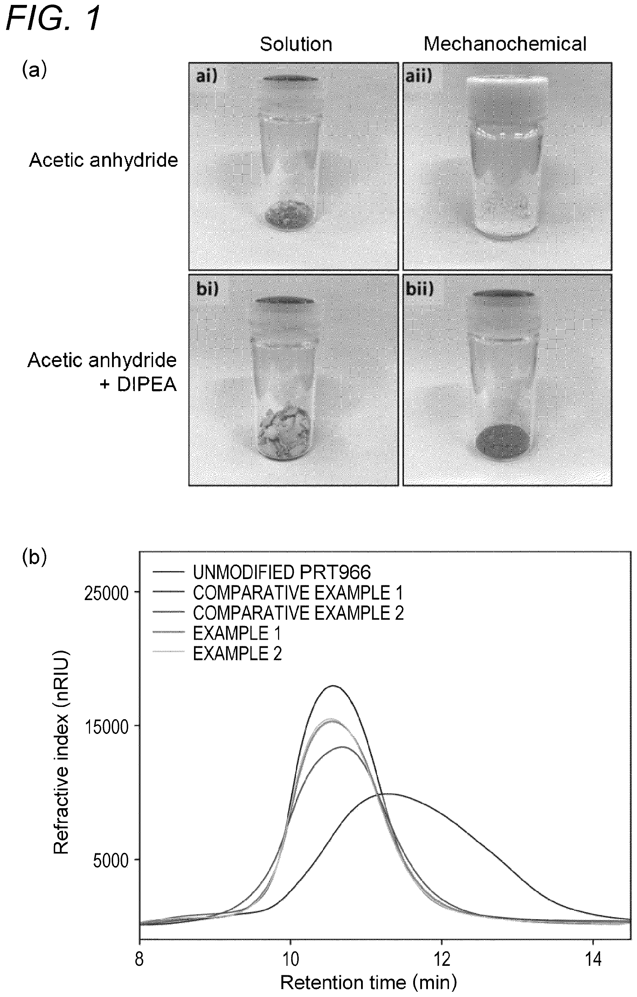

Fig. 1(a) illustrates a comparison among appearances of Comparative Examples 1 and 2 and Examples 1 and 2, and Fig. 1 (b) is a graph in which GPC chromatograms of Comparative Examples 1 and 2, Examples 1 and 2, and an unmodified protein PRT966 are superimposed on each other. The modified proteins of Comparative Examples 1 and 2 prepared by the solution method were brown lumps and had to be ground for use. On the other hand, Examples 1 and 2 prepared by the mechanochemical method were obtained as white powders, and were also excellent in handleability.

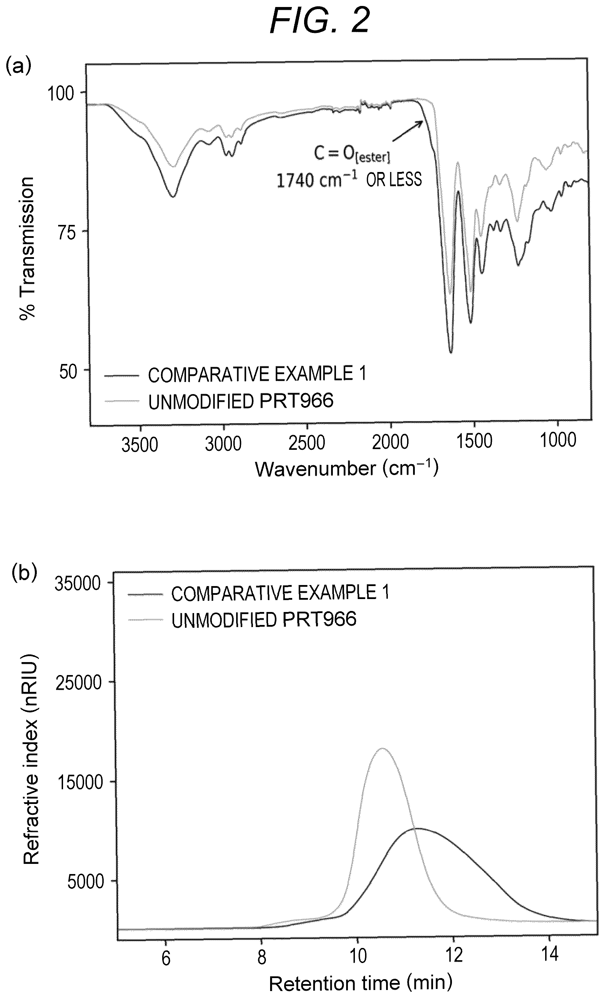

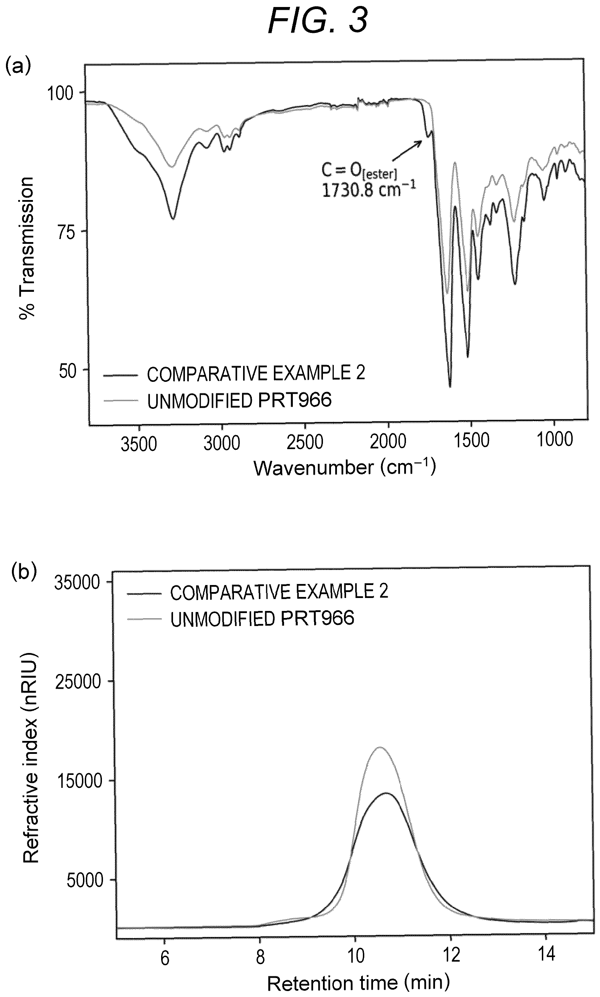

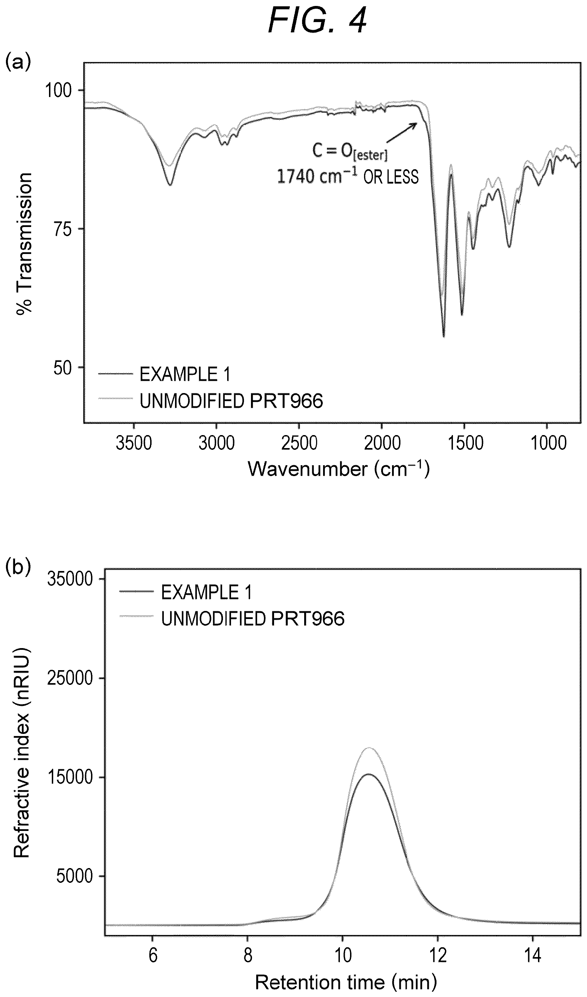

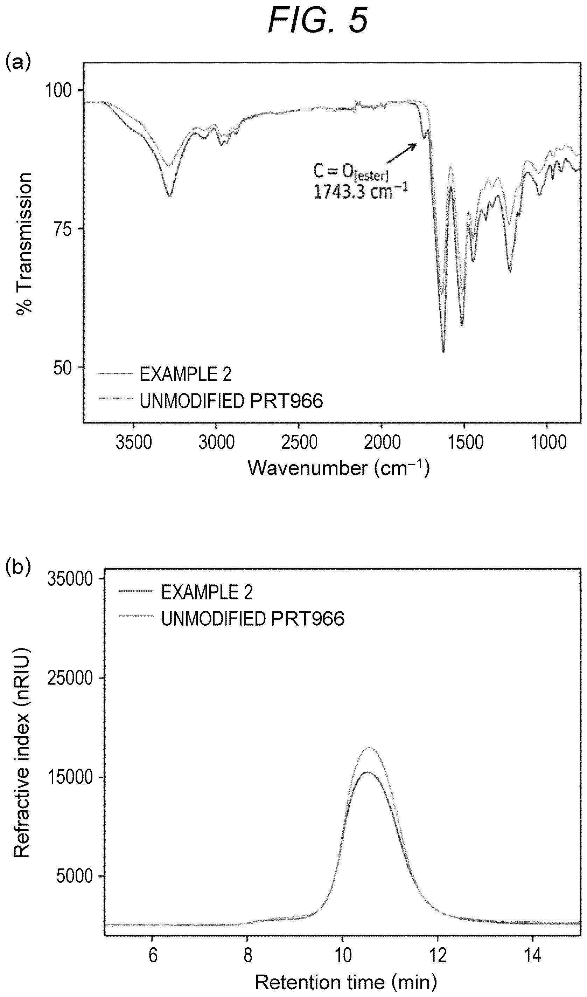

IR spectra and results of GPC analysis of Comparative Examples 1 and 2 and Examples 1 and 2 are illustrated in Figs. 2 to 5, respectively. Fig. 2(a) illustrates IR spectra of Comparative Example 1 and the unmodified protein PRT966, and a peak appeared at 1740 cm⁻¹, indicating that acetylation proceeded. Fig. 2(b) illustrates GPC chromatograms of Comparative Example 1 and the unmodified protein PRT966, and the retention time was shorter than that of the unmodified protein. Fig. 3(a) illustrates IR spectra of Comparative Example 2 and the unmodified protein PRT966, and a peak appeared at 1730 cm⁻¹, indicating that acetylation proceeded. Fig. 3(b) illustrates GPC chromatograms of Comparative Example 2 and the unmodified protein PRT966, and the retention time was slightly shorter than that of the unmodified protein. Fig. 4(a) illustrates IR spectra of Example 1 and the unmodified protein PRT966, and a peak appeared at 1740 cm⁻¹, indicating that acetylation proceeded. Fig. 4(b) illustrates GPC chromatograms of Example 1 and the unmodified protein PRT966, and the retention time was slightly shorter than that of the unmodified protein. Fig. 5(a) illustrates IR spectra of Example 2 and the unmodified protein PRT966, and a peak appeared at 1743 cm⁻¹, indicating that acetylation proceeded. Fig. 5(b) illustrates GPC chromatograms of Example 2 and the unmodified protein PRT966, and the retention time was slightly shorter than that of the unmodified protein.

### (3) SDS-PAGE analysis

Sodium dodecyl sulfate-polyacrylamide gel electrophoresis (SDS-PAGE) was performed using a precast separation gel (trade name: 4 to 20% Mini-PROTEAN TGX, manufactured by Bio-Rad Laboratories Inc.). The gel was inserted into an electrode assembly (trade name: Mini-PROTEAN Tetra Electrode Assembly, manufactured by Bio-Rad Laboratories Inc.) and disposed inside an electrophoresis tank together with an SDS running buffer (manufactured by Bio-Rad Laboratories Inc.). A protein was dissolved in DMSO, lithium chloride (final concentration: 2 M) was added thereto and dissolved at a final concentration of 1 wt% using a heating shaker (trade name: TMS-200 Turbo Thermoshaker Incubator, manufactured by Hangzhou Allsheng Instruments Co. Ltd.) (80°C, 2000 rpm, 45 minutes), and then the mixture was diluted 50 fold with a 10 M urea aqueous solution. Thereafter, the mixture was mixed with the same amount of a sample buffer containing 3-mercapto-1,2-propanediol, heated (five minutes) to 95°C (trade name: Genius Dry Bath Incubator MD-01N, manufactured by Major Science Co. Ltd.), then supplied onto a gel (10 µL), and a molecular weight ladder (trade name: XL-Ladder Brood, manufactured by Approscience Co., Ltd.) was added thereto for reference. Electrophoresis was performed at a constant voltage (200 V, 30 minutes) (trade name: PowerPac Universal Power Supply, manufactured by Bio-Rad Laboratories Inc.). The protein was stained by gently stirring (30 rpm, 1 hour) the gel (trade name: Duomax 1030, manufactured by HeidolphInstruments) using Oriole Fluorescent GelStain (manufactured by Bio-Rad Laboratories Inc.) and visualized with imaging software (GelDoc EZ gel imaging system and ImageLabsoftware (manufactured by Bio-Rad Laboratories Inc.)).

According to the results of SDS-PAGE, in the solution method using DMSO as a solution, a band of a low molecular weight protein generated by decomposition of a protein main chain due to oxidative stress in the system was observed, but in the mechanochemical method, such a band was not observed.

### 4. Influence of difference in molecular weight (from 7.5kDa to 300 kDa)

### (Example 3) Acetylation of 7.5 kDa recombinant protein

A recombinant protein PRT2882 (SEQ ID NO: 8, 500 mg, 50.5 µmol), Ac₂O (301 µL, 3.18 mmol, 3.5 equivalents per hydroxy group), and DIPEA (317 µL, 1.82 mmol, 2 equivalents per hydroxy group) were put in a 10 mL stainless steel cup (manufactured by Retsch GmbH), 20 stainless steel balls (diameter: 5 mm, manufactured by Retsch GmbH) were added thereto, and the mixture was milled (frequency: 30 Hz, 90 minutes) with a mixer mill type ball mill (trade name: mixer mill MM400, manufactured by Retsch GmbH) to obtain a dark gray massive solid. The reaction mixture was removed from the grinding jar and suspended in RO water (25 mL) while being stirred (10 minutes). Thereafter, the product was filtered under reduced pressure, and sufficiently washed with RO water and acetone. Thereafter, the product was completely dried under reduced pressure in a vacuum oven (80°C, 1 hour) to obtain a dark gray amorphous powder, which was subjected to analysis without further treatment.

### (Example 4) Acetylation of 15 kDa recombinant protein

A recombinant protein PRT2841 (SEQ ID NO: 9, 500 mg, 28.1 µmol), Ac₂O (316 µL, 3.34 mmol, 3.5 equivalents per hydroxy group), and DIPEA (333 µL, 1.91 mmol, 2 equivalents per hydroxy group) were put in a 10 mL stainless steel cup (manufactured by Retsch GmbH), 20 stainless steel balls (diameter: 5 mm, manufactured by Retsch GmbH) were added thereto, and the mixture was milled (frequency: 30 Hz, 90 minutes) with a mixer mill type ball mill (trade name: mixer mill MM400, manufactured by Retsch GmbH) to obtain a light gray massive solid. The reaction mixture was removed from the grinding jar and suspended in RO water (25 mL) while being stirred (10 minutes). Thereafter, the product was filtered under reduced pressure, and sufficiently washed with RO water and acetone. Thereafter, the product was completely dried under reduced pressure in a vacuum oven (80°C, 1 hour) to obtain a pale gray amorphous powder, which was subjected to analysis without further treatment.

### (Example 5) Acetylation of 25 kDa recombinant protein

A recombinant protein PRT2662 (SEQ ID NO: 10, 500 mg, 18.2 µmol), Ac₂O (331 µL, 3.50 mmol, 3.5 equivalents per hydroxy group), and DIPEA (349 µL, 2.00 mmol, 2 equivalents per hydroxy group) were put in a 10 mL stainless steel cup (manufactured by Retsch GmbH), 20 stainless steel balls (diameter: 5 mm, manufactured by Retsch GmbH) were added thereto, and the mixture was milled (frequency: 30 Hz, 90 minutes) with a mixer mill type ball mill (trade name: mixer mill MM400, manufactured by Retsch GmbH) to obtain a pale gray waxy solid. The reaction mixture was removed from the grinding jar and suspended in RO water (25 mL) while being stirred (10 minutes). Thereafter, the product was filtered under reduced pressure, and sufficiently washed with RO water and acetone. Thereafter, the product was completely dried under reduced pressure in a vacuum oven (80°C, 1 hour) to obtain a pale gray amorphous powder, which was subjected to analysis without further treatment.

### (Example 6) Acetylation of 50 kDa recombinant protein

A recombinant protein PRT1000 (500 mg, 9.44 µmol), Ac₂O (319 µL, 3.37 mmol, 3.5 equivalents per hydroxy group), and DIPEA (336 µL, 1.93 mmol, 2 equivalents per hydroxy group) were put in a 10 mL stainless steel cup (manufactured by Retsch GmbH), and the mixture was milled (frequency: 30 Hz, 90 minutes) using 20 stainless steel balls (diameter: 5 mm, manufactured by Retsch GmbH) with a mixer mill type ball mill (trade name: mixer mill MM400, manufactured by Retsch GmbH) to obtain a white amorphous solid. The reaction mixture was removed from the grinding jar and suspended in RO water (25 mL) while being stirred (10 minutes). Thereafter, the product was filtered under reduced pressure, and sufficiently washed with RO water and acetone. Thereafter, the product was completely dried under reduced pressure in a vacuum oven (80°C, 1 hour) to obtain a white amorphous powder, which was directly subjected to analysis.

### (Example 7) Acetylation of 50 kDa recombinant protein

A recombinant protein PRT918 (500 mg, 9.48 µmol), Ac₂O (314 µL, 3.32 mmol, 3.5 equivalents per hydroxy group), and DIPEA (330 µL, 1.90 mmol, 2 equivalents per hydroxy group) were put in a 10 mL stainless steel cup (manufactured by Retsch GmbH), 20 stainless steel balls (diameter: 5 mm, manufactured by Retsch GmbH) were added thereto, and the mixture was milled (frequency: 30 Hz, 90 minutes) with a mixer mill type ball mill (trade name: mixer mill MM400, manufactured by Retsch GmbH) to obtain a white amorphous solid. The reaction mixture was removed from the grinding jar and suspended in RO water (25 mL) while being stirred (10 minutes). Thereafter, the product was filtered under reduced pressure, and sufficiently washed with RO water and acetone. Thereafter, the product was completely dried under reduced pressure in a vacuum oven (80°C, 1 hour) to obtain a white amorphous powder, which was directly subjected to analysis.

### (Example 8) Acetylation of 100 kDa recombinant protein

A recombinant protein PRT966 (500 mg, 4.80 µmol), Ac₂O (311 µL, 3.30 mmol, 3.5 equivalents per hydroxy group), and DIPEA (328 µL, 1.88 mmol, 2 equivalents per hydroxy group) were put in a 10 mL stainless steel cup (manufactured by Retsch GmbH), 20 stainless steel balls (diameter: 5 mm, manufactured by Retsch GmbH) were added thereto, and the mixture was milled (frequency: 30 Hz, 90 minutes) with a mixer mill type ball mill (trade name: mixer mill MM400, manufactured by Retsch GmbH) to obtain a light brown paste. The reaction mixture was removed from the grinding jar and suspended in RO water (25 mL) while being stirred (10 minutes). Thereafter, the product was filtered under reduced pressure, and sufficiently washed with RO water and acetone. Thereafter, the product was completely dried under reduced pressure in a vacuum oven (80°C, 1 hour) to obtain a pale beige amorphous powder, which was directly subjected to analysis.

### (Example 9) Acetylation of 200 kDa recombinant protein

A recombinant protein PRT799 (500 mg, 2.37 µmol), Ac₂O (305 µL, 3.23 mmol, 3.5 equivalents per hydroxy group), and DIPEA (321 µL, 1.84 mmol, 2 equivalents per hydroxy group) were put in a 10 mL stainless steel cup (manufactured by Retsch GmbH), 20 stainless steel balls (diameter: 5 mm, manufactured by Retsch GmbH) were added thereto, and the mixture was milled (frequency: 30 Hz, 90 minutes) with a mixer mill type ball mill (trade name: mixer mill MM400, manufactured by Retsch GmbH) to obtain a pale gray gummy solid. The reaction mixture was removed from the grinding jar and suspended in RO water (25 mL) while being stirred (10 minutes). Thereafter, the product was filtered under reduced pressure, and sufficiently washed with RO water and acetone. Thereafter, the product was completely dried under reduced pressure in a vacuum oven (80°C, 1 hour) to obtain a pale gray amorphous powder, which was subjected to analysis without further treatment.

### (Example 10) Acetylation of 300 kDa recombinant protein

A recombinant protein PRT1186 (SEQ ID NO: 11, 500 mg, 1.59 µmol), Ac₂O (307 µL, 3.25 mmol, 3.5 equivalents per hydroxy group), and DIPEA (324 µL, 1.86 mmol, 2 equivalents per hydroxy group) were put in a 10 mL stainless steel cup (manufactured by Retsch GmbH), 20 stainless steel balls (diameter: 5 mm, manufactured by Retsch GmbH) were added thereto, and the mixture was milled (frequency: 30 Hz, 90 minutes) with a mixer mill type ball mill (trade name: mixer mill MM400, manufactured by Retsch GmbH) to obtain a light gray gummy solid. The reaction mixture was removed from the grinding jar and suspended in RO water (25 mL) while being stirred (10 minutes). Thereafter, the product was filtered under reduced pressure, and sufficiently washed with RO water and acetone. Thereafter, the product was completely dried under reduced pressure in a vacuum oven (80°C, 1 hour) to obtain a white amorphous solid, which was directly subjected to analysis.

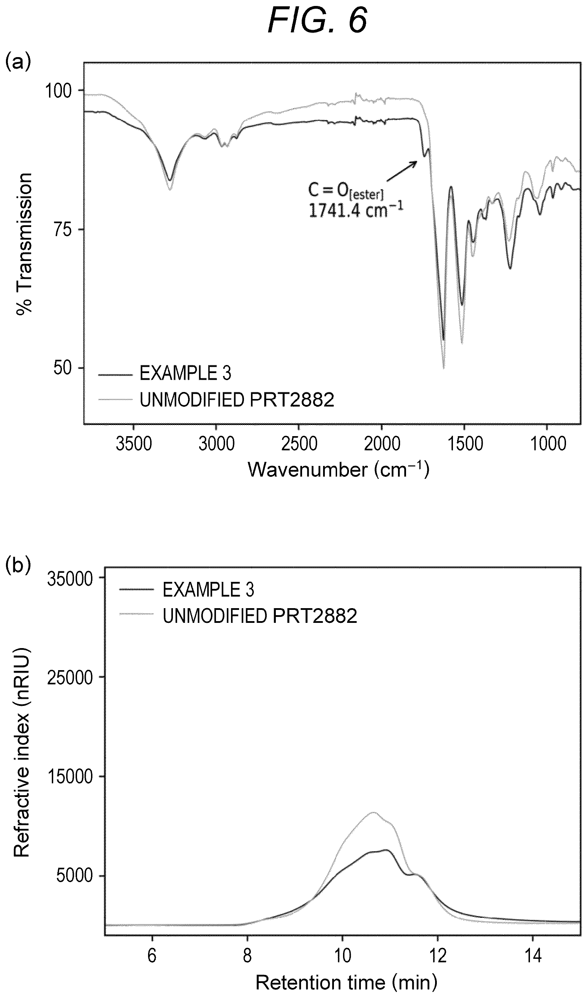

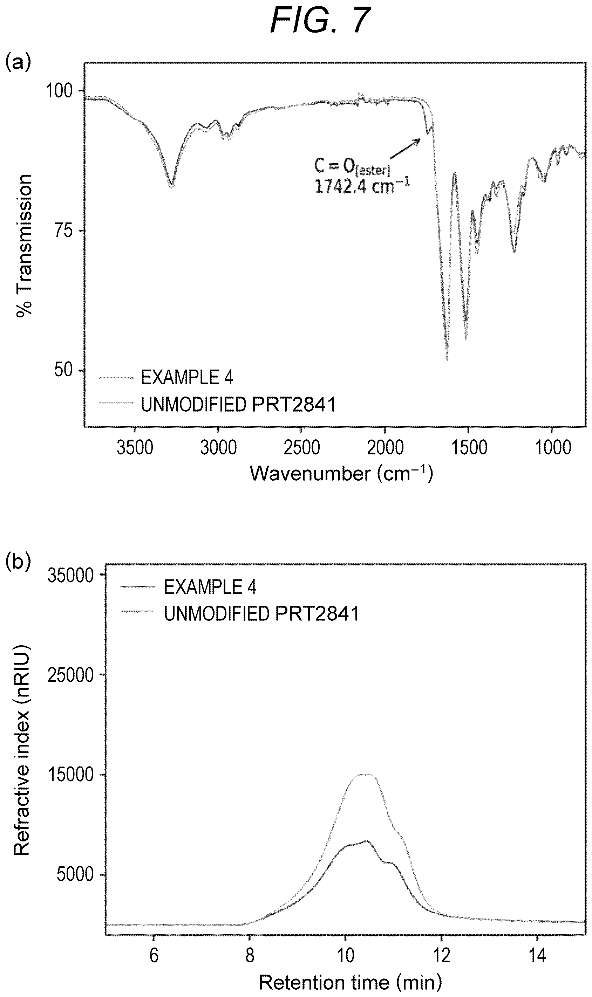

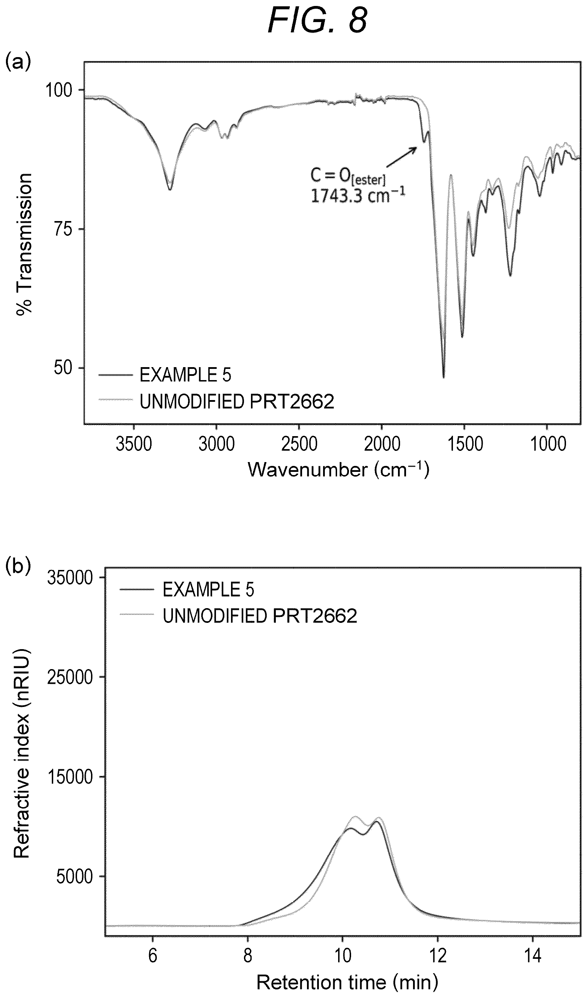

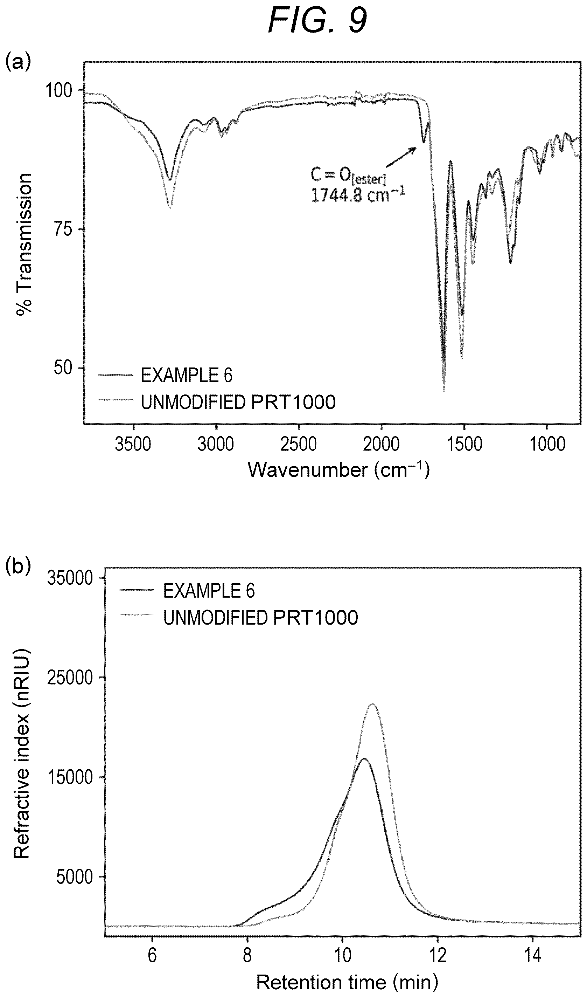

IR spectra and results of GPC analysis of Examples 3 to 10 are illustrated in Figs. 6 to 13, respectively. Fig. 6(a) illustrates IR spectra of Example 3 and the unmodified protein PRT2882, and a peak appeared at 1741 cm⁻¹, indicating that acetylation proceeded. Fig. 6(b) illustrates GPC chromatograms of Example 3 and the unmodified protein PRT2882, and the retention time was slightly shorter than that of the unmodified protein. Fig. 7(a) illustrates IR spectra of Example 4 and the unmodified protein PRT2841, and a peak appeared at 1742 cm⁻¹, indicating that acetylation proceeded. Fig. 7(b) illustrates GPC chromatograms of Example 4 and the unmodified protein PRT2841, and the retention time was slightly shorter than that of the unmodified protein. Fig. 8(a) illustrates IR spectra of Example 5 and the unmodified protein PRT2662, and a peak appeared at 1743 cm⁻¹, indicating that acetylation proceeded. Fig. 8(b) illustrates GPC chromatograms of Example 5 and the unmodified protein PRT2662, and the retention time was slightly shorter than that of the unmodified protein. Fig. 9(a) illustrates IR spectra of Example 6 and the unmodified protein PRT1000, and a peak appeared at 1744 cm⁻¹, indicating that acetylation proceeded. Fig. 9(b) illustrates GPC chromatograms of Example 6 and the unmodified protein PRT1000, and the retention time was slightly shorter than that of the unmodified protein.

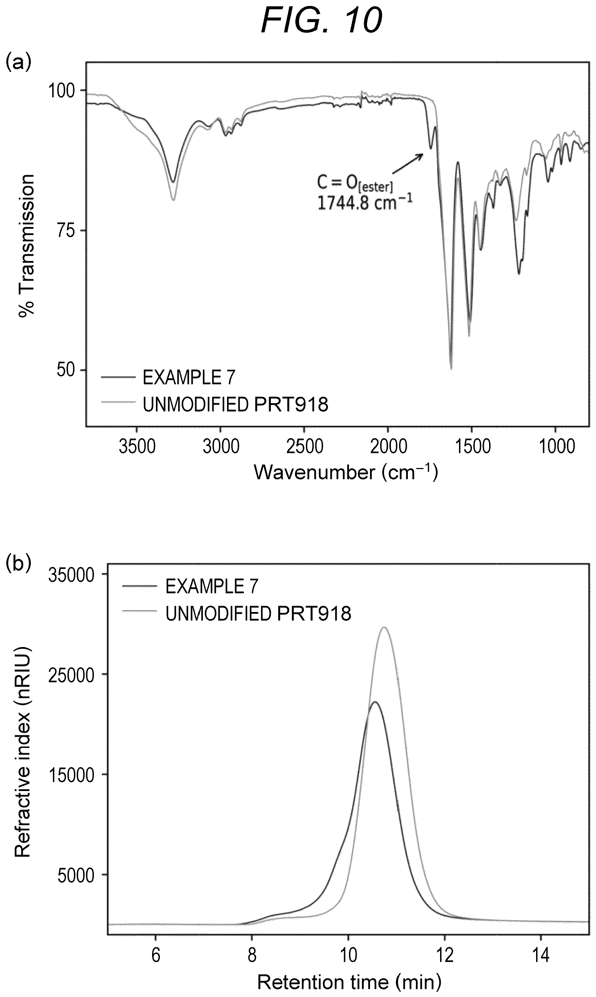

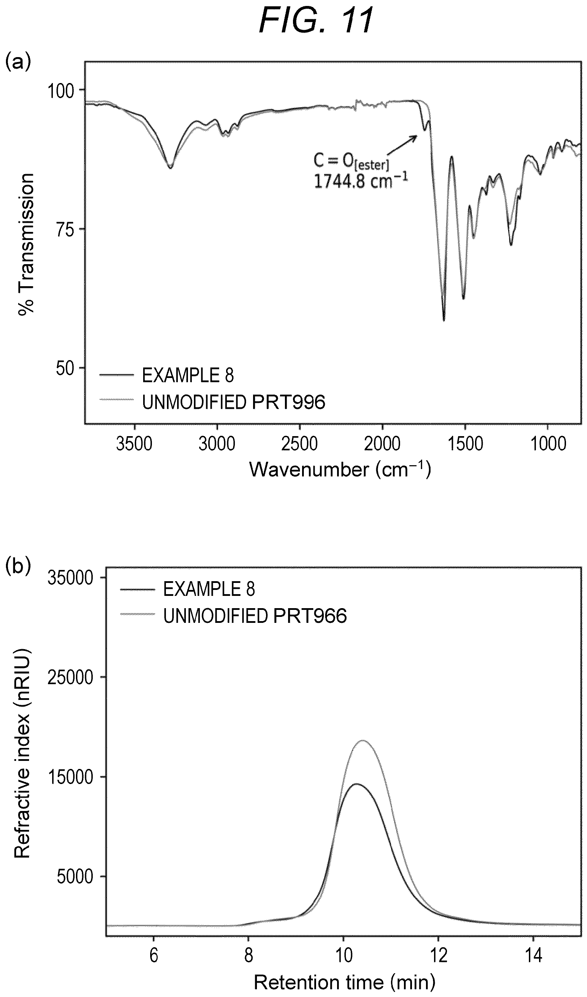

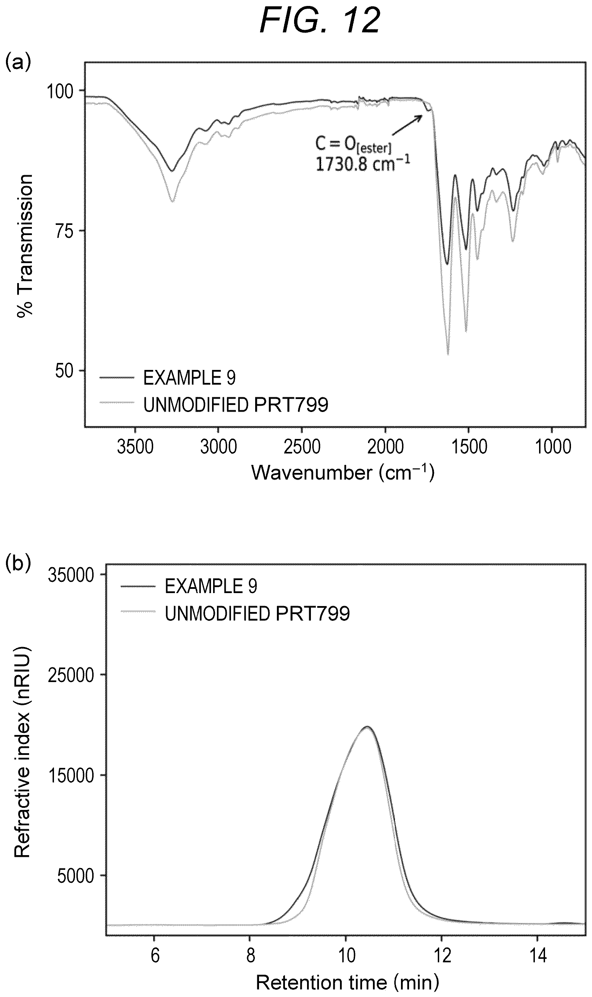

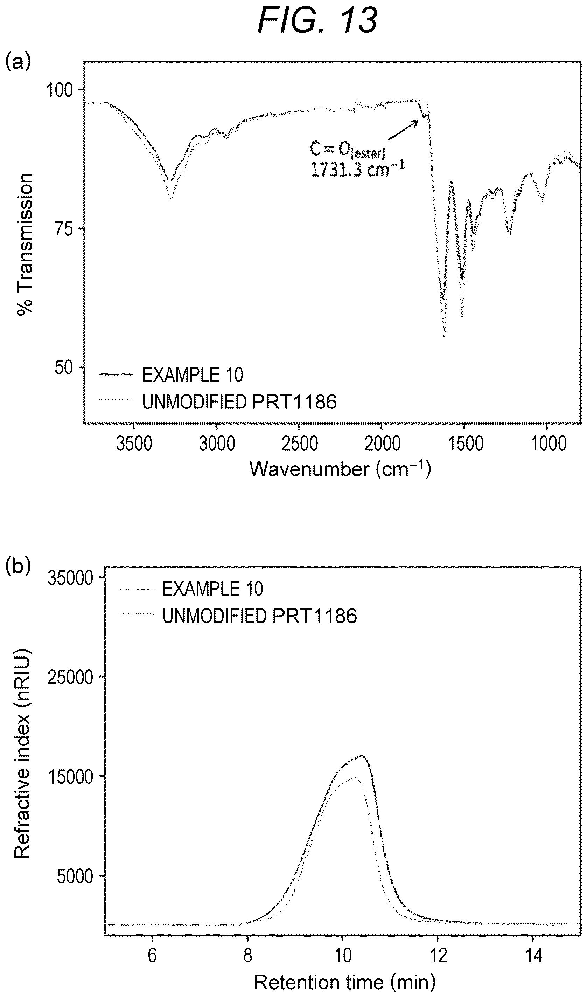

Fig. 10(a) illustrates IR spectra of Example 7 and the unmodified protein PRT918, and a peak appeared at 1744 cm⁻¹, indicating that acetylation proceeded. Fig. 10(b) illustrates GPC chromatograms of Example 7 and the unmodified protein PRT918, and the retention time was slightly shorter than that of the unmodified protein. Fig. 11(a) illustrates IR spectra of Example 8 and the unmodified protein PRT966, and a peak appeared at 1744 cm⁻¹, indicating that acetylation proceeded. Fig. 11(b) illustrates GPC chromatograms of Example 8 and the unmodified protein PRT966, and the retention time was slightly shorter than that of the unmodified protein. Fig. 12(a) illustrates IR spectra of Example 9 and the unmodified protein PRT799, and a peak appeared at 1730 cm⁻¹, indicating that acetylation proceeded. Fig. 12(b) illustrates GPC chromatograms of Example 9 and the unmodified protein PRT799, and the retention time was slightly shorter than that of the unmodified protein. Fig. 13(a) illustrates IR spectra of Example 10 and the unmodified protein PRT1186, and a peak appeared at 1731 cm⁻¹, indicating that acetylation proceeded. Fig. 13(b) illustrates GPC chromatograms of Example 10 and the unmodified protein PRT1186, and the retention time was slightly shorter than that of the unmodified protein.

### 5. Influence of type of base

### (Example 11) Acetylation of 100 kDa recombinant protein

A recombinant protein PRT966 (500 mg, 4.80 µmol), Ac₂O (311 µL, 3.30 mmol, 3.5 equivalents per hydroxy group), and sodium hydroxide (41.5 mg, 1.04 mmol, 1.1 equivalents per hydroxy group) were put in a 10 mL stainless steel cup (manufactured by Retsch GmbH), 20 stainless steel balls (diameter: 5 mm, manufactured by Retsch GmbH) were added thereto, and the mixture was milled (frequency: 30 Hz, 90 minutes) with a mixer mill type ball mill (trade name: mixer mill MM400, manufactured by Retsch GmbH) to obtain a white amorphous solid. The reaction mixture was removed from the grinding jar and suspended in RO water (25 mL) while being stirred (10 minutes). Thereafter, the product was filtered under reduced pressure, and sufficiently washed with RO water and acetone. Thereafter, the product was completely dried under reduced pressure in a vacuum oven (80°C, 1 hour) to obtain a white amorphous fine powder, which was directly subjected to analysis.

### (Example 12) Acetylation of 100 kDa recombinant protein

A recombinant protein PRT966 (500 mg, 4.80 µmol), Ac₂O (311 µL, 3.30 mmol, 3.5 equivalents per hydroxy group), and sodium carbonate (110 mg, 1.04 mmol, 1.1 equivalents per hydroxy group) were put in a 10 mL stainless steel cup (manufactured by Retsch GmbH), 20 stainless steel balls (diameter: 5 mm, manufactured by Retsch GmbH) were added thereto, and the mixture was milled (frequency: 30 Hz, 90 minutes) with a mixer mill type ball mill (trade name: mixer mill MM400, manufactured by Retsch GmbH) to obtain a white amorphous solid. The reaction mixture was removed from the grinding jar and suspended in RO water (25 mL) while being stirred (10 minutes). Thereafter, the product was filtered under reduced pressure, and sufficiently washed with RO water and acetone. Thereafter, the product was completely dried under reduced pressure in a vacuum oven (80°C, 1 hour) to obtain a white amorphous fine powder, which was directly subjected to analysis.

### (Example 13) Acetylation of 100 kDa recombinant protein

A recombinant protein PRT966 (500 mg, 4.80 µmol), Ac₂O (445 µL, 4.71 mmol, 5 equivalents per hydroxy group), DIPEA (820 µL, 4.71 mmol, 5 equivalents per hydroxy group), and DMAP (575 mg, 4.71 mmol, 5 equivalents per hydroxy residue) were put in a 10 mL stainless steel cup (manufactured by Retsch GmbH), 20 stainless steel balls (diameter: 5 mm, manufactured by Retsch GmbH) were added thereto, and the mixture was milled (frequency: 30 Hz, 8.6 minutes) with a mixer mill type ball mill (trade name: mixer mill MM400, manufactured by Retsch GmbH) to obtain a blackish brown waxy solid. The reaction mixture was removed from the grinding jar and suspended in RO water while being stirred (10 minutes). Thereafter, the product was filtered under reduced pressure, and sufficiently washed with RO water and acetone. Thereafter, the product was completely dried under reduced pressure in a vacuum oven (80°C, 1 hour) to obtain a brown amorphous powder, which was directly subjected to analysis.

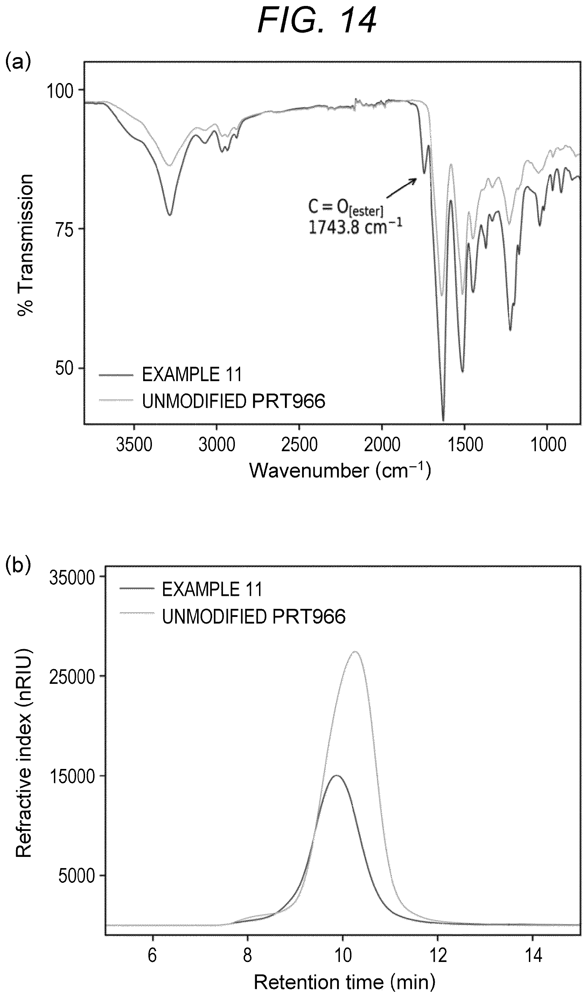

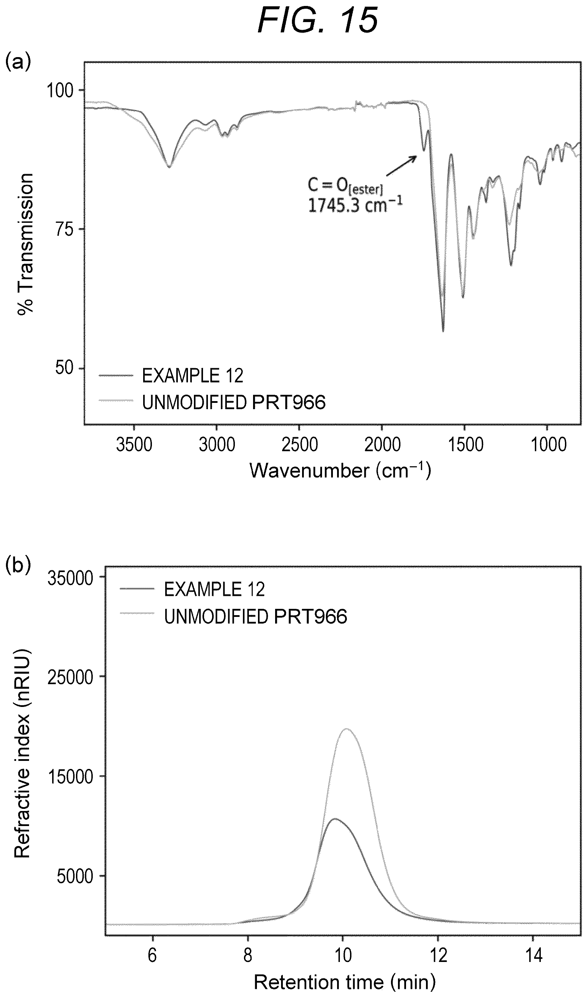

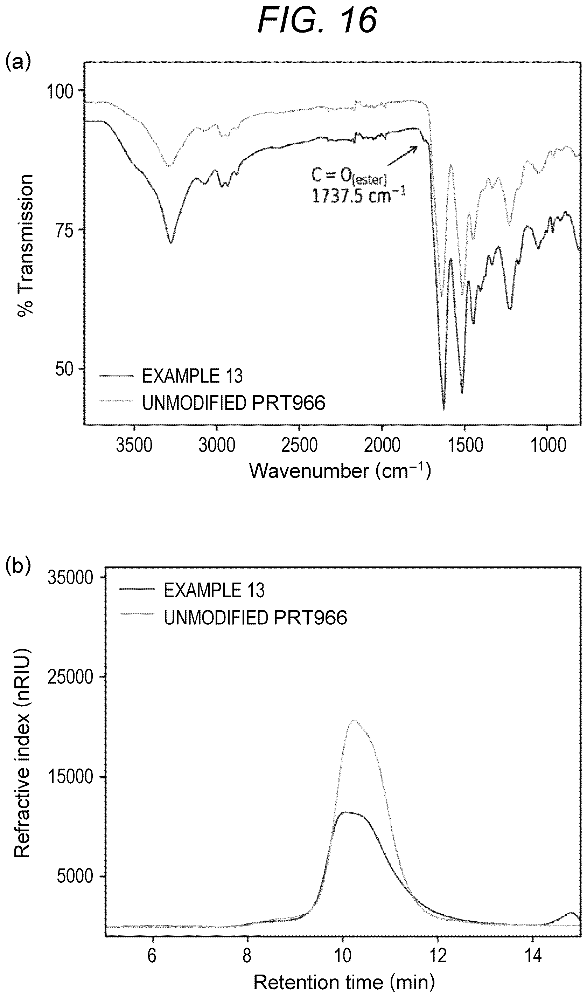

IR spectra and results of GPC analysis of Examples 11 to 13 are illustrated in Figs. 14 to 16, respectively. Fig. 14(a) illustrates IR spectra of Example 11 and the unmodified protein PRT966, and a peak appeared at 1743 cm⁻¹, indicating that acetylation proceeded. Fig. 14(b) illustrates GPC chromatograms of Example 11 and the unmodified protein PRT966, and the retention time was slightly shorter than that of the unmodified protein. Fig. 15(a) illustrates IR spectra of Example 12 and the unmodified protein PRT966, and a peak appeared at 1745 cm⁻¹, indicating that acetylation proceeded. Fig. 15(b) illustrates GPC chromatograms of Example 12 and the unmodified protein PRT966, and the retention time was slightly shorter than that of the unmodified protein. Fig. 16(a) illustrates IR spectra of Example 13 and the unmodified protein PRT966, and a peak appeared at 1737 cm⁻¹, indicating that acetylation proceeded. Fig. 16(b) illustrates GPC chromatograms of Example 13 and the unmodified protein PRT966, and the retention time was slightly shorter than that of the unmodified protein.

### 6. Succinylation by mechanochemical method

### (Example 14) Succinylation of 100 kDa recombinant protein

A recombinant protein PRT966 (500 mg, 4.80 µmol), succinic anhydride (330 mg, 3.30 mmol, 3.5 equivalents per hydroxy group), DIPEA (181 µL, 1.04 mmol, 1.1 equivalents per hydroxy group), and DMAP (127 mg, 1.04 mmol, 1.1 equivalents per hydroxy group) were put in a 10 mL stainless steel cup (manufactured by Retsch GmbH), 20 stainless steel balls (diameter: 5 mm, manufactured by Retsch GmbH) were added thereto, and the mixture was milled (frequency: 30 Hz, 90 minutes) with a mixer mill type ball mill (trade name: mixer mill MM400, manufactured by Retsch GmbH) to obtain a weak yellowish white amorphous solid. The reaction mixture was removed from the grinding jar and suspended in RO water (25 mL) while being stirred (10 minutes). Thereafter, the product was filtered under reduced pressure, and sufficiently washed with RO water and acetone. Thereafter, the product was completely dried under reduced pressure in a vacuum oven (80°C, 1 hour) to obtain a white amorphous solid, which was directly subjected to analysis.

### (Example 15) Succinylation of 100 kDa recombinant protein

A recombinant protein PRT966 (500 mg, 4.80 µmol), succinic anhydride (330 mg, 3.30 mmol, 3.5 equivalents per hydroxy group), DIPEA (181 µL, 1.04 mmol, 1.1 equivalents per hydroxy group), and DBU (155 µL, 1.04 mmol, 1.1 equivalents per hydroxy group) were put in a 10 mL stainless steel cup (manufactured by Retsch GmbH), 20 stainless steel balls (diameter: 5 mm, manufactured by Retsch GmbH) were added thereto, and the mixture was milled (frequency: 30 Hz, 90 minutes) with a mixer mill type ball mill (trade name: mixer mill MM400, manufactured by Retsch GmbH) to obtain a sticky blackish brown paste. The reaction mixture was removed from the grinding jar and suspended in RO water (25 mL) while being stirred (10 minutes). Thereafter, the product was filtered under reduced pressure, and sufficiently washed with RO water and acetone. Thereafter, the product was completely dried under reduced pressure in a vacuum oven (80°C, 1 hour) to obtain a grayish brown amorphous solid, which was directly subjected to analysis.

### (Example 16) Succinylation of 100 kDa recombinant protein

A recombinant protein PRT966 (500 mg, 4.80 µmol), succinic anhydride (330 mg, 3.30 mmol, 3.5 equivalents per hydroxy group), and potassium carbonate (455 mg, 3.30 mmol, 3.5 equivalents per hydroxy group) were added to a 10 mL stainless steel cup (manufactured by Retsch GmbH), 20 stainless steel balls (diameter: 5 mm, manufactured by Retsch GmbH) were put therein, and the mixture was milled (frequency: 30 Hz, 90 minutes) with a mixer mill type ball mill (trade name: mixer mill MM400, manufactured by Retsch GmbH) to obtain a weak yellowish white waxy solid. The reaction mixture was removed from the grinding jar and suspended in RO water (25 mL) while being stirred (10 minutes). Thereafter, the product was filtered under reduced pressure, and sufficiently washed with RO water and acetone. Thereafter, the product was completely dried under reduced pressure in a vacuum oven (80°C, 1 hour) to obtain a weak yellowish white amorphous powder, which was directly subjected to analysis.

### (Example 17) Succinylation of 100 kDa recombinant protein

A recombinant protein PRT966 (500 mg, 4.80 µmol), succinic anhydride (471 mg, 4.71 mmol, 5 equivalents per hydroxy group), DIPEA (820 µL, 4.71 mmol, 5 equivalents per hydroxy group), and DMAP (575 mg, 4.71 mmol, 5 equivalents per hydroxy group) were put in a 10 mL stainless steel cup (manufactured by Retsch GmbH), 20 stainless steel balls (diameter: 5 mm, manufactured by Retsch GmbH) were added thereto, and the mixture was milled (frequency: 30 Hz, 8.6 hours) with a mixer mill type ball mill (trade name: mixer mill MM400, manufactured by Retsch GmbH) to obtain a chalky amorphous solid. The reaction mixture was removed from the grinding jar and suspended in RO water (25 mL) while being stirred (10 minutes). Thereafter, the product was filtered under reduced pressure, and sufficiently washed with RO water and acetone. Thereafter, the product was completely dried under reduced pressure in a vacuum oven (80°C, 1 hour) to obtain a white amorphous powder, which was directly subjected to analysis.

### (Example 18) Succinylation of 100 kDa recombinant protein

A recombinant protein PRT966 (500 mg, 4.80 µmol), succinic anhydride (471 mg, 4.71 mmol, 5 equivalents per hydroxy group), DIPEA (820 µL, 4.71 mmol, 5 equivalents per hydroxy group), and DMAP (575 mg, 4.71 mmol, 5 equivalents per hydroxy group) were put in a 10 mL stainless steel cup (manufactured by Retsch GmbH), 20 stainless steel balls (diameter: 5 mm, manufactured by Retsch GmbH) were added thereto, and the mixture was milled (frequency: 20 Hz, 2 hours) with a mixer mill type ball mill (trade name: mixer mill MM400, manufactured by Retsch GmbH) to obtain a weak yellowish white paste. The reaction mixture was removed from the grinding jar and suspended in RO water (25 mL) while being stirred (10 minutes). Thereafter, the product was filtered under reduced pressure, and sufficiently washed with RO water and acetone. Thereafter, the product was completely dried under reduced pressure in a vacuum oven (80°C, 1 hour) to obtain a white amorphous powder, which was directly subjected to analysis.

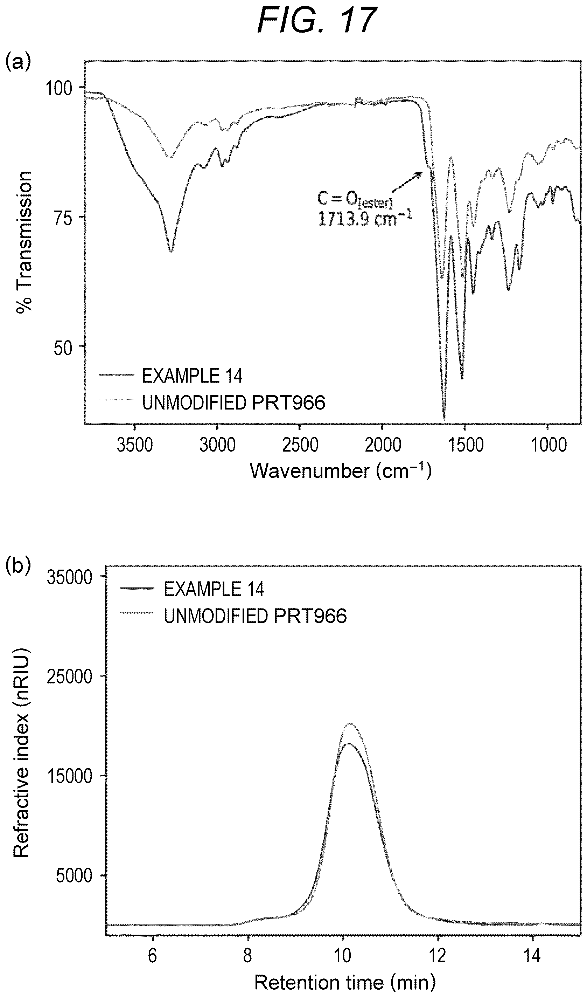

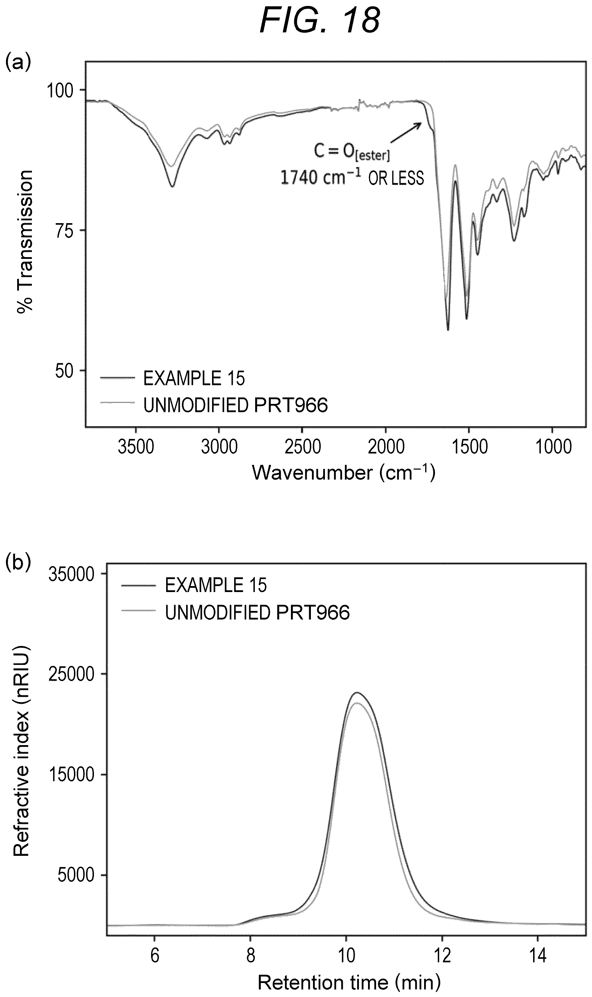

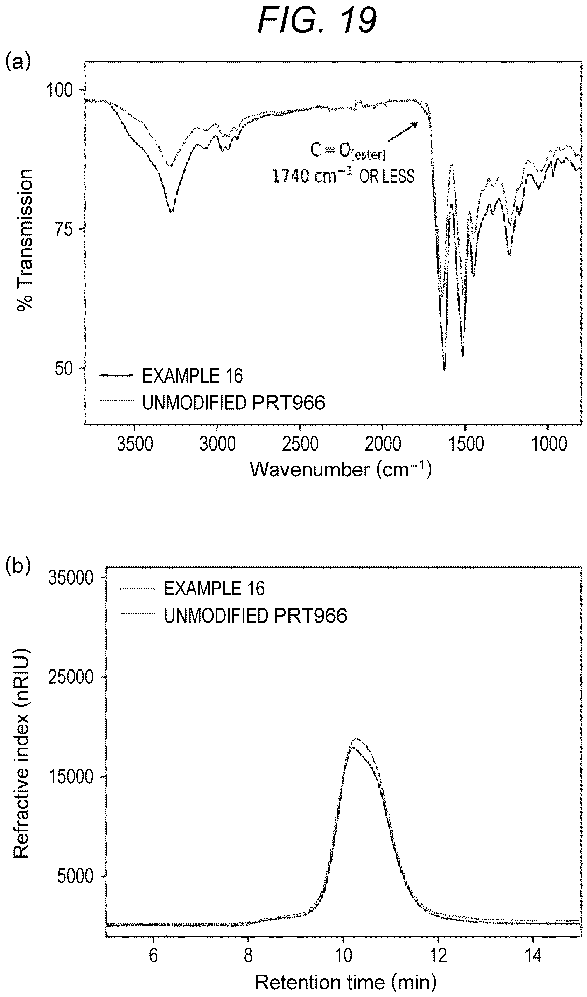

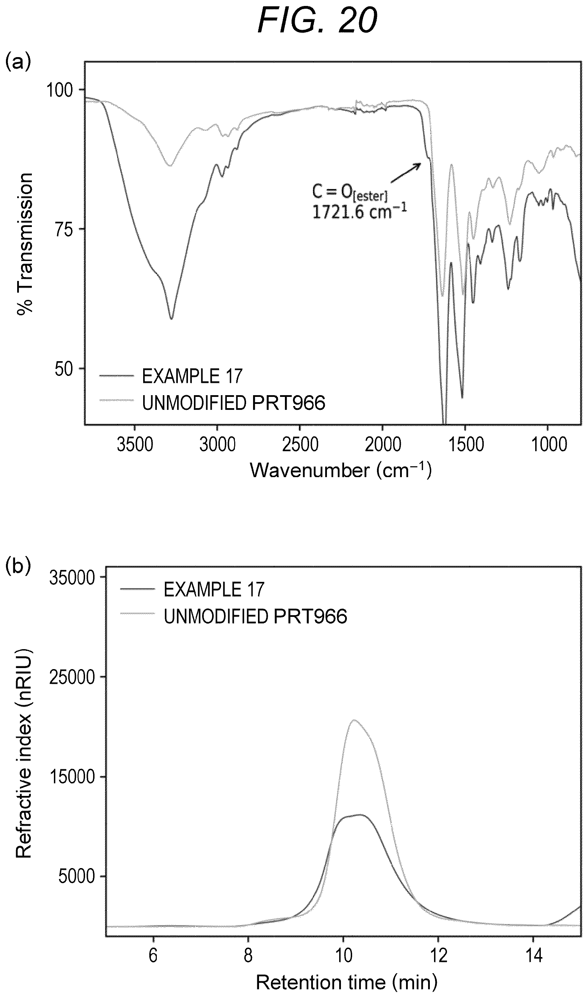

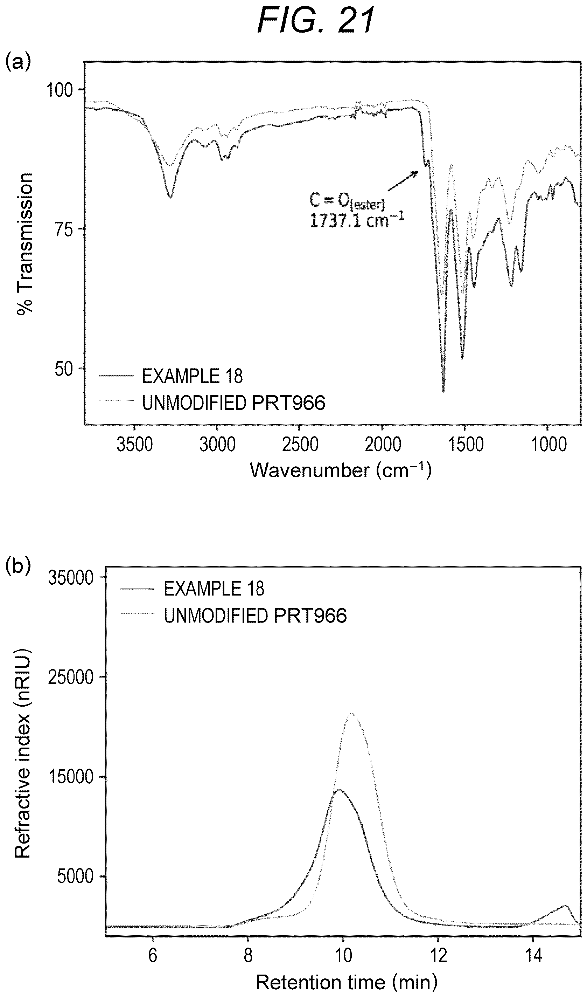

IR spectra and results of GPC analysis of Examples 14 to 18 are illustrated in Figs. 17 to 21, respectively. Fig. 17(a) illustrates IR spectra of Example 14 and the unmodified protein PRT966, and a peak appeared at 1713 cm⁻¹, indicating that succinylation proceeded. Fig. 17(b) illustrates GPC chromatograms of Example 14 and the unmodified protein PRT966, and the retention time was almost the same as that of the unmodified protein. Fig. 18(a) illustrates IR spectra of Example 15 and the unmodified protein PRT966, and a peak appeared at 1740 cm⁻¹, indicating that succinylation proceeded. Fig. 18(b) illustrates GPC chromatograms of Example 15 and the unmodified protein PRT966, and the retention time was almost the same as that of the unmodified protein. Fig. 19(a) illustrates IR spectra of Example 16 and the unmodified protein PRT966, and a peak appeared at 1740 cm⁻¹, indicating that succinylation proceeded. Fig. 19(b) illustrates GPC chromatograms of Example 16 and the unmodified protein PRT966, and the retention time was almost the same as that of the unmodified protein. Fig. 20(a) illustrates IR spectra of Example 17 and the unmodified protein PRT966, and a peak appeared at 1721 cm⁻¹, indicating that succinylation proceeded. Fig. 20(b) illustrates GPC chromatograms of Example 17 and the unmodified protein PRT966, and the retention time was almost the same as that of the unmodified protein. Fig. 21(a) illustrates IR spectra of Example 18 and the unmodified protein PRT966, and a peak appeared at 1737 cm⁻¹, indicating that succinylation proceeded. Fig. 21(b) illustrates GPC chromatograms of Example 18 and the unmodified protein PRT966, and the retention time was slightly shorter than that of the unmodified protein.

### 7. Stearylation by mechanochemical method

### (Example 19) Stearylation of 100 kDa recombinant protein

A recombinant protein PRT966 (500 mg, 4.80 µmol), stearic anhydride (1.82 g, 3.30 mmol, 3.5 equivalents per hydroxy group), and DIPEA (574 µL, 3.30 mmol, 3.5 equivalents per hydroxy group) were put in a 10 mL stainless steel cup (manufactured by Retsch GmbH), 20 stainless steel balls (diameter: 5 mm, manufactured by Retsch GmbH) were added thereto, and the mixture was milled (frequency: 30 Hz, 90 minutes) with a mixer mill type ball mill (trade name: mixer mill MM400, manufactured by Retsch GmbH) to obtain a waxy amorphous solid. The reaction mixture was removed from the grinding jar and suspended in RO water (25 mL) while being stirred (10 minutes). Next, the crude product was filtered under reduced pressure, and sufficiently washed with RO water and acetone. Subsequently, the product was partially dried on filter paper, suspended in ethyl acetate (25 mL), and stirred using a magnetic stirrer (10 minutes). Thereafter, the product was collected by vacuum filtration and sufficiently washed with ethyl acetate. Thereafter, a white amorphous powder was obtained in a vacuum oven (80°C, 1 hour), and was directly subjected to analysis.

### (Example 20) Stearylation of 100 kDa recombinant protein

A recombinant protein PRT966 (500 mg, 4.80 µmol), stearoyl chloride (998 mg, 3.30 mmol, 3.5 equivalents per hydroxy group), and DIPEA (574 µL, 3.30 mmol, 3.5 equivalents per hydroxy group) were put in a 10 mL stainless steel cup (manufactured by Retsch GmbH), 20 stainless steel balls (diameter: 5 mm, manufactured by Retsch GmbH) were added thereto, and the mixture was milled (frequency: 30 Hz, 90 minutes) with a mixer mill type ball mill (trade name: mixer mill MM400, manufactured by Retsch GmbH) to obtain a waxy yellow paste. The reaction mixture was removed from the grinding jar and suspended in RO water (25 mL) while being stirred (10 minutes). Next, the crude product was filtered under reduced pressure, and sufficiently washed with RO water and acetone. Subsequently, the product was partially dried on filter paper, suspended in ethyl acetate (25 mL), and stirred using a magnetic stirrer (10 minutes). Thereafter, the product was collected by vacuum filtration, sufficiently washed with ethyl acetate, and then dried in a vacuum oven (80°C, 1 hour) to obtain a weak yellowish white amorphous powder, which was directly subjected to analysis.

### (Example 21) Stearylation of 100 kDa recombinant protein

A recombinant protein PRT966 (500 mg, 4.80 µmol), stearic anhydride (1.82 g, 3.30 mmol, 3.5 equivalents per hydroxy group), DIPEA (574 µL, 3.30 mmol, 3.5 equivalents per hydroxy group), and DMAP (403 mg, 3.30 mmol, 3.5 equivalents per hydroxy group) were put in a 10 mL stainless steel cup (manufactured by Retsch GmbH), 20 stainless steel balls (diameter: 5 mm, manufactured by Retsch GmbH) were added thereto, and the mixture was milled (frequency: 30 Hz, 90 minutes) with a mixer mill type ball mill (trade name: mixer mill MM400, manufactured by Retsch GmbH) to obtain a waxy amorphous solid. The reaction mixture was removed from the grinding jar and suspended in RO water (25 mL) while being stirred (10 minutes). Next, the crude product was filtered under reduced pressure, and sufficiently washed with RO water and acetone. Subsequently, the product was partially dried on filter paper, suspended in ethyl acetate (25 mL), and stirred using a magnetic stirrer (10 minutes). Thereafter, the product was collected by filtration under reduced pressure, sufficiently washed with ethyl acetate, and then dried in a vacuum oven (80°C, 1 hour) to obtain a white amorphous powder, which was directly subjected to analysis.

### (Example 22) Stearylation of 100 kDa recombinant protein

A recombinant protein PRT966 (500 mg, 4.80 µmol), stearoyl chloride (998 mg, 3.30 mmol, 3.5 equivalents per hydroxy group), DIPEA (574 µL, 3.30 mmol, 3.5 equivalents per hydroxy group), and DMAP (403 mg, 3.30 mmol, 3.5 equivalents per hydroxy group) were put in a 10 mL stainless steel cup (manufactured by Retsch GmbH), 20 stainless steel balls (diameter: 5 mm, manufactured by Retsch GmbH) were added thereto, and the mixture was milled (frequency: 30 Hz, 90 minutes) with a mixer mill type ball mill (trade name: mixer mill MM400, manufactured by Retsch GmbH) to obtain a waxy yellow paste. The reaction mixture was removed from the grinding jar and suspended in RO water (25 mL) while being stirred (10 minutes). Next, the crude product was filtered under reduced pressure, and sufficiently washed with RO water and acetone. Subsequently, the product was partially dried on filter paper, suspended in ethyl acetate (25 mL), and stirred using a magnetic stirrer (10 minutes). Thereafter, the product was collected by filtration under reduced pressure, sufficiently washed with ethyl acetate, and then dried in a vacuum oven (80°C, 1 hour) to obtain a beige amorphous powder, which was directly subjected to analysis.

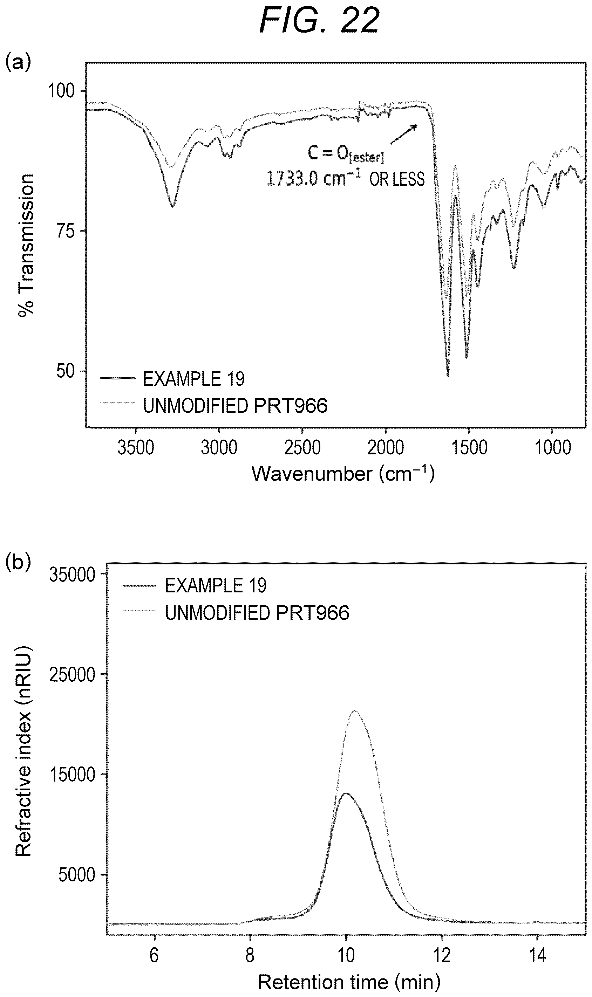

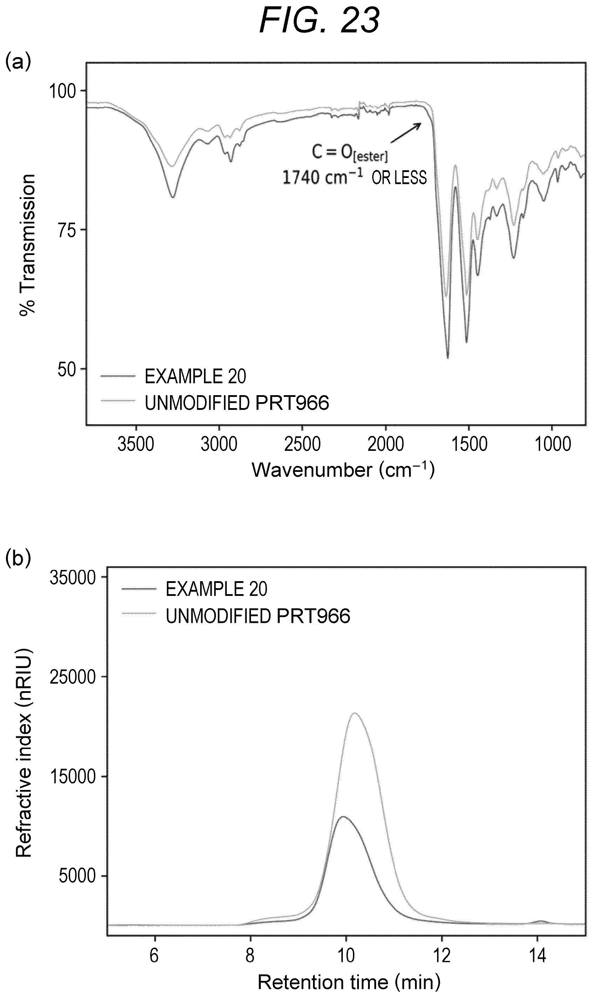

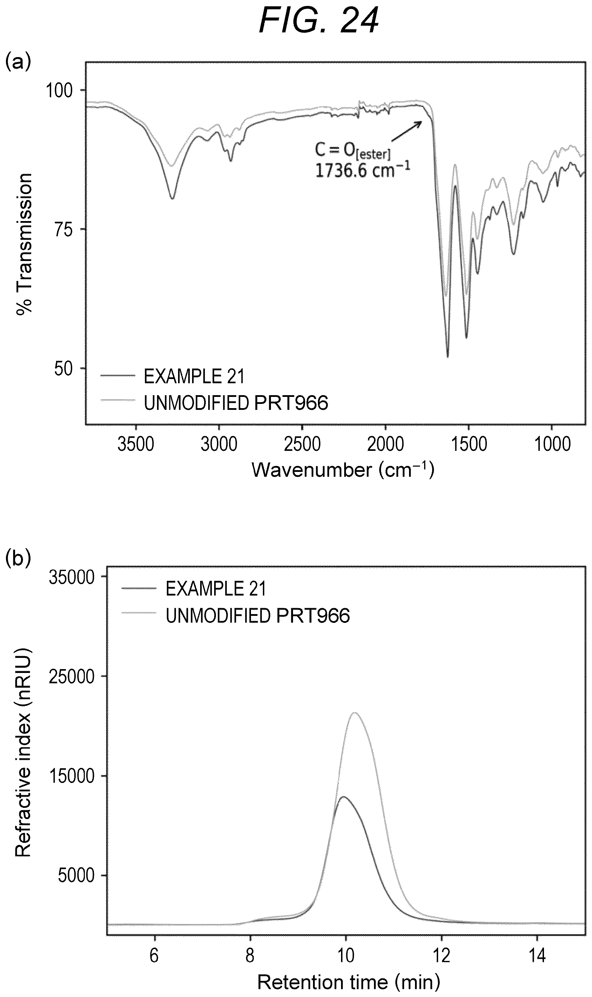

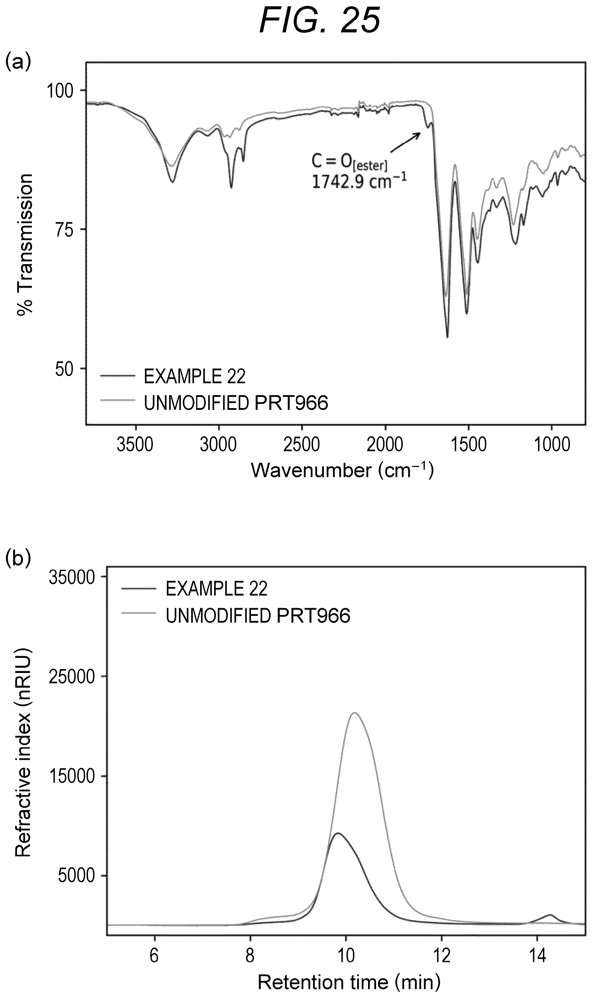

IR spectra and results of GPC analysis of Examples 19 to 22 are illustrated in Figs. 22 to 25, respectively. Fig. 22(a) illustrates IR spectra of Example 19 and the unmodified protein PRT966, and a peak appeared at 1733 cm⁻¹, indicating that stearylation proceeded. Fig. 22(b) illustrates GPC chromatograms of Example 19 and the unmodified protein PRT966, and the retention time was slightly shorter than that of the unmodified protein. Fig. 23(a) illustrates IR spectra of Example 20 and the unmodified protein PRT966, and a peak appeared at 1740 cm⁻¹, indicating that stearylation proceeded. Fig. 23(b) illustrates GPC chromatograms of Example 20 and the unmodified protein PRT966, and the retention time was almost the same as that of the unmodified protein. Fig. 24(a) illustrates IR spectra of Example 21 and the unmodified protein PRT966, and a peak appeared at 1736 cm⁻¹, indicating that stearylation proceeded. Fig. 24(b) illustrates GPC chromatograms of Example 21 and the unmodified protein PRT966, and the retention time was slightly shorter than that of the unmodified protein. Fig. 25(a) illustrates IR spectra of Example 22 and the unmodified protein PRT966, and a peak appeared at 1742 cm⁻¹, indicating that stearylation proceeded. Fig. 25(b) illustrates GPC chromatograms of Example 22 and the unmodified protein PRT966, and the retention time was slightly shorter than that of the unmodified protein.

### 8. Degree of esterification

The degree of esterification of an obtained modified protein was calculated by the following procedure. First, each of a modified protein and an unmodified protein was subjected to FT-IR spectrum analysis. From each of the obtained spectra, a peak area of a C=O stretching vibration peak corresponding to each of a formed ester and an amide bond of a protein was calculated. The peak area of a C=O stretching vibration peak of an ester bond with respect to a peak area of a C=O stretching vibration peak of an amide bond in the modified protein was calculated. Each calculation formula is described below. Ester/amide ratio =[(peak area of ester C=O stretching vibration peak of modified protein) - (peak area of ester C=O stretching vibration peak of unmodified protein)]/(peak area of amide C=O stretching vibration peak of modified protein) Estimated number of esterified residues = ester/amide ratio × total number of amino acid residues in protein Degree of esterification (%) = (estimated number of esterified residues/total number of hydroxy groups) ×100

The degree of esterification of each modified protein is presented in Table 3.

**[Table 3]**

| Example | Modified protein | Degree of esterification (calculated value) |
|---|---|---|
| 1 | 100 kDa Ac-VFI 3 | 6.7 |
| 2 | 100 kDa Ac-VFI 4 | 37.7 |
| 3 | 7.5 kDa Ac-Cys2 | 71.8 |
| 4 | 15 kDa Ac-Cys2 | 35.8 |
| 5 | 25 kDa Ac-Cys2 | 40.2 |
| 6 | 50 kDa Ac-Cys2 | 46.6 |
| 7 | 50 kDa Ac-VFI | 52.3 |
| 8 | 100 kDa Ac-VFI | 38.7 |
| 9 | 200 kDa Ac-QQQ | 12.2 |
| 10 | 300 kDa Ac-Cys1 | 17.4 |
| 11 | 100 kDa Ac-VFI 5 | 59.0 |
| 12 | 100 kDa Ac-VFI 6 | 60.3 |
| 13 | 100 kDa Ac-VFI 7 | 24.5 |
| 14 | 100 kDa Succ-VFI 1 | 56.2 |
| 15 | 100 kDa Succ-VFI 2 | 27.8 |
| 16 | 100 kDa Succ-VFI 3 | 8.4 |
| 17 | 100 kDa Succ-VFI 4 | 45.8 |
| 18 | 100 kDa Succ-VFI 5 | 50.3 |
| 19 | 100 kDa St-VFI 1 | 4.4 |
| 20 | 100 kDa St-VFI 2 | 9.7 |
| 21 | 100 kDa St-VFI 3 | 11.0 |
| 22 | 100 kDa St-VFI 4 | 27.8 |

The degree of esterification is an index indicating how much hydroxy groups of amino acid residues constituting a protein are esterified. Acetylation of a nucleophilic functional group (an amino group or a sulfhydryl group) other than a hydroxy group in a protein forms an amide bond or a thioester bond, and does not affect a C=O stretching vibration peak of an ester.

### (4) Dispersibility of modified protein

Each of a 100 kDa recombinant protein PRT966, the acetylated protein obtained in Example 11, and the succinylated protein obtained in Example 14 was suspended in any one of water, acetone, and a 0.5 w/v% sodium hydroxide aqueous solution (final concentration: 1 wt%) and mixed by a vortex mixer (FRONT LAB FLX-S50, manufactured by AS ONE Corporation) at room temperature (2500 rpm, 30 seconds).

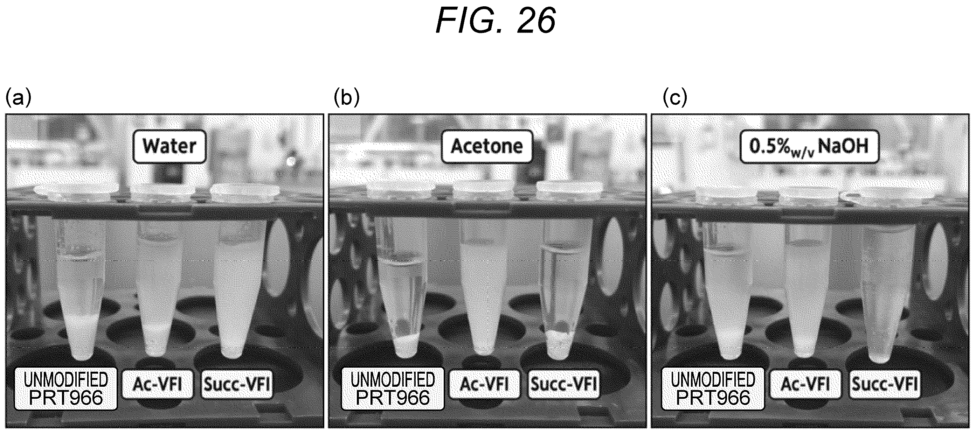

Results thereof are illustrated in Fig. 26 and Table 4. Fig. 26 is a photograph in which a solvent is added to each of the recombinant protein PRT966, the acetylated protein PRT966, and the succinylated protein PRT966. The solvents used in Figs. 26(a), 26(b), and 26(c) are water, acetone, and a 0.5 w/v% sodium hydroxide aqueous solution, respectively. The proteins were not much dispersed in any of the solvents, whereas the acetylated protein was uniformly dispersed in acetone. The succinylated protein was uniformly dispersed in water and dissolved in the alkaline solution.

**[Table 4]**

| | Water | Acetone | NaOH aqueous solution |
|---|---|---|---|
| Recombinant protein | Hardly dispersed | Not dispersed | Slightly dispersed |
| Example 11 (acetylated protein) | Slightly dispersed | Uniformly dispersed | Slightly dispersed |
| Example 14 (succinylated protein) | Uniformly dispersed | Not dispersed | Dissolved |

### (5) Dispersibility of modified protein

Each of a 100 kDa recombinant protein PRT966, an acetylated protein, and a succinylated protein was suspended in DMSO (final concentration: 5 wt%) and heated in a heating shaker (90°C, 1500 rpm, 1 hour).

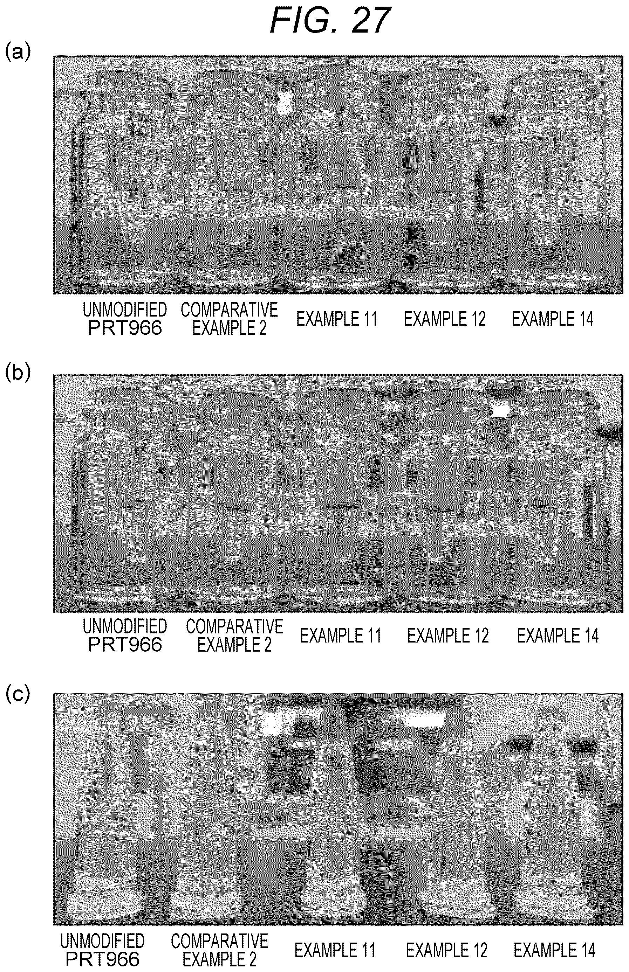

A state of the solution after heating and shaking is illustrated in Fig. 27. Fig. 27(a) is a photograph illustrating a state of a protein immediately after DMSO is added, Fig. 27(b) is a photograph illustrating a state of being dissolved in DMSO, and Fig. 27(c) is a photograph comparing gelation states of the proteins. All of these were transparent viscous solutions (gel-like), and no precipitation was observed. The degree of gelation descended in order of Example 11 (acetylated protein), Example 12 (acetylated protein), an unmodified protein PRT966, Comparative Example 2 (acetylated protein), and Example 14 (succinylated protein).

### 9. Spinning

A recombinant protein PRT966 and Example 8 (acetylated PRT966) were mixed at a predetermined ratio (weight). DMSO (final concentration: 20 wt%) and lithium chloride (final concentration: 4 wt%) were added to the obtained mixture, and heated and stirred (100°C, 30 minutes) with a magnetic stirrer hot plate to completely dissolve the proteins, thereby obtaining a dope solution (final concentration: 20 wt%). The obtained dope solution was loaded into a plastic syringe (trade name: ss-05Lz, manufactured by Terumo Corporation) equipped with a disposable 22G Luer lock needle (trade name: Neolus NN-2225R, manufactured by Terumo Corporation, inner diameter: 0.413 mm). Then, a tip of the needle was immersed in a coagulation bath (RO water at 75°C), and the dope solution was slowly and constantly injected into the coagulation bath while the needle was slowly moved around the coagulation bath all the time.

Results thereof are presented in Table 5. As a ratio of the acetylated protein was larger, adhesion after spinning was suppressed more. In addition, the unmodified protein had a flat shape in the coagulation bath, but it was found that when a dope solution containing the acetylated protein was used, coagulation occurred in a state where a cross-sectional shape was closer to a circular shape.

**[Table 5]**

| Example | Protein | Fiber rating |
|---|---|---|
| 1 | Only PRT966 | 3 |
| 2 | PRT966 and acetylated PRT966 (2:1) | 4 |
| 3 | PRT966 and acetylated PRT966 (1:1) | 4 |
| 4 | PRT966 and acetylated PRT966 (1:2) | 5 |
| 5 | Only acetylated PRT966 | 4 |

### 10. Modified protein-containing adhesive

### <Production of solution-state adhesive>

A 100 kDa recombinant protein PRT966 (7.5 g, 72.1 µmol) and sodium hydroxide (621 mg, 15.5 mmol, 1.1 equivalents per hydroxy group) were put in a 50 mL stainless steel cup (manufactured by Retsch GmbH), one stainless steel ball (diameter: 25 mm, manufactured by Retsch GmbH) was added thereto, and then the mixture was milled (frequency: 30 Hz, 30 minutes) with a mixer mill type ball mill (mixer mill MM400, manufactured by Retsch GmbH) to obtain a homogeneous off-white amorphous powder. Next, maleic anhydride (4.85 g, 49.4 mmol, 3.5 equivalents per hydroxy group) was put in the stainless steel cup, and then the mixture was further milled (frequency: 30 Hz, 60 minutes) with a mixer mill type ball mill (mixer mill MM400, manufactured by Retsch GmbH) to obtain a homogeneous off-white amorphous powder. The reaction mixture was removed from the stainless steel cup and put in RO water (200 mL) and suspended while being stirrer (5 minutes). Next, a 1 M HCl aqueous solution (50 mL) was added to the obtained suspension to acidify the suspension, and the suspension was further continuously stirred (5 minutes). Subsequently, acetonitrile (100 mL) was added to the suspension, and then a product was collected by vacuum filtration (Kiriyama No. 5A, φ60 mm filter paper, Kiriyama Seisakusho: PG201 diaphragm vacuum pump, Yamato Scientific Co., Ltd.). Thereafter, the collected product was sufficiently washed with RO water and acetone, and then completely dried under reduced pressure in a vacuum oven (VOS-310C vacuum oven, EYELA Tokyo Rikakikai Co., Ltd.: GCD-051X oil rotary vacuum pump, ULVAC Co., Ltd.) (80°C, 1 hour) to obtain a maleated (maleic acid esterified) protein powder formed of a light yellow amorphous solid. Next, 1 g of the thus obtained maleated protein powder was put in a 1 M sodium hydroxide aqueous solution (5 mL) heated to 80°C, and rapidly stirred for one hour to be dissolved. As a result, a solution-state adhesive in which the maleated protein was dissolved in a basic aqueous solution at a concentration of 20 wt% was obtained. This was designated as Example 23.

### <Production of adhesive body>

Two longitudinal rectangular cardboard pieces (20.4 cm × 5.9 cm × 0.3 cm) and two longitudinal rectangular glass pieces (7.6 cm × 2.6 cm × 0.1 cm) were prepared as adherends. Thereafter, the solution-state adhesive of Example 23 was applied to a part of one surface of one cardboard piece, and then the piece was allowed to stand for three minutes. Subsequently, the other cardboard piece was superimposed on the surface of the one cardboard piece to which the adhesive had been applied, and then the two cardboard pieces were sandwiched by clips such that the entire superimposed parts of the two cardboard pieces were pressed against each other, and allowed to stand overnight By drying the solution-state adhesive in this manner, a solvent in the solution-state adhesive was removed, and the solution-state adhesive was solidified to bond the two cardboard pieces to each other, thereby obtaining an adhesive body A in which the two cardboard pieces were bonded to each other. In addition, a similar operation to that used when the adhesive body A was obtained was performed except that two glass pieces were used instead of the two cardboard pieces, thereby obtaining an adhesive body B in which the two glass pieces were bonded to each other.

### <Strength of adhesive body A>

Next, an upper end of one cardboard piece in the adhesive body A was fixed and hung in midair, and a weight of 770 g was attached to a lower end of the other cardboard piece. Whether or not peeling occurred at a bonding portion located at an intermediate portion in the vertical direction was observed. As a result, it was found that peeling did not occur at the bonding portion, and the adhesive body A had sufficient bonding strength.

### <Strength of adhesive body B>

Next, in a state where the adhesive body B was disposed so as to extend in the horizontal direction, a portion of one cardboard piece located on a lower side, opposite to a bonding side, was fixed, and a weight of 190 g was placed on an end of the other cardboard piece located on an upper side, opposite to the bonding side, and presence or absence of occurrence of peeling at the bonding portion was observed. As a result, it was found that peeling did not occur at the bonding portion, and the adhesive body A had sufficient bonding strength.

### 11. Mechanochemical esterification using roller type mixer mill

In a reaction, a test kneader (Labo Plast Mill 3S150, manufactured by Toyo Seiki Seisaku-sho, Ltd.) equipped with a roller mixer model R60 (manufactured by Toyo Seiki Seisaku-sho, Ltd., blade shape: roller type) was used as a different direction rotating roller mixer.

### (Example 24) Acetylation of PRT966

Before the reaction was performed, a temperature in a feed chamber (60 mL) of the mixer mill was adjusted to 65°C. A ground mixture of a 100 kDa recombinant protein PRT966 (20.0 g, 0.192 mmol) and sodium hydroxide (1.66 g, 41.4 mmol, 1.1 equivalents per nucleophilic residue) was put in the feed chamber while a blade of the mixer mill was inched (10 rpm). Subsequently, acetic anhydride (17.8 mL, 188 mmol, 5 equivalents per nucleophilic residue) was carefully put therein dividedly. Inching (10 rpm) was performed until the reactants were homogenized. After it was confirmed that the blade of the mixer was free to rotate and both torque and temperature were stable, the milling procedure was initiated, and the reactants were continuously kneaded (50 rpm, 65°C, 1.5 hours) to obtain a beige amorphous powder. The reaction mixture was removed from the feed chamber and suspended in RO water (200 mL) while being stirred for ten minutes. The product was collected by vacuum filtration, then sufficiently washed with RO water and acetone, and then completely dried under reduced pressure in a vacuum oven (1 hour, 80°C) to obtain an off-white amorphous fine powder. The obtained powder was subjected to FT-IR and GPC analysis without further treatment.

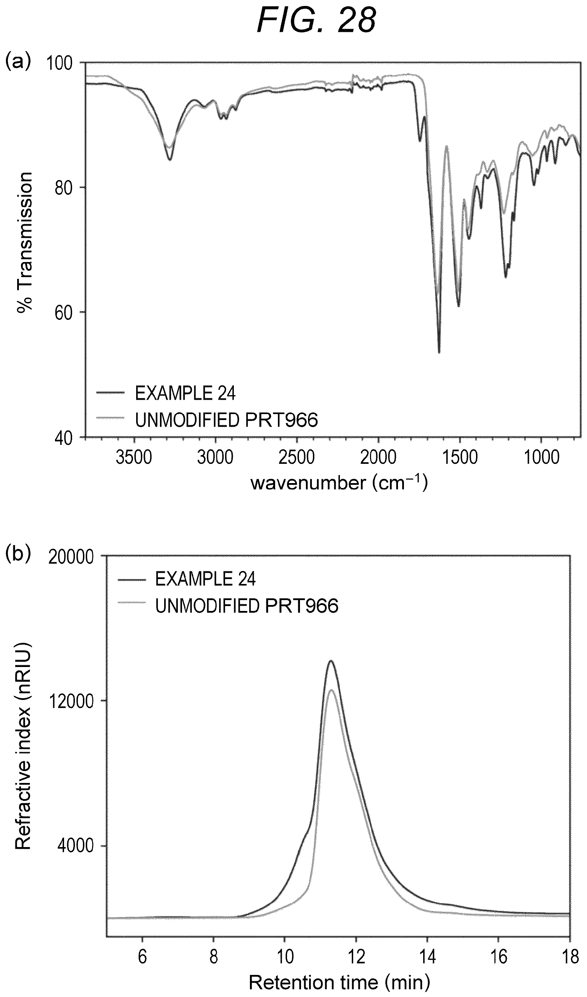

Results of FT-IR analysis and GPC analysis of the obtained amorphous powder are illustrated in Figs. 28(a) and 28(b), respectively, together with results of the recombinant protein PRT966 as a raw material. In the IR spectrum of Example 24, a new peak was observed around 1750 cm⁻¹, and it is considered that acetylation proceeded.

### (Example 25) Succinylation of PRT966

Before the reaction was performed, a temperature in a feed chamber (60 mL) of the mixer mill was adjusted to 65°C. A ground mixture of a 100 kDa recombinant protein PRT966 (20.0 g, 0.192 mmol) and sodium hydroxide (1.66 g, 41.4 mmol, 1.1 equivalents per nucleophilic residue) was put in the feed chamber while a blade of the mixer mill was inched (10 rpm). Subsequently, succinic anhydride (18.8 g, 188 mmol, 5 equivalents per nucleophilic residue) was carefully put therein dividedly. Inching (10 rpm) was performed until the reactants were homogenized. After it was confirmed that the blade of the mixer was free to rotate and both torque and temperature were stable, the milling procedure was initiated, and the reactants were continuously kneaded (50 rpm, 65°C, 1.5 hours) to obtain a beige amorphous powder. The reaction mixture was removed from the feed chamber and suspended in RO water (200 mL) while being stirred for five minutes. Thereafter, the suspension was acidified by addition of 1 M hydrochloric acid (50 mL) and further stirred for five minutes. Acetonitrile (200 mL) was added to the suspension and further stirred for five minutes. The product was collected by vacuum filtration, then sufficiently washed with RO water and acetone, and then completely dried under reduced pressure in a vacuum oven (1 hour, 80°C) to obtain an off-white amorphous fine powder. The obtained powder was subjected to FT-IR and GPC analysis without further treatment.

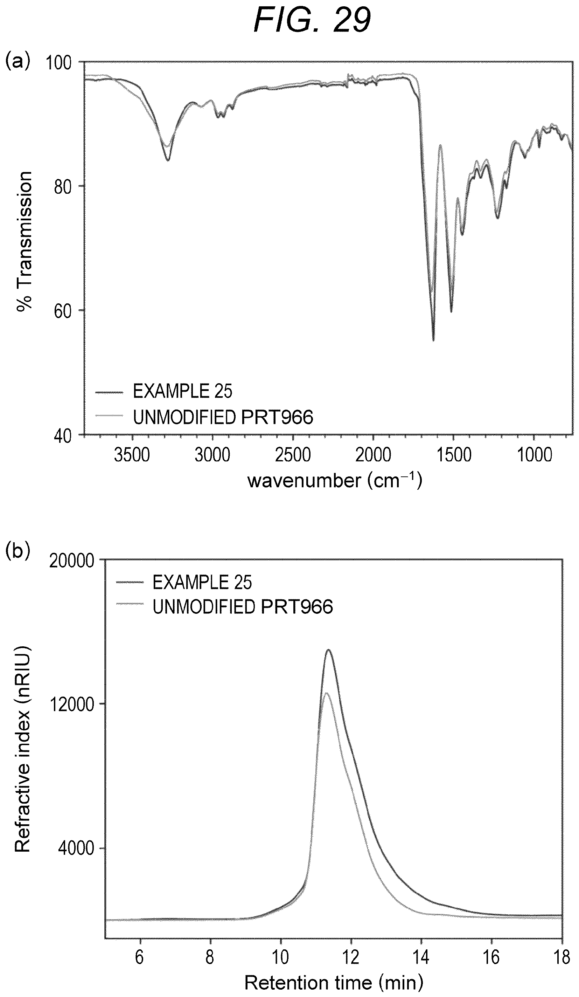

Results of FT-IR analysis and GPC analysis of the obtained amorphous powder are illustrated in Figs. 29(a) and 29(b), respectively, together with results of the recombinant protein PRT966 as a raw material. In the IR spectrum of Example 25, a new peak was observed around 1750 cm⁻¹, and it is considered that succinylation proceeded.

### 12. Mechanochemical esterification using twin screw extruder

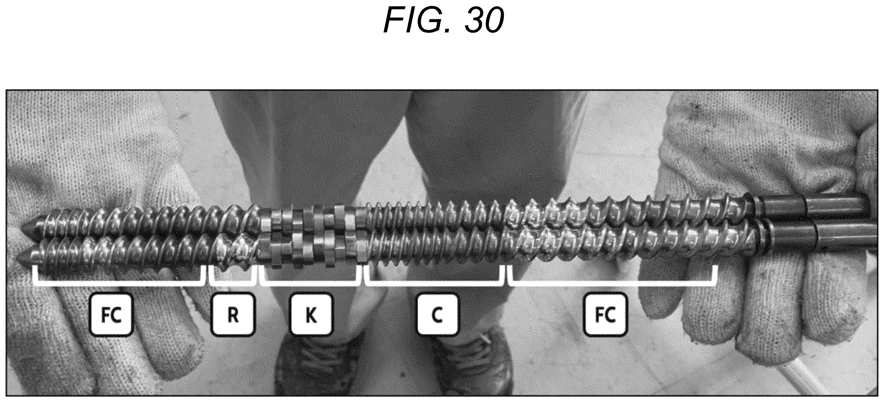

For the reaction, a test kneader (Labo Plast Mill Model 3S150, manufactured by Toyo Seiki Seisaku-sho, Ltd.) equipped with a unidirectionally rotating twin screw extruder (2D15W type, manufactured by Toyo Seiki Seisaku-sho, Ltd.) from which a die head had been removed was used. A screw segment of the extruder was configured as illustrated in Fig. 30, and a Stagger angle between adjacent discs of a kneading section was adjusted to be 45°. In Fig. 30, FC represents an advancing conveying element, R represents a reversing element, K represents a kneading element, and C represents a compression element.

### (Example 26) Succinylation of PRT966

Before the reaction was performed, a temperature of an extruder barrel was adjusted to 160°C. During the reaction, a rotational speed of a screw (100 rpm) was maintained. Succinic anhydride (5.0 g, 50.0 mmol) was gradually added to the extruder in order to lubricate the screw. Extrusion of the molten succinic anhydride was confirmed. Thereafter, a mixture of a 100 kDa recombinant protein PRT966 (50.0 g, 0.480 mmol), sodium hydroxide (4.14 g, 103 mmol, 1.1 equivalents per nucleophilic residue), and succinic anhydride (33.0 g, 330 mmol, 3.5 equivalents per nucleophilic residue) was gradually added to the extruder over 15 minutes while a torque was controlled so as not to exceed 40 N•m. The reaction mixture was extruded as a light brown amorphous powder, collected, and suspended in RO water (400 mL) while being stirred for ten minutes. Thereafter, the suspension was acidified by addition of 1 M hydrochloric acid (100 mL) and further stirred for five minutes. Acetonitrile (400 mL) was added to the suspension and further stirred for ten minutes. The product was collected by vacuum filtration, sufficiently washed with RO water and acetone, and then completely dried under reduced pressure in a vacuum oven (1 hour, 80°C) to obtain an off-white amorphous fine powder. The obtained powder was subjected to FT-IR and GPC analysis without further treatment.

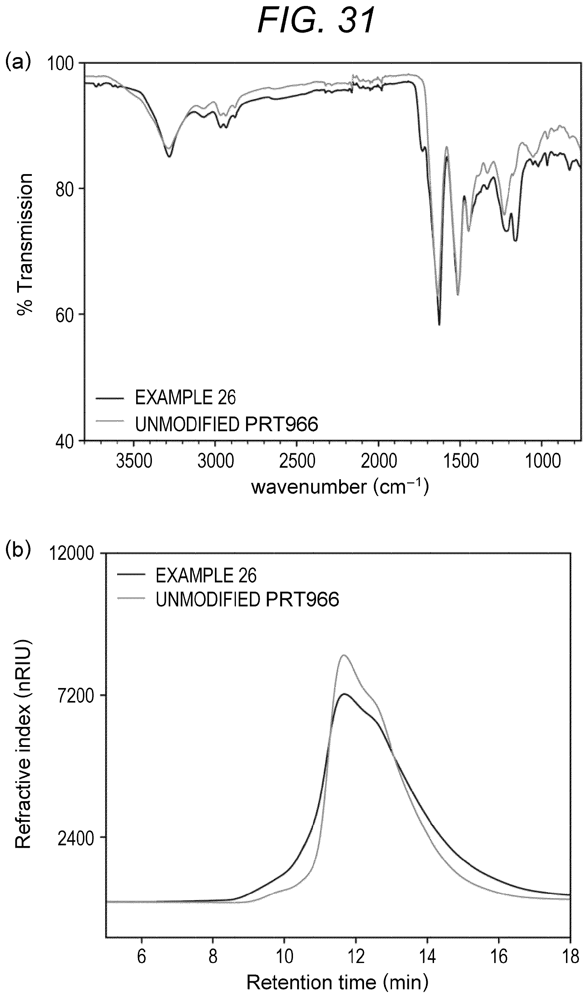

Results of FT-IR analysis and GPC analysis of the obtained amorphous powder are illustrated in Figs. 31(a) and 31(b), respectively, together with results of the recombinant protein PRT966 as a raw material. In the IR spectrum of Example 26, a new peak was observed around 1750 cm⁻¹, and it is considered that succinylation proceeded.

### 13. Acylation by mechanochemical method

### (Example 27) Sulfonation of 100 kDa recombinant protein

A recombinant protein PRT966 (7.5 g, 72.1 µmol) and a sulfur trioxide pyridine complex (7.87 g, 49.4 mmol, 3.5 equivalents per nucleophilic residue) were put in a 125 mL hardened steel grinding jar (manufactured by Retsch GmbH) together with stainless steel grinding media (8 × φ20 mm, manufactured by Retsch GmbH), and ground (frequency: 30 Hz, 90 minutes) using a mixer mill type ball mill (trade name: Mixer mill MM500 Control, manufactured by Retsch GmbH), then allowed to stand for 30 minutes, and ground (frequency: 30 Hz, 90 minutes) again to obtain an off-white amorphous powder. Next, sodium 2-ethylhexanoate (8.2 g, 49.4 mmol, 3.5 equivalents per nucleophilic residue) was added to the grinding jar and the reactants were ground (frequency: 30 Hz, 10 minutes) to obtain an off-white soft paste. The reaction mixture was removed from the jar, and the crude product was sequentially suspended, separated, and washed with CLYNSOLVE (registered trademark) P-7 (manufactured by Nippon Alcohol Sales Co., Ltd. 2 × 100 mL), RO water (2 x 100 mL), and acetone (2 x 100 mL). Thereafter, the product was collected by vacuum filtration (Kiriyama No. 5A, φ95 mm filter paper, Kiriyama Seisakusho: PG201 diaphragm vacuum pump, Yamato Scientific Co., Ltd.), and sufficiently washed with acetone. The product was dried at 80°C for one hour under reduced pressure using a vacuum oven, and then an off-white amorphous fine powder (NaSO₃-PRT) was obtained and directly subjected to analysis.

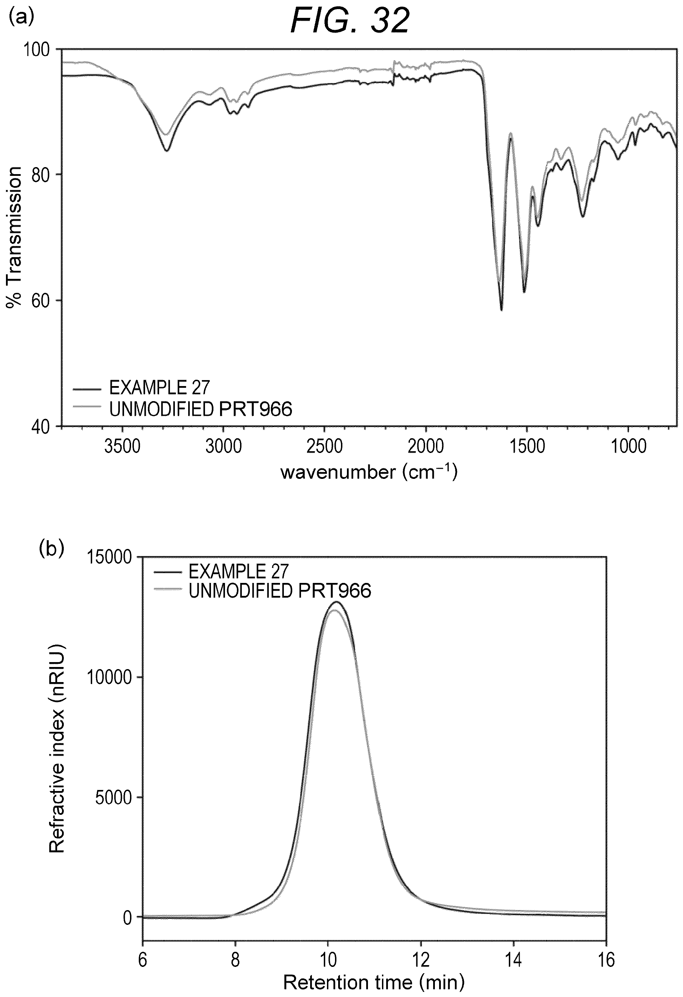

Results of FT-IR analysis and GPC analysis of the obtained amorphous powder are illustrated in Figs. 32(a) and 32(b), respectively, together with results of the recombinant protein PRT966 as a raw material.

### (Example 28) Sulfonation of 100 kDa recombinant protein

A recombinant protein PRT966 (7.5 g, 72.1 µmol) and a sulfur trioxide pyridine complex (7.87 g, 49.4 mmol, 3.5 equivalents per nucleophilic residue) were put in a 125 mL hardened steel grinding jar (manufactured by Retsch GmbH) together with stainless steel grinding media (8 × φ20 mm, manufactured by Retsch GmbH), and ground (frequency: 30 Hz, 90 minutes) using a mixer mill type ball mill (trade name: Mixer mill MM500 Control, manufactured by Retsch GmbH), then allowed to stand for 30 minutes, and ground (frequency: 30 Hz, 90 minutes) again to obtain an off-white amorphous powder. Next, sodium 2-ethylhexanoate (8.2 g, 49.4 mmol, 3.5 equivalents per nucleophilic residue) was added to the grinding jar and the reactants were ground (frequency: 30 Hz, 10 minutes) to obtain an off-white soft paste. The reaction mixture was removed from the jar, and the crude product was sequentially suspended, separated, and washed with CLYNSOLVE (registered trademark) P-7 (manufactured by Nippon Alcohol Sales Co., Ltd. 2 × 100 mL), RO water (2 x 100 mL), and acetone (2 x 100 mL). The crude product was suspended in 0.1 M hydrochloric acid (100 mL) and acidified, and continuously stirred for five minutes. Thereafter, a product was collected by vacuum filtration (Kiriyama No. 5A, φ95 mm filter paper, Kiriyama Seisakusho: PG201 diaphragm vacuum pump, Yamato Scientific Co., Ltd.), and sufficiently washed with acetone. The product was dried at 80°C for one hour under reduced pressure using a vacuum oven, and then an off-white amorphous fine powder (HSO₃-PRT) was obtained and directly subjected to analysis.

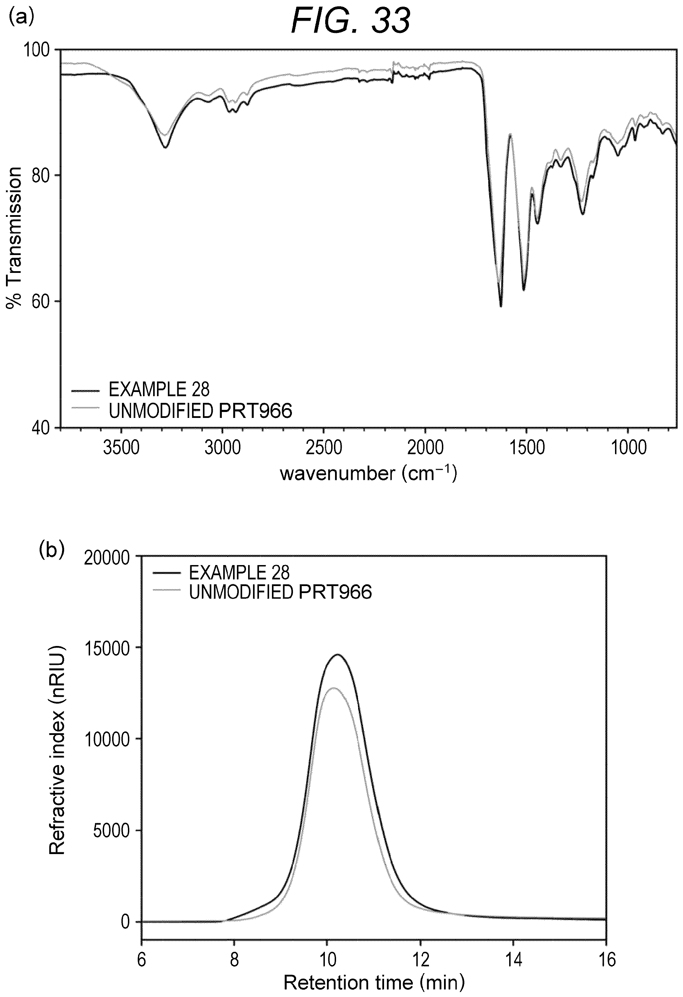

Results of FT-IR analysis and GPC analysis of the obtained amorphous powder are illustrated in Figs. 33(a) and 33(b), respectively, together with results of the recombinant protein PRT966 as a raw material.

### (Example 29) Stearylation of 100 kDa recombinant protein

Stearic acid (7.50 g, 26.4 mmol, 3.5 equivalents per nucleophilic residue) and 1,1-carbonyldiimidazole (4.27 g, 26.4 mmol, 3.5 equivalents per nucleophilic residue) were put in a 50 mL stainless steel grinding jar (manufactured by Retsch GmbH), and a stainless steel grinding medium (1 × φ25 mm ball, manufactured by Retsch GmbH) was added thereto. The mixture was ground (frequency: 30 Hz, 30 minutes) using a mixer mill type ball mill (Trade name: Mixer mill MM400, manufactured by Retsch GmbH) to obtain a colorless amorphous powder. Next, a 100 kDa recombinant protein PRT966 (4.0 g, 38.4 µmol) was added to the grinding jar and ground (frequency: 30 Hz, 90 minutes) to obtain a colorless amorphous powder. The reaction mixture was removed from the grinding jar and suspended in tetrahydrofuran (200 mL) while being stirred over ten minutes. Thereafter, a product was collected by vacuum filtration (Kiriyama No. 5A, φ95 mm filter paper, Kiriyama Seisakusho: PG201 diaphragm vacuum pump, Yamato Scientific Co., Ltd.), and sufficiently washed with acetone. The product was dried at 80°C for one hour under reduced pressure using a vacuum oven, and then an off-white amorphous fine powder (St-PRT) was obtained and directly subjected to analysis.

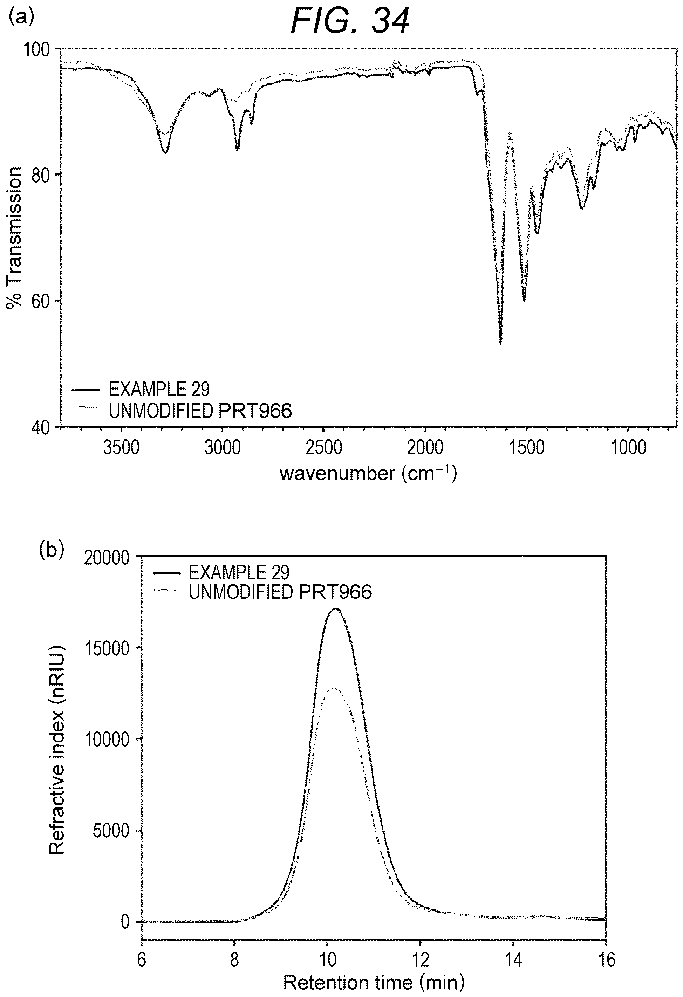

Results of FT-IR analysis and GPC analysis of the obtained amorphous powder are illustrated in Figs. 34(a) and 34(b), respectively, together with results of the recombinant protein PRT966 as a raw material.

### (Example 30) Oleinization of 100 kDa recombinant protein

In an inert nitrogen atmosphere, while oleic acid (8.32 mL, 26.4 mmol, 3.5 equivalents per nucleophilic residue) and 1,1-carbonyldiimidazole (4.27 g, 26.4 mmol, 3.5 equivalents per nucleophilic residue) were stirred in a closed flask using a magnetic stirrer and a hot plate, the mixture was continuously heated and stirred at 60°C until generation of carbon dioxide from the reaction mixture stopped. The reaction mixture and a recombinant protein PRT966 (4.0 g, 38.4 µmol) were put in a 50 mL stainless steel milling cup (manufactured by Retsch GmbH). A stainless steel milling medium (1 × φ25 mm ball, manufactured by Retsch GmbH) was put therein. The reaction mixture was ground (frequency: 30 Hz, 90 minutes) with a mixer mill type ball mill (mixer mill MM 400, manufactured by Retsch GmbH) to obtain a granular brown powder. The reaction mixture was removed from the grinding jar and suspended in tetrahydrofuran (200 mL) while being stirred over ten minutes. Thereafter, the product was collected by vacuum filtration (Kiriyama No. 5A, φ95 mm filter paper, Kiriyama Seisakusho: PG201 diaphragm vacuum pump, Yamato Scientific Co., Ltd.), and sufficiently washed with acetone. The product was dried at 80°C for one hour under reduced pressure using a vacuum oven, and then an off-white amorphous fine powder (Ol-PRT) was obtained and directly subjected to analysis.

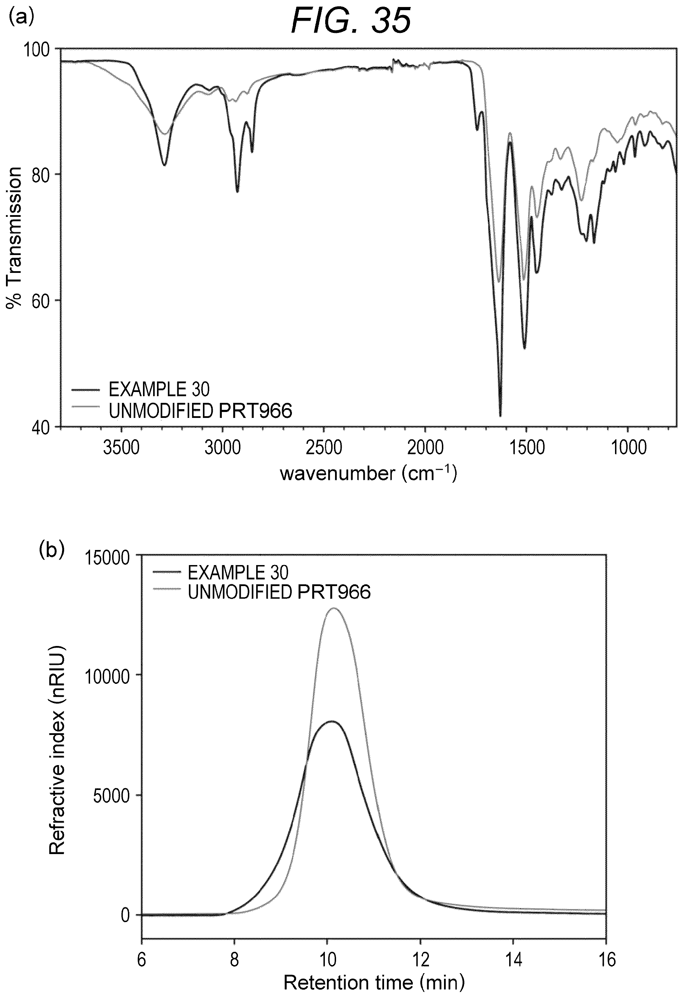

Results of FT-IR analysis and GPC analysis of the obtained amorphous powder are illustrated in Figs. 35(a) and 35(b), respectively, together with results of the recombinant protein PRT966 as a raw material.

### (Example 31) Sulfosuccinylation of 100 kDa recombinant protein

A 100 kDa maleic acid esterified recombinant protein (5.0 g, 40.5 µmol) and sodium bisulfite (4.13 g, 39.7 mmol, 5.0 equivalents per nucleophilic residue) were put in a 50 mL stainless steel grinding jar (manufactured by Retsch GmbH), and a stainless steel grinding medium (1 × φ25 mm ball, manufactured by Retsch GmbH) was added thereto. The mixture was subjected to liquid assist grinding (frequency: 30 Hz, 10 minutes) with RO water (1.0 mL) using a mixer mill type ball mill (trade name: Mixer mill MM400, manufactured by Retsch GmbH) to obtain a light beige amorphous powder. The reaction mixture was removed from the grinding jar and suspended in RO water (200 mL) while being stirred over ten minutes. Acetonitrile (100 mL) was added to the suspension, and a product was collected by vacuum filtration (Kiriyama No. 5A, φ95 mm filter paper, Kiriyama Seisakusho: PG201 diaphragm vacuum pump, Yamato Scientific Co., Ltd.), and sufficiently washed with acetone. The product was dried at 80°C for one hour under reduced pressure using a vacuum oven, and then an off-white amorphous fine powder (SulfoSucc-PRT) was obtained and directly subjected to analysis.

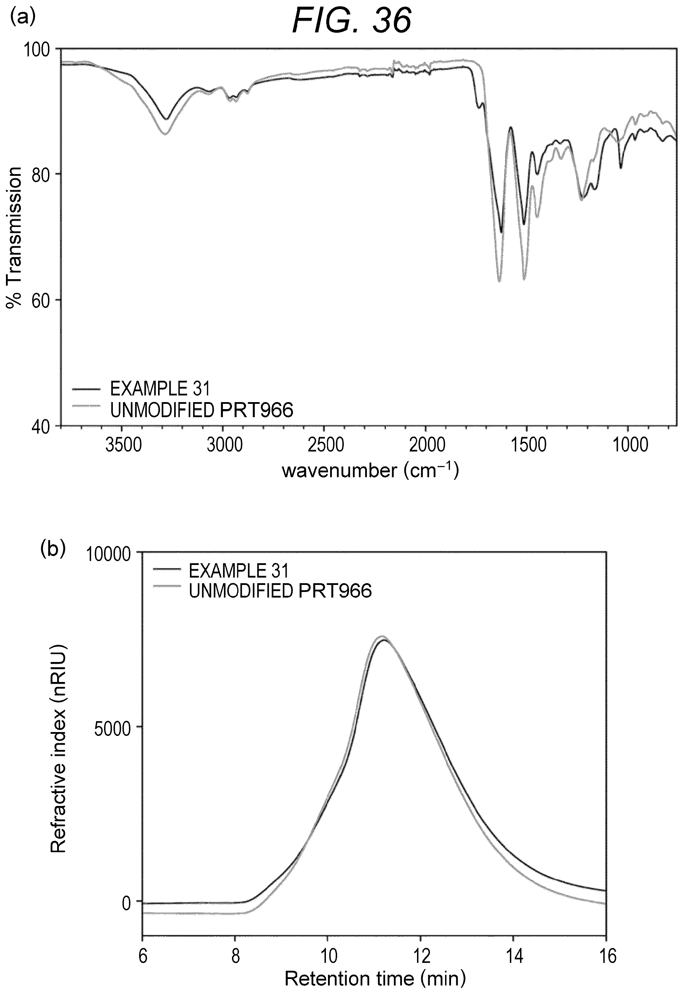

Results of FT-IR analysis and GPC analysis of the obtained amorphous powder are illustrated in Figs. 36(a) and 36(b), respectively, together with results of the recombinant protein PRT966 as a raw material.

### (Example 32) (Diethoxyphosphoryl) succinylation of 100 kDa recombinant protein

A 100 kDa maleic acid esterified PRT966 (5.0 g, 40.5 µmol), diethyl phosphite (3.59 mL, 27.9 mmol, 3.5 equivalents per nucleophilic residue), and sodium ethoxide (570 mg, 8.76 mmol, 1.1 equivalents per nucleophilic residue) were put in a 50 mL stainless steel grinding jar (manufactured by Retsch GmbH), and a stainless steel grinding medium (1 × φ25 mm ball) was added thereto. The mixture was subjected to liquid assist grinding (frequency: 30 Hz, 10 minutes) with RO water (1.0 mL) using a mixer mill type ball mill (trade name: Mixer mill MM400, manufactured by Retsch GmbH) to obtain a light beige amorphous powder. The reaction mixture was removed from the grinding jar and suspended in RO water (200 mL) while being stirred over ten minutes. Acetonitrile (100 mL) was added to the suspension, and a product was collected by vacuum filtration (Kiriyama No. 5A, φ95 mm filter paper, Kiriyama Seisakusho: PG201 diaphragm vacuum pump, Yamato Scientific Co., Ltd.), and sufficiently washed with acetone. The product was dried at 80°C for one hour under reduced pressure using a vacuum oven, and then an off-white amorphous fine powder (DEPS-PRT) was obtained. The obtained amorphous powder was directly subjected to analysis.

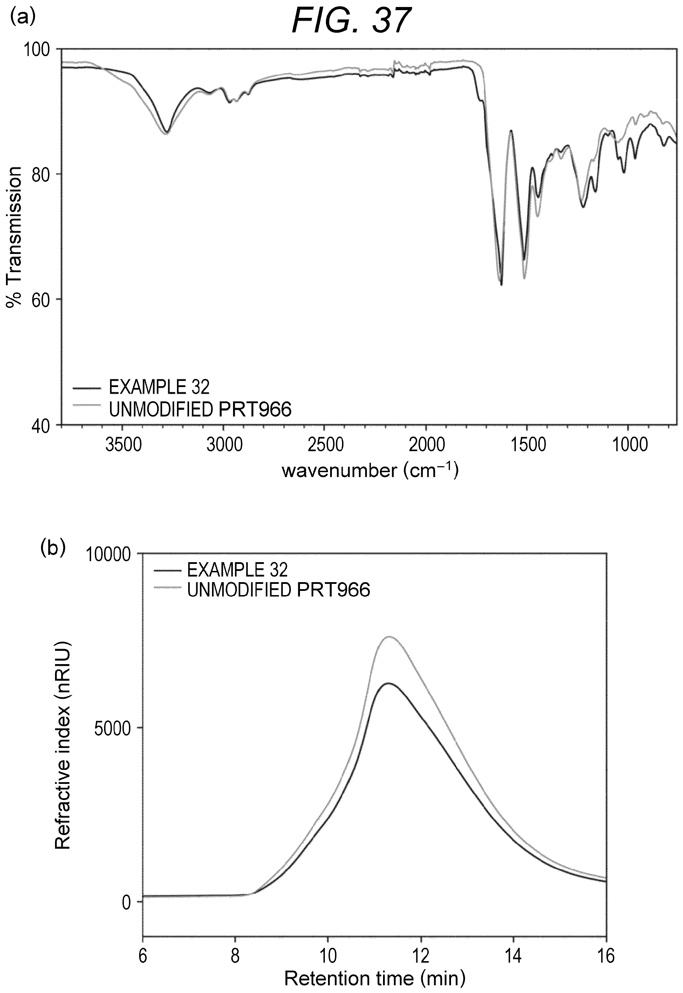

Results of FT-IR analysis and GPC analysis of the obtained amorphous powder are illustrated in Figs. 37(a) and 37(b), respectively, together with results of the recombinant protein PRT966 as a raw material.

### 14. Surface wettability test

### (1) Production of sample for analysis

A recombinant protein derivative to be analyzed was put in a 50 mL stainless steel milling jar (manufactured by Retsch GmbH) together with a stainless steel milling medium (1 × φ25 mm ball, manufactured by Retsch GmbH), and ground (frequency: 30 Hz, 5 minutes) with a mixer mill type ball mill (mixer mill MM400, manufactured by Retsch GmbH) to obtain a uniform powder. The ground sample was stored in a thermohygrostat (temperature range: 20 to 25°C±3°C, humidity range: 50 to 65%±5%RH) and allowed to equilibrate with the atmosphere over one hour.

### (2) Cast film

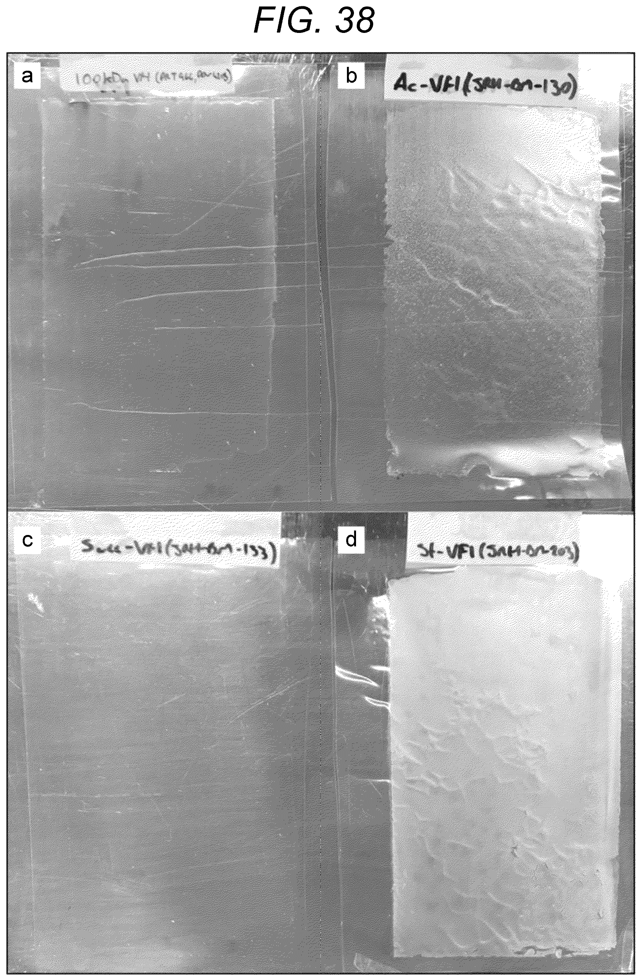

The obtained uniform recombinant protein powder was dissolved in DMSO at 80°C over one hour while being stirred to obtain a 15 wt% dope solution. This dope solution was cast on a backing sheet (trade name: SUNDAY PET PG1, transparent color, thickness 0.5 mm, Acrysunday Co., Ltd.) made of polyethylene terephthalate. A film thickness was controlled using a doctor blade coating applicator (80 mm × 400 µm, Imoto machinery Co., Ltd.) to form a film having a uniform film thickness of 400 µm. The obtained film was dried at 60°C for four hours using a constant temperature forced convection oven (DN411H, Yamato Scientific Co., Ltd.), and then dried at 80°C for eight hours under reduced pressure using a vacuum oven. The obtained film was cut into a size of 35 mm × 15 mm and fixed to a slide glass with an adhesive tape in order to keep the film flat at the time of contact angle analysis. Figs. 38(a) to 38(d) are photographs illustrating cast films of PRT966, Ac-PRT, Succ-PRT, and St-PRT, respectively.

### (3) Compressed powder

The obtained uniform recombinant protein powder (1.2 g) was added to a stainless steel die (Global Machine Co., Ltd.), compressed at 50 MPa using a tabletop Newton press (trade name: NT-100H, Sansho Industry Co., Ltd.), and held under pressure for one minute. The die used has a cylindrical shape and has a rectangular through hole having a cross section of 35 mm × 15 mm. Thereafter, the pressure was released, and the sample was removed from the die to obtain a compressed sample having a rectangular parallelepiped shape of 35 mm × 15 mm × 2 mm.

### (4) Contact angle measurement experiment

Prior to analysis, an analysis sample was stored in a thermohygrostat (temperature range: 20 to 25°C±3°C, humidity range: 50 to 65%±5%RH) and allowed to equilibrate with the atmosphere over one hour. Surface wettability was evaluated by measuring a static contact angle between one droplet (about 2 µL) of RO water and a sample surface. The contact angle was measured by a sessile drop method using a commercially available contact angle meter (trade name: DMs-601, Kyowa Interface Science Co., Ltd.) and image capture and data fitting software (FAMAS, Kyowa Interface Science Co., Ltd.). Six measurements were performed in total for each recombinant protein derivative, and an average value of the contact angles was calculated. An experimental result was expressed as an average value ± a standard deviation, and a case of contact angle < 90° was evaluated as "being hydrophilic", and a case of contact angle ≥ 90° was evaluated as "being hydrophobic".

### (5) Results

Water contact angles were measured for both a compressed powder sample and a cast film prepared from each of the recombinant protein PRT966, Ac-PRT (Example 8), Succ-PRT (Example 14), and St-PRT (Example 29). Figs. 38(a) to 38(d)are photographs illustrating cast films of PRT966, Ac-PRT, Succ-PRT, and St-PRT, respectively.

The measured water contact angles for each recombinant protein derivative (n=6) are summarized in Table 6. An error represents a standard deviation. "Ellipse" in Table 6 is an average value of left and right contact angles. From a steepness of the measured water contact angle, contact angle analysis using the compressed powder samples indicated that order from a sample having the highest hydrophobicity to a sample having the highest hydrophilicity was Ac-PRT > St-PRT > PRT966 > Succ-PRT. In any of the experiments, Ac-PRT and St-PRT were observed to be more hydrophobic than PRT966, and Succ-PRT was observed to be more hydrophilic than PRT966.

**[Table 6]**

| Sample | Film | | | Compressed powder | | |
|---|---|---|---|---|---|---|
| | Contact angle (°) | | | Contact angle (°) | | |
| | Left | Right | Ellipse | Left | Right | Ellipse |
| PRT966 | 78.5 ± 3.3 | 78.8 ± 3.0 | 78.7 ± 3.0 | 72.3 ± 4.4 | 72.5 ± 4.4 | 72.4 ± 4.0 |
| Ac-PRT | 87.6 ± 3.9 | 88.3 ± 3.3 | 87.9 ± 2.8 | 114.9 ± 3.1 | 114.7 ± 2.7 | 114.8 ± 2.9 |
| Suc-PRT | 68.1 ± 2.7 | 68.3 ± 2.3 | 68.2 ± 2.1 | 59.2 ± 6.7 | 57.7 ± 3.9 | 58.5 ± 5.2 |
| St-PRT | 102.5 ± 3.7 | 102.1 ± 3.7 | 102.3 ± 3.5 | 110.1 ± 5.7 | 108.6 ± 7.8 | 109.4 ± 6.5 |

### 15. Flame retardancy test

### (1) Method for producing sample for analysis

A recombinant protein derivative to be analyzed was put in a 50 mL stainless steel milling jar (manufactured by Retsch GmbH) together with a stainless steel milling medium (1 × φ25 mm ball), and ground (frequency: 30 Hz, 5 minutes) with a mixer mill type ball mill (mixer mill MM400, manufactured by Retsch GmbH) to obtain a uniform powder. The ground sample was stored in a thermohygrostat (temperature range: 20 to 25°C±3°C, humidity range: 50 to 65%±5%RH) and allowed to equilibrate with the atmosphere over one hour. The obtained uniform protein powder (1.2 g) was added to a stainless steel die (Global Machine Co., Ltd.), and pressurized at a pressure of 50 MPa for one minute using a tabletop Newton press (trade name: NT-100H, Sansho Industry Co., Ltd.). The die used has a cylindrical shape and has a dumbbell-shaped through hole having a cross section of 45 mm × 15 mm. Thereafter, the pressure was released, and the sample was removed from the die to obtain a compressed sample having a dumbbell shape of 45 mm × 15 mm × 2 mm.

### (2) Flame retardancy analysis

Prior to analysis, an analysis sample was allowed to equilibrate with the atmosphere over one hour in a thermohygrostat (20 to 25°C±3°C, 50 to 65%±5%RH). Each sample was placed in a support such that the length of the sample was horizontal, and at least 40 mm of a strip was extended from the support. An extended end of the sample was ignited with a laboratory burner having an orange flame of 25 mm. The flame was applied to the sample for 30 seconds, then the flame was removed, and a time until the flame disappeared and a change in sample mass were recorded. The measurement was performed for each recombinant protein derivative (n=6), and an average value of the combustion times and an average value of the mass losses were calculated. An experimental result was expressed as an average value ± standard deviation.

### (3) First flame retardancy test

In a first flame retardancy test, flame retardancy of each of an unmodified PRT966, NaSO₃-PRT (Example 27), HSO₃-PRT (Example 28), and SulfoSucc-PRT (Example 31) was evaluated. The test was performed in an environment of 5 to 10°C and 30 to 35%RH.

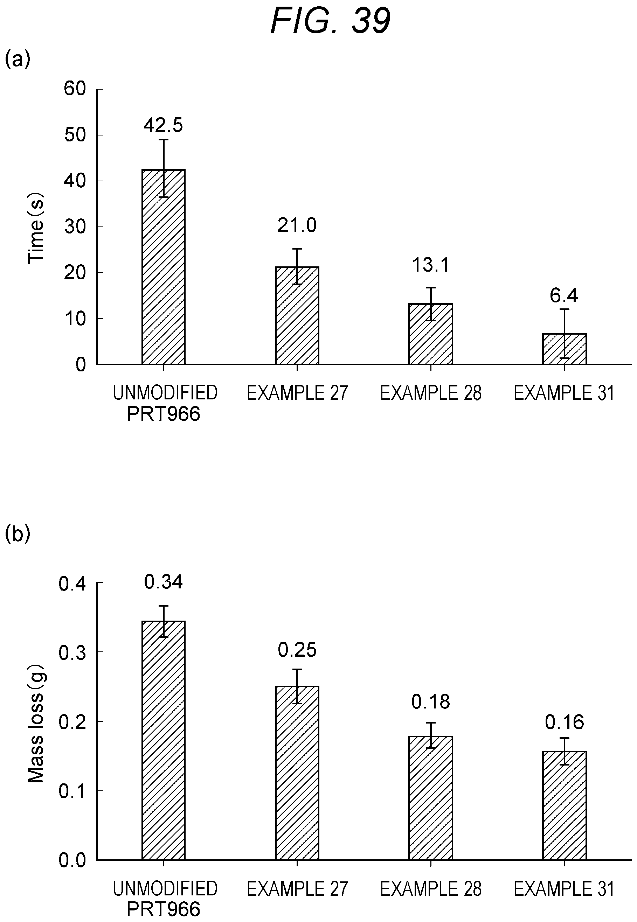

A water content, a combustion time, and a mass loss of each recombinant protein (n=6) were summarized in Table 7. Fig. 39(a) is a graph illustrating combustion times of samples, and Fig. 39(b) is a graph illustrating mass losses of the samples. An error represents a standard deviation. From the measured combustion times and mass losses, all the protein derivative samples exhibited better fire resistance than PRT966, and Example 31 exhibited the best fire resistance. Order of the fire resistance was Example 31 > Example 28 > Example 27 > PRT966.

**[Table 7]**

| Sample | Water content (wt%) | Combustion time (s) | Mass loss (g) |
|---|---|---|---|
| Unmodified PRT966 | 9.02 | 42.5 ± 6.2 | 0.344 ± 0.022 |
| NaSO₃-PRT | 9.22 | 21.0 + 3.8 | 0.250 ± 0.023 |
| HSO₃-PRT | 8.93 | 13.1 ± 3.6 | 0.179 ± 0.018 |
| SulfoSucc-PRT | 8.32 | 6.4 ± 5.4 | 0.145 ± 0.019 |

### (4) Second flame retardancy test

In a second flame retardancy test, fire resistance of each of the unmodified PRT966, SulfoSucc-PRT (Example 31), and DEPS-PRT (Example 32) was evaluated. The test was performed in an ambient environment of 20 to 25°C and 70 to 75%RH.

A water content, a combustion time, and a mass loss of each recombinant protein derivative (n=6) were summarized in Table 8. An error represents a standard deviation. From the measured combustion times and mass losses, the modified protein derivatives exhibited better fire resistance than the unmodified PRT966, and Example 32 exhibited the best fire resistance. Order of the fire resistance was Example 32 > Example 31 > PRT966.

**[Table 8]**

| Sample | Water content (wt%) | Combustion time (s) | Mass loss (g) |
|---|---|---|---|
| Unmodified PRT966 | 9.18 | 40.2 ± 2.1 | 0.263 ± 0.037 |
| SulfoSucc-PRT | 8.52 | 10.2 ± 4.4 | 0.165 ± 0.012 |
| DEPS-PRT | 8.13 | 0.2 ± 0.2 | 0.118 ± 0.011 |

### 15. Thermoplastic test

### (1) Production of resin sample for analysis

A recombinant protein derivative (neat or containing 20 wt% glycerol as a plasticizer) to be analyzed was put in a 50 mL stainless steel milling jar (manufactured by Retsch GmbH) together with a stainless steel milling medium (1 × φ25 mm ball), and ground (frequency: 30 Hz, 5 minutes) with a mixer mill type ball mill (mixer mill MM400, manufactured by Retsch GmbH) to obtain a uniform mixture. The obtained uniform mixture (3.0 g) was added to a stainless steel die (Global Machine **Co.,** Ltd.) to which a fluorine-based release agent (trade name: DAIFREE MS-600, DAIKIN INDUSTRIES, LTD.) was thinly applied, and pressurized at a pressure of 30 MPa using a tabletop Newton press (trade name: NT-100H, Sansho Industry **Co.,** Ltd.). The die used has a cylindrical shape and has a rectangular through hole having a cross section of 35 mm × 15 mm. The compressed sample was heated to 170°C using a temperature control controller (trade name: MTCD 15-EN, MISUMI Group, Inc.) and held at this temperature for five minutes. The sample was cooled to 60°C while being pressurized at 30 MPa. Thereafter, the pressure was released, and the sample was removed from the die to obtain a resin tablet having dimensions of 35 mm × 15 mm × 4.5 mm. The molded resin tablet was cut into an analysis sample of 5 mm × 5 mm × 4.5 mm using a table round saw (trade name: K-210, HOZAN Co., Ltd.) equipped with a diamond disc cutter (particle size: #150, trade name: K-210-3, HOZAN Co., Ltd.).

### (2) Thermomechanical analysis

A glass transition point (Tg) onset and a softening point of each recombinant protein resin sample were determined using a thermomechanical analyzer (trade name: TMA4000, manufactured by NETZSCH japan Co., Ltd.) equipped with a flat-tipped quartz compression-loaded probe (φ8 mm compression detection rod P/N T1442-04; compressed support pipe P/N T1442 03, manufactured by NETZSCH japan Co., Ltd.) pressed against the sample with a load of 4.9 N. Thereafter, the sample was heated to 220°C at a constant rate (10 K•min⁻¹), and deformation of the sample was recorded. Six measurements were performed in total for each analyzed recombinant protein derivative, and an average value of the glass transition point (Tg) onsets and an average value of the softening points were calculated. An experimental result was expressed as an average value ± a standard deviation. Thermomechanical analysis was performed on each of resin samples prepared using the unmodified PRT966, Ac-PRT (Example 8), Succ-PRT (Example 14), Mal-PRT (Example 23), St-PRT (Example 29), and Ol-PRT (Example 30). Analysis was performed on both neat protein resin samples and resin samples plasticized with 20 wt% glycerol.

### (3) Results

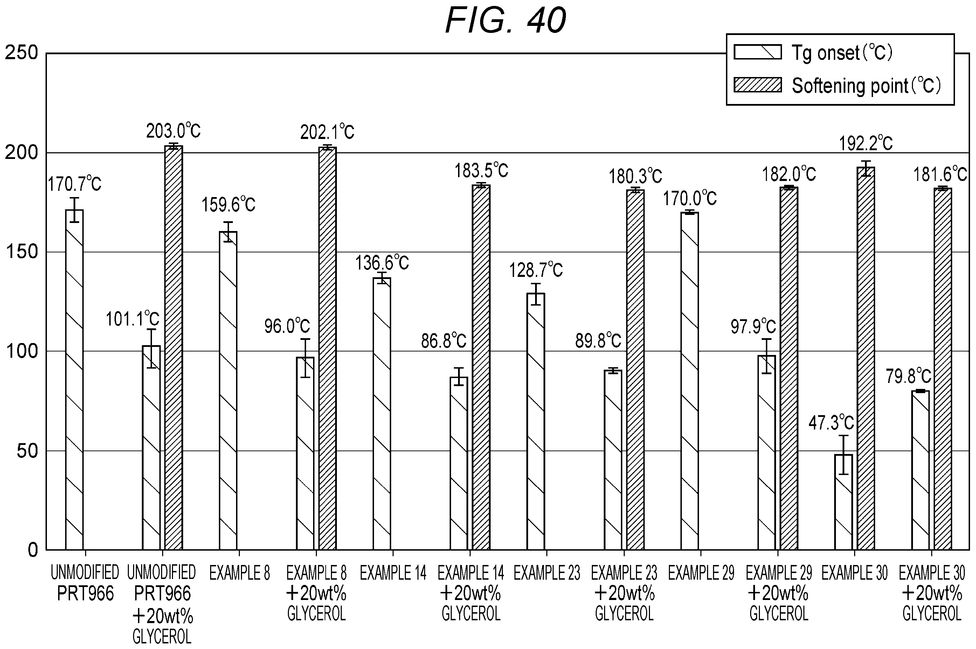

The Tg onset and the softening point of each recombinant protein resin sample (n=6) were summarized in Table 9. An error represents a standard deviation. Fig. 40 is a graph illustrating softening points, Tg onsets, and melting temperatures Tm of the unmodified PRT966 and the modified proteins. Thermomechanical analysis revealed that, as for both the unmodified protein and the plasticized recombinant protein resin samples, all the modified derivatives had lower glass transition points (Tg) and softening points than the unmodified PRT966, and Example 30 had the lowest Tg onset and softening point. Furthermore, the analysis indicated that addition of 20 wt% glycerol as a plasticizer could improve thermal processability of the recombinant protein resin, and it was confirmed that the softening points of all the plasticized samples were equal to or lower than a maximum temperature (220°C) .

**[Table 9]**

| Sample | No plasticizer | | Glycerol added | |
|---|---|---|---|---|
| | Tg onset (°C) | Softening point (°C) | Tg onset (°C) | Tg onset (°C) |
| PRT966 | 170.7 ± 6.2 | N/A | 101.1 ± 9.6 | 203.0 ± 1.0 |
| Ac-PRT | 159.6 ± 5.0 | N/A | 96.0 ± 9.5 | 202.1 ± 1.2 |
| Succ-PRT | 136.6 ± 3.1 | N/A | 86.8 ± 4.4 | 183.5 ± 1.2 |
| Mal-PRT | 128.7 ± 5.3 | N/A | 89.8 ± 1.8 | 180.3 ± 0.6 |
| St-PRT | 170.0 ± 1.2 | N/A | 97.9 ± 8.5 | 182.0 ± 1.2 |
| Ol-PRT | 47.3 ± 9.9 | N/A | 79.8 ± 0.6 | 181.6 ± 0.8 |

## Claims

1. A method for producing a modified protein, the method comprising treating a mixture containing a protein and an acylating agent by a mechanochemical method to obtain a modified protein.

2. The method according to claim 1, wherein the mixture further contains a base and/or a reaction accelerator.

3. The method according to claim 1, wherein the protein contains a hydrophobic protein.

4. The method according claim 3, wherein the hydrophobic protein has a hydropathy index of more than 0.

5. The method according to claim 1, wherein the protein contains an artificial protein.

6. The method according to claim 5, wherein the artificial protein contains an artificial structural protein.

7. The method according to any one of claims 1 to 6, further comprising a step of obtaining the protein by a microbiological method.

8. The method according to claim 1, wherein the treatment by the mechanochemical method is implemented by applying a shear force to the mixture.

9. The method according to claim 8, wherein the shear force is applied to the mixture using a mixer mill or an extruder.

10. A modified protein obtained by the method according to any one of claims 1 to 6.

11. A modified protein obtained by the method according to claim 7.

12. A modified protein composition comprising the modified protein according to claim 10.

13. A modified protein composition comprising the modified protein according to claim 11.

14. A modified protein molded body obtained by molding the modified protein composition according to claim 12.

15. A modified protein molded body obtained by molding the modified protein composition according to claim 13.

16. A molding material comprising the modified protein according to claim 10.

17. A molding material comprising the modified protein according to claim 11.

18. A modified protein molded body obtained by molding the molding material according to claim 16.

19. A modified protein molded body obtained by molding the molding material according to claim 17.

20. A method for producing a solution-state adhesive, the method comprising a step of dissolving a modified protein obtained by the method according to claim 1 in a solvent.

21. A method for producing a water-dispersible adhesive, the method comprising a step of dispersing a modified protein obtained by the method according to claim 1 in an aqueous medium.

22. A method for producing a film-shaped adhesive, the method comprising a step of molding a film from a solution in which a modified protein obtained by the method according to claim 1 is dissolved.

23. A method for producing a powdery adhesive, the method comprising a step of obtaining a powder containing a modified protein obtained by the method according to claim 1.

24. A method for producing an adhesive body, the method comprising interposing a solution obtained by dissolving a modified protein obtained by the method according to claim 1 in a solvent between a plurality of adherends, and then removing the solvent from the solution to solidify the modified protein, thereby bonding the adherends to each other.

25. A method for producing an adhesive body, the method comprising interposing an aqueous dispersion obtained by dispersing a modified protein obtained by the method according to claim 1 in an aqueous medium between a plurality of adherends, and then removing the aqueous medium from the aqueous dispersion to solidify the modified protein, thereby bonding the adherends to each other.

26. A method for producing an adhesive body, the method comprising softening a film containing a modified protein obtained by the method according to claim 1 by swelling or heating, interposing the film between a plurality of adherends, and then curing the film in a state of being brought into pressure contact with the adherends, thereby bonding the adherends to each other.

27. A method for producing an adhesive body, the method comprising heating a powder containing a modified protein obtained by the method according to claim 1 in a state where the powder is interposed between a plurality of adherends, and pressurizing the powder via the adherends to solidify the powder, thereby bonding the adherends to each other.

28. A method for producing a solution-state coating agent, the method comprising a step of dissolving a modified protein obtained by the method according to claim 1 in a solvent.

29. A method for producing a water-dispersible coating agent, the method comprising a step of dispersing a modified protein obtained by the method according to claim 1 in an aqueous medium.

30. A method for producing a film-shaped coating agent, the method comprising a step of molding a film from a solution in which a modified protein obtained by the method according to claim 1 is dissolved.

31. A method for producing a powdery coating agent, the method comprising a step of obtaining a powder containing a modified protein obtained by the method according to claim 1.

32. A method for producing a laminate including a base material and a coating layer laminated on at least a part of a surface of the base material, the method comprising
supplying a solution obtained by dissolving a modified protein obtained by the method according to claim 1 in a solvent to at least a part of the surface of the base material to coat at least a part of the surface of the base material with the solution, and then removing the solvent from the solution to solidify the modified protein, thereby laminating the coating layer on at least a part of the surface of the base material.

33. A method for producing a laminate including a base material and a coating layer laminated on at least a part of a surface of the base material, the method comprising
supplying an aqueous dispersion obtained by dispersing a modified protein obtained by the method according to claim 1 in an aqueous medium to at least a part of the surface of the base material to coat at least a part of the surface of the base material with the aqueous dispersion, and then removing the aqueous medium from the aqueous dispersion to solidify the modified protein, thereby laminating the coating layer on at least a part of the surface of the base material.

34. A method for producing a laminate including a base material and a coating layer laminated on at least a part of a surface of the base material, the method comprising
softening a film containing a modified protein obtained by the method according to claim 1 by swelling or heating, placing the film on at least a part of the surface of the base material, and then curing the film in a state of being brought into pressure contact with the base material, thereby laminating the coating layer on at least a part of the surface of the base material.

35. A method for producing a laminate including a base material and a coating layer laminated on at least a part of a surface of the base material, the method comprising:
heating a powder containing a modified protein obtained by the method according to claim 1 while the powder is placed on at least a part of the surface of the base material, and pressurizing the powder between a pressurizing body and the base material to solidify the powder, thereby laminating the coating layer on at least a part of the surface of the base material.
